# EUROPÄISCHE PATENTSCHRIFT

(11) **EP 0 597 360 B1**
(45) Veröffentlichungstag und Bekanntmachung des Hinweises auf die Patenterteilung: **17.10.2001**
(21) Anmeldenummer: 93117748.9
(22) Anmeldetag: 02.11.1993
(51) Int. Cl.: C07D 249/12, C07D 405/12, C07D 401/12, C07F 7/08, C07C 255/50, A01N 43/653

(54) **Substituierte N-Phenyl-Triazolin(thi)one, Verfahren sowie neue Zwischenprodukte zu ihrer Herstellung und ihre Verwendung als Herbizide, Insektizide und Akarizide**
Substituted N-phenyl-triazolin (thi)ones, processes and intermediates for their production and their use as herbicides, insecticides and acaricides
N-phényle-triazolin (thi)ones substitués, procédés et produits intermédiaires pour leur préparation et leur utilisation comme herbicides, insecticides et acaricides

(30) Priorität: 12.11.1992 DE 4238125
(43) Veröffentlichungstag der Anmeldung: 18.05.1994
(73) Patentinhaber: BAYER AG, 51368 Leverkusen (DE)
(72) Erfinder: Haas, Wilhelm, Dr., D-50259 Pulheim (DE); Findeisen, Kurt, Prof.Dr., D-51375 Leverkusen (DE); Linker, Karl-Heinz, D-51377 Leverkusen (DE); Schallner, Otto, Dr., D-40789 Monheim (DE); König, Klaus, Dr., D-51519 Odenthal (DE); Marhold, Albrecht, Dr., D-51373 Leverkusen (DE); Santel, Hans-Joachim, Dr., D-51371 Leverkusen (DE); Dollinger, Markus, Dr., D-42799 Leichlingen (DE); Wachendorff-Neumann, Ulrike, D-40789 Monheim (DE)

(56) Entgegenhaltungen:
- EP-A- 0 370 332
- EP-A- 0 422 469
- EP-A- 0 431 373
- EP-A- 0 441 004
- WO-A-91/03470
- US-A- 4 702 763
- US-A- 4 705 557
- US-A- 4 818 275
- US-A- 4 846 875
- CHEMICAL ABSTRACTS, vol. 102, no. 23, 10. Juni 1985, Columbus, Ohio, US; abstract no. 204066k, PANDEY O P ET AL. 'Mono(cyclopentadienyl)titanium(IV) derivatives with heterocyclic thiones' Seite 609 ;Spalte 1 ;
- CHEMICAL ABSTRACTS, Band 111, Nr. 17, 26. April 1993, Columbus, Ohio, US, Seite 850, Spalte 2, Zusammenfassung Nr. 168823z, KUMAI S. ET AL.: "Preparation of 2,4,5-trifluorobenzoic acid from trichlorobenzonitrile. & JP-A-04 321 644 (ASAHI GLASS CO., LTD.)"

## Beschreibung

Die Erfindung betrifft neue substituierte Triazolinone, mehrere Verfahren zu ihrer Herstellung und ihre Verwendung als Herbizide, Insektizide und Akarizide.

Es ist bekannt, daß bestimmte substituierte Triazolinone wie beispielsweise die Verbindung 3,4-Dimethyl-1-(3-fluor-4-cyano-phenyl)-1,2,4-triazolin-5-on oder die Verbindung 3-Methyl-4-propargyl-1-(2,5-difluor-4-cyano-phenyl)-1,2,4-triazolin-5-on herbizide Eigenschaften besitzen (vergl. z.B. DE 38 39 480).

Weiterhin wird in der Schrift WO 9 103 470 ein Verfahren zur Herstellung von Phenyltriazolinonen und in der US 4 818 275 Phenyltriazolinone selber mit herbiziden Wirkeigenschaften beschrieben. Ebenso beschreiben die US 4 846 875, US 4 705 557 und US 4 702 763 eine Reihe verschiedener 1 Phenyltriazolinone als Herbizide mit speziellen Substituenten und Substitutionsmustern.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolinone der allgemeinen Formel (I), 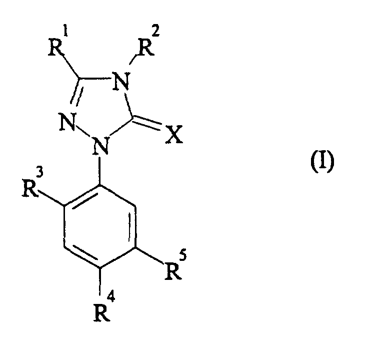 in welcher
- R¹: für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom oder Iod - steht,
- R²: für Amino, für geradkettiges oder verzweigtes Alkyl mit 1 bis 8 Kohlenstoffatomen, für geradkettiges oder verzweigtes Alkenyl mit jeweils 2 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom oder Iod, für geradkettiges oder verzweigtes Alkoxyalkyl mit jeweils 1 bis 4 Kohlenstoffatomen in den einzelnen Alkylteilen, für geradkettiges oder verzweigtes Alkylidenimino mit 1 bis 8 Kohlenstoffatomen oder für jeweils gegebenenfalls im Cycloalkylteil einfach oder mehrfach, gleich oder verschieden durch Halogen - insbesondere Fluor, Chlor, Brom und/oder Iod - substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im geradkettigen oder verzweigten Alkylteil steht,
- R³: für Wasserstoff, Fluor, Chlor, Brom oder Iod steht,
- R⁴: für Cyano oder Nitro steht,
- R⁵: für Nitro, Cyano, Fluor, Chlor, Brom, Iod, R⁶, -O-R⁶, -S-R⁶, -NR⁶R⁷, oder für einen Rest der Formel 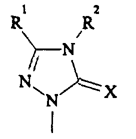 steht und
- X: für Sauerstoff oder Schwefel steht, wobei
- R⁶ und R⁷: unabhängig voneinander jeweils für Wasserstoff oder für gegebenenfalls einfach oder mehrfach, gleich oder verschieden substituiertes, geradkettiges oder verzweigtes Alkyl mit 1 bis 8 Kohlenstoffatomen stehen, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, Alkoxycarbonylalkyl, N-Alkylaminocarbonyl, Cycloalkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefelsteht;
- R⁶ und R⁷: außerdem für jeweils gegebenenfalls einfach oder mehrfach, gleich oder verschieden durch Halogen - insbesondere Fluor, Chlor, Brom und/oder Iodsubstituiertes Alkenyl oder Alkinyl mit jeweils 2 bis 8 Kohlenstoffatomen stehen;
- R⁶ und R⁷: außerdem für gegebenenfalls einfach oder mehrfach, gleich oder verschieden durch Halogen - insbesondere Fluor, Chlor, Brom und/oder Iodund/oder geradkettiges oder verzweigtes Alkyl mit 1 bis 4 Kohlenstoffatomen substituiertes Cycloalkyl mit 3 bis 7 Kohlenstoffatomen oder für C₃-C₇-Cycloalkyl-C₁-C₃-alkyl stehen oder
- R⁶ und R⁷: für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, wobei als Arylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I), 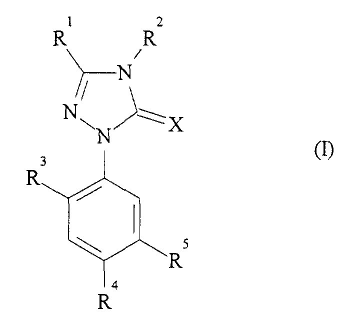 in welcher
- R¹, R², R³, R⁴, R⁵ und X: die oben angegebenen Bedeutungen haben
erhält, wenn man
a) 1H-Triazolinone der Formel (II), 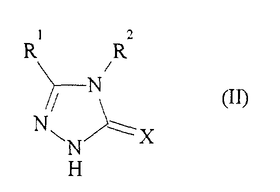 in welcher
   - R¹, R² und X: die oben angegebenen Bedeutungen haben,
   mit Halogenbenzol-Derivaten der Formel (III), 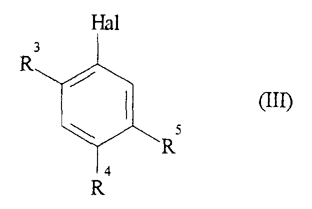 in welcher
   - R³, R⁴ und R⁵: die oben angegebenen Bedeutungen haben und
   - Hal: für Halogen steht,
   gegebenenfalls in Gegenwart eines Verdünnungsmittels und gegebebenfalls in Gegenwart eines Reaktionshilfsmittels umsetzt,
   oder wenn man
b) substituierte Triazolinone der Formel (I), 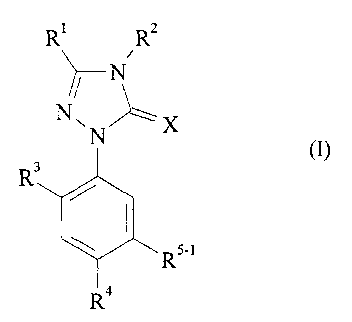 in welcher
   - R¹, R², R³, R⁴ und X: die oben angegebenen Bedeutungen haben und
   - R⁵⁻¹: für Halogen steht,
   mit Nukleophilen der Formel (IV),

   R⁶⁻¹-Z-H (IV)

   in welcher
   - z: für Sauerstoff oder Schwefel steht und
   - R⁶⁻¹: für jeweils geradkettiges oder verzweigtes, gegebenenfalls substituiertes Alkyl, Alkenyl, Alkinyl, Cycloalkyl oder Aryl steht,
   gegebenenfalls in Gegenwart eines Verdünnungsmittels und gegebenenfalls in Gegenwart eines Reaktionshilfsmittels umsetzt oder wenn man
c) substituierte Triazolinone der Formel (I), 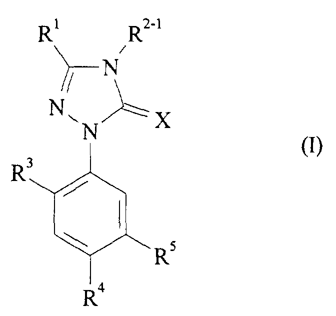 in welcher
   - R¹, R³, R⁴, R⁵ und X: die oben angegebenen Bedeutungen haben und
   - R²⁻¹: für Amino steht,
   mit Natriumnitrit in Gegenwart einer Säure und gegebenenfalls in Gegenwart eines Verdünnungsmittels umsetzt oder wenn man
d) substituierte Triazolinone der Formel (I), 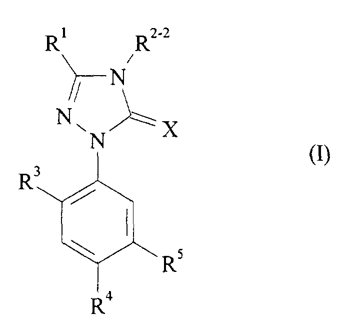 in welcher
   - R¹,R³,R⁴,R⁵ und X: die oben angegebenen Bedeutungen haben und
   - R²⁻²: für Wasserstoff steht,
   mit Alkylierungsmitteln der Formel (V),

   R²⁻³-E (V)

   in welcher
   - R²⁻³: für Alkyl, Alkenyl, Alkinyl, Halogenalkyl, Halogenalkenyl, Halogenalkinyl, Alkoxyalkyl oder für gegebenenfalls substituiertes Cycloalkyl steht und
   - E: für eine elektronenanziehende Abgangsgruppe steht,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide, insektizide und akarizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern und gleichzeitig unerwarteterweise auch eine erheblich bessere akarizide Wirksamkeit im Vergleich zu den aus dem Stand der Technik bekannten substituierten Triazolinonen, wie beispielsweise die Verbindung 3,4-Dimethyl-1-(3-fluor-4-cyano-phenyl)-1,2,4-triazolin-5-on oder die Verbindung 3-Methyl-4-propargyl-1-(2,5-difluor-4-cyano-phenyl)-1,2,4-triazolin-5-on welche chemisch und wirkungsmäßig naheliegende Verbindungen sind.

Besonders bevorzugt sind Verbindungen der Formel (I), bei welchen
- R¹: für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht,
- R²: für Amino, für geradkettiges oder verzweigtes Alkyl mit 1 bis 6 Kohlenstoffatomen, für geradkettiges oder verzweigtes Alkenyl mit jeweils 2 bis 4 Kohlenstoffatomen, für geradkettiges oder verzweigtes Halogenalkyl mit 1 bis 4 Kohlenstoffatomen und 1 bis 9 gleichen oder verschiedenen Halogenatomeninsbesondere Fluor, Chlor oder Brom -, für geradkettiges oder verzweigtes Alkoxyalkyl mit jeweils 1 bis 3 Kohlenstoffatomen in den einzelnen Alkylteilen, für geradkettiges oder verzweigtes Alkylidenimino mit 1 bis 6 Kohlenstoffatomen oder für jeweils gegebenenfalls im Cycloalkylteil einfach bis vierfach, gleich oder verschieden durch Halogen - insbesondere Fluor, Chlor und/ oder Brom - substituiertes Cycloalkyl oder Cycloalkylalkyl mit jeweils 3 bis 7 Kohlenstoffatomen im Cycloalkylteil und gegebenenfalls 1 bis 3 Kohlenstoffatomen im geradkettigen oder verzweigten Alkylteil steht,
- R³: für Wasserstoff, Fluor, Chlor oder Brom steht,
- R⁴: für Cyano oder Nitro steht,
- R⁵: für Nitro, Cyano, Fluor, Chlor, Brom, R⁶, -O-R⁶, -S-R⁶, -NR⁶R⁷, oder für einen Rest der Formel 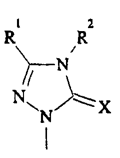 steht und
- X: für Sauerstoff oder Schwefel steht, wobei
- R⁶ und R⁷: unabhängig voneinander jeweils für Wasserstoff oder für gegebenenfalls einfach substituiertes geradkettiges oder verzweigtes Alkyl mit 1 bis 6 Kohlenstoffatomen stehen, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, Alkoxycarbonylalkyl, N-Alkylaminocarbonyl, N,N-Dialkylaminocarbonyl, Trialkylsilyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁶ und R⁷: außerdem für geradkettiges oder verzweigtes und gegebenenfalls neben Halogen zusätzlich substituiertes Halogenalkyl mit 1 bis 4 Kohlenstoffatomen und 1 bis 9 gleichen oder verschiedenen Halogenatomen - insbesondere Fluor, Chlor oder Brom - stehen, wobei als Substituenten C₁-C₂-Alkoxycarbonyl, C₁₋₆-Cycloalkylaminocarbonyl und Cyano infrage kommen;
- R⁶ und R⁷: außerdem für jeweils gegebenenfalls einfach bis dreifach, gleich oder verschieden durch Halogen - insbesondere Fluor, Chlor oder Brom - substituiertes Alkenyl oder Alkinyl mit jeweils 2 bis 6 Kohlenstoffatomen stehen;
- R⁶ und R⁷: außerdem für gegebenenfalls einfach bis vierfach, gleich oder verschieden durch Halogen - insbesondere Fluor, Chlor oder Brom - und/oder geradkettiges oder verzweigtes Alkyl mit 1 bis 3 Kohlenstoffatomen substituiertes Cycloalkyl mit 3 bis 6 Kohlenstoffatomen oder für C₃-C₆-Cycloalkyl-C₁-C₂-alkyl stehen oder
für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil stehen, wobei als Phenylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl.

Ganz besonders bevorzugt sind Verbindungen der Formel (I), bei welchen
- R¹: für Halogenalkyl mit 1 oder 2 Kohlenstoffatomen und 1 bis 5 gleichen oder verschiedenen Halogenatomen - insbesondere Fluor oder Chlor - steht,
- R²: für Amino, für geradkettiges oder verzweigtes Alkyl mit 1 bis 4 Kohlenstoffatomen, für geradkettiges oder verzweigtes Alkenyl mit jeweils 2 bis 3 Kohlenstoffatomen, für Halogenalkyl mit 1 oder 2 Kohlenstoffatomen und 1 bis 5 gleichen oder verschiedenen Halogenatomen - insbesondere Fluor, Chlor oder Brom -, für geradkettiges oder verzweigtes Alkoxyalkyl mit jeweils 1 oder 2 Kohlenstoffatomen in den einzelnen Alkylteilen, für geradkettiges oder verzweigtes Alkylidenimino mit 1 bis 6 Kohlenstoffatomen oder für jeweils gegebenenfalls im Cycloalkylteil einfach oder zweifach, gleich oder verschieden durch Halogen - insbesondere Fluor oder Chlor - substituiertes Cyclopropyl, Cyclopropylmethyl, Cyclohexyl oder Cyclohexylmethyl steht,
- R³: für Wasserstoff, Fluor oder Chlor steht,
- R⁴: für Cyano oder Nitro steht,
- R⁵: für Nitro, Cyano, Fluor, Chlor, Brom, R⁶, -O-R⁶, -S-R⁶, -NR⁶R⁷, oder für einen Rest der Formel 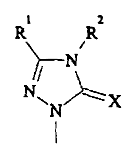 steht und
- X: für Sauerstoff oder Schwefel steht, wobei
- R⁶ und R⁷: unabhängig voneinander jeweils für Wasserstoff oder für gegebenenfalls einfach substituiertes geradkettiges oder verzweigtes Alkyl mit 1 bis 4 Kohlenstoffatomen stehen, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, Alkylcarbonylalkyl, N-Alkylaminocarbonyl, N,N-Dialkylaminocarbonyl, Trialkylsilyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁶ und R⁷: außerdem für gegebenenfalls neben Halogen zusätzlich substituiertes Halogenalkyl mit 1 bis 3 Kohlenstoffatomen und 1 bis 7 gleichen oder verschiedenen Halogenatomen - insbesondere Fluor oder Chlor - stehen, wobei als Substituenten jeweils Methoxycarbonyl, Ethoxycarbonyl, Cyano oder Cyclopropylaminocarbonyl infrage kommen;
- R⁶ und R⁷: außerdem für jeweils gegebenenfalls einfach durch Halogen - insbesondere Fluor oder Chlor - substituiertes Alkenyl oder Alkinyl mit jeweils 2 bis 5 Kohlenstoffatomen stehen;
- R⁶ und R⁷: außerdem für gegebenenfalls einfach oder zweifach, gleich oder verschieden durch Fluor, Chlor, Methyl und/oder Ethyl substituiertes Cyclopropyl oder Cyclohexyl oder für Cyclopropylmethyl, Cyclopentylmethyl oder Cyclohexylmethyl stehen oder
- R⁶ und R⁷: für jeweils gegebenenfalls im Phenylteil einfach oder zweifach, gleich oder verschieden substituiertes Phenylalkyl oder Phenyl mit gegebenenfalls 1 oder 2 Kohlenstoffatomen im Alkylteil stehen, wobei als Phenylsubstituenten jeweils infrage kommen:
Fluor, Chlor, Brom, Cyano, Nitro, Methyl, Ethyl, n- oder i-Propyl, n-, i-, s-oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trfluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis zweifach, gleich oder verschieden durch Fluor, Chlor, Brom, Methyl, Ethyl, Methoxy, Ethoxy, Trifluormethyl und/oder Trifluormethoxy substituiertes Phenyl.

Im einzelnen seien außer den bei den Herstellungsbeispielen aufgeführten Verbindungen die folgenden substituierten Triazolinone der allgemeinen Formel (I) genannt:

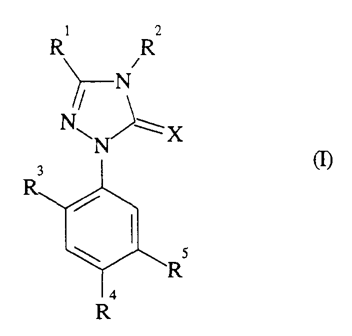

| **R**^{**1**} | **R**^{**2**} | **R**^{**3**} | **R**^{**4**} | **R**^{**5**} | **X** |
|---|---|---|---|---|---|
| CF₃ | CH₃ | F | CN | OH | O |
| CF₃ | CH₃ | Cl | CN | OH | O |
| CF₃ | CH₃ | F | NO₂ | OH | O |
| CF₃ | CH₃ | Cl | NO₂ | OH | O |
| CF₃ | CH₃ | Cl | CN | CH₃O | O |
| CF₃ | CH₃ | Cl | NO₂ | CH₃O | O |
| CF₃ | CH₃ | F | NO₂ | CH₃O | O |
| CF₃ | CH₃ | F | CN | -O-CH₂-C≡CH | O |
| CF₃ | CH₃ | Cl | CN | -O-CH₂-C≡CH | O |

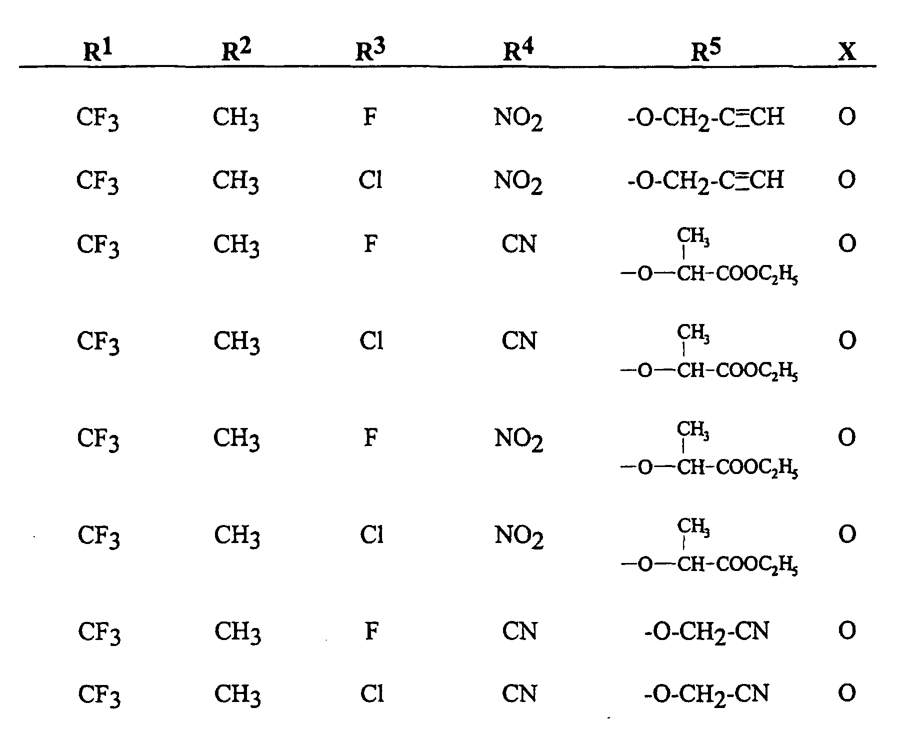

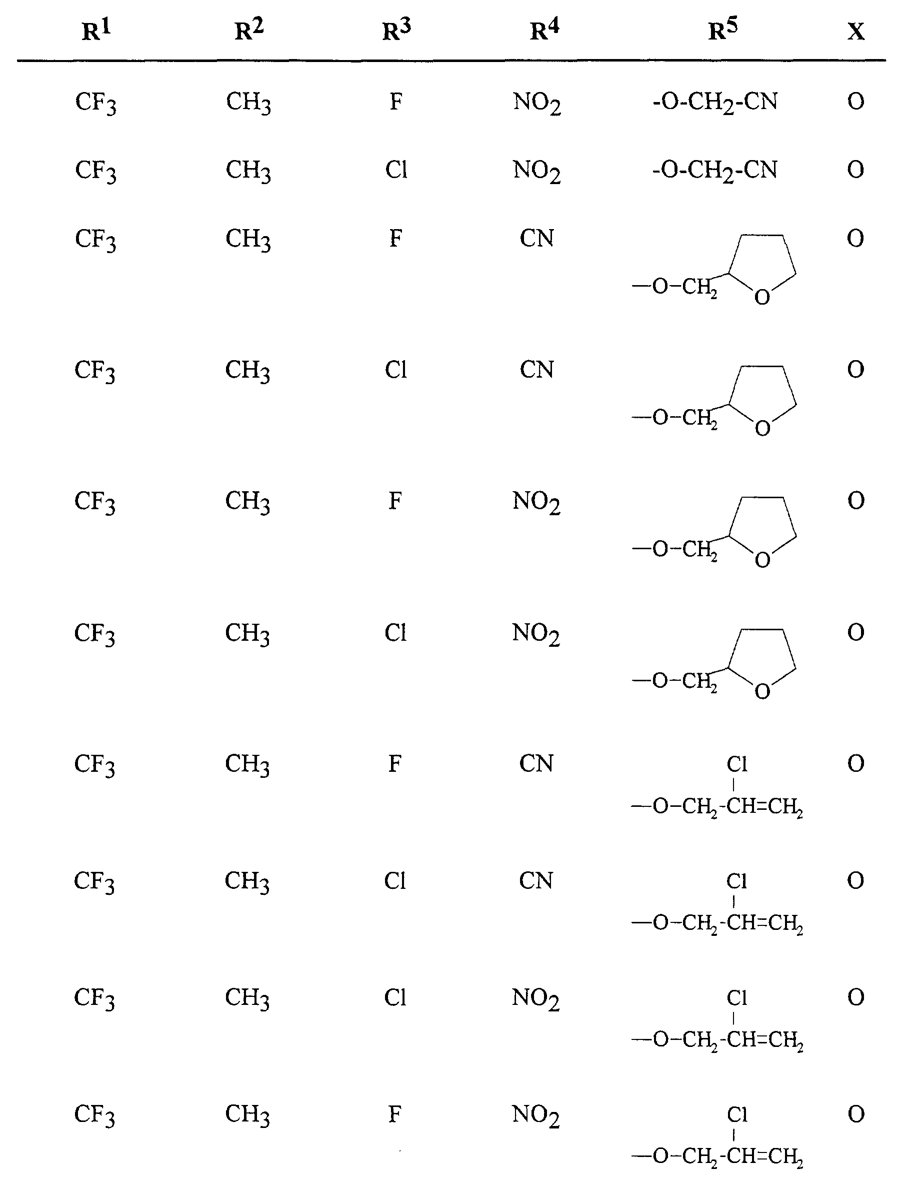

| **R**^{**1**} | **R**^{**2**} | **R**^{**3**} | **R**^{**4**} | **R**^{**5**} | **X** |
|---|---|---|---|---|---|
| CF₃ | CH₃ | F | CN | -O-CH₂-COOCH₃ | O |
| CF₃ | CH₃ | Cl | CN | -O-CH₂-COOCH₃ | O |
| CF₃ | CH₃ | F | NO₂ | -O-CH₂-COOCH₃ | O |
| CF₃ | CH₃ | Cl | NO₂ | -O-CH₂-COOH₃ | O |
| CF₃ | CH₃ | F | NO₂ | F | O |
| CF₃ | CH₃ | Cl | NO₂ | F | O |
| CF₃ | CH₃ | F | NO₂ | Cl | O |
| CF₃ | CH₃ | Cl | NO₂ | Cl | O |
| CF₃ | CH₃ | F | CN | -O-CHF₂ | O |
| CF₃ | CH₃ | Cl | CN | -O-CHF₂ | O |
| CF₃ | CH₃ | F | NO₂ | -O-CHF₂ | O |
| CF₃ | CH₃ | Cl | NO₂ | -O-CHF₂ | O |
| CF₃ | CH₃ | F | CN | -S-CH₃ | O |
| CF₃ | CH₃ | Cl | CN | -S-CH₃ | O |
| CF₃ | CH₃ | F | NO₂ | -S-CH₃ | O |
| CF₃ | CH₃ | Cl | NO₂ | -S-CH₃ | O |

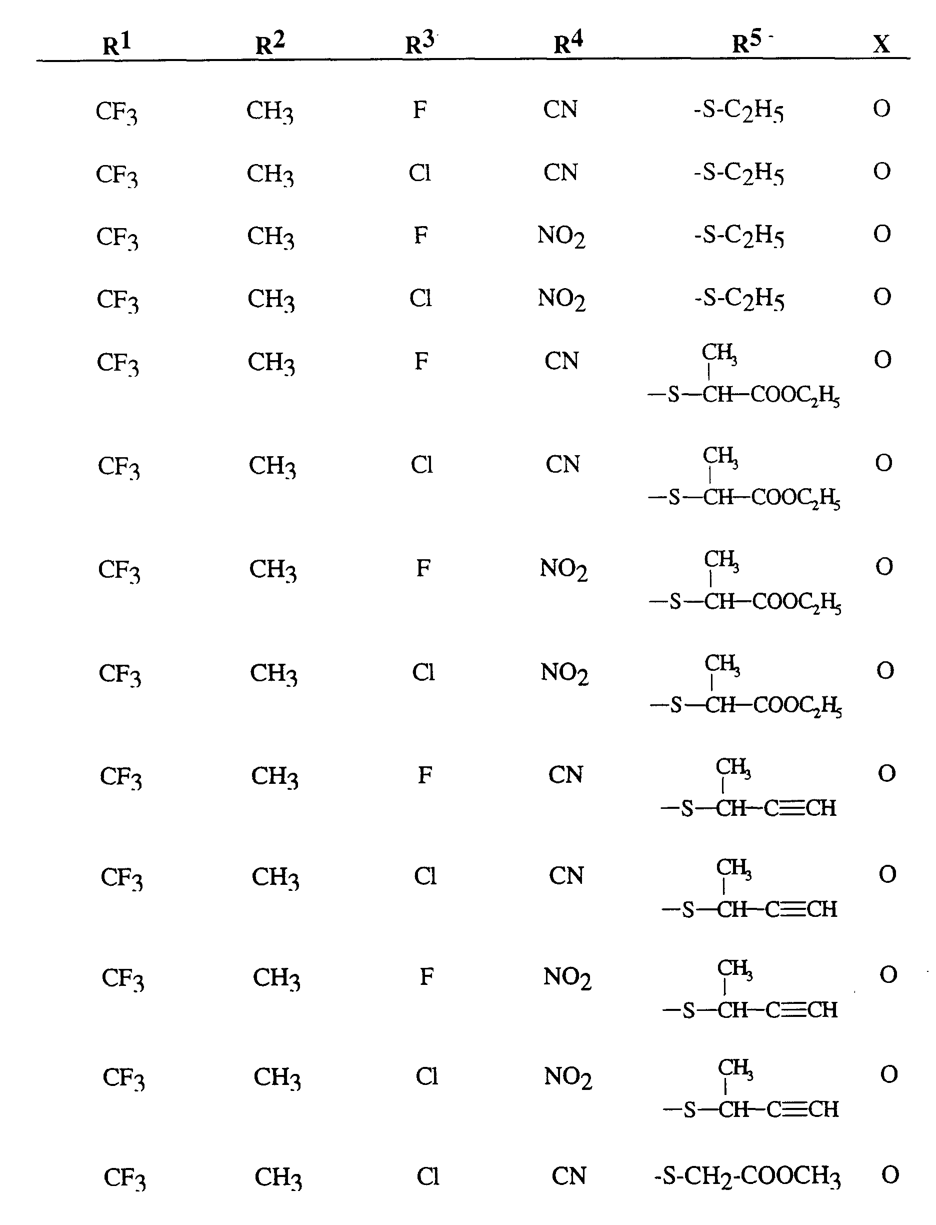

| **R**^{**1**} | **R**^{**2**} | **R**^{**3**} | **R**^{**4**} | **R**^{**5**} | **X** |
|---|---|---|---|---|---|
| CF₃ | CH₃ | F | CN | -S-CH₂-COOCH₃ | O |
| CF₃ | CH₃ | Cl | NO₂ | -S-CH₂-COOCH₃ | O |
| CF₃ | CH₃ | F | NO₂ | -S-CH₂-COOCH₃ | O |
| CF₃ | CH₃ | F | CN | CH₃ | O |
| CF₃ | CH₃ | F | CN | -NH-CH₃ | O |
| CF₃ | CH₃ | F | CN | -N(CH₃)₂ | O |

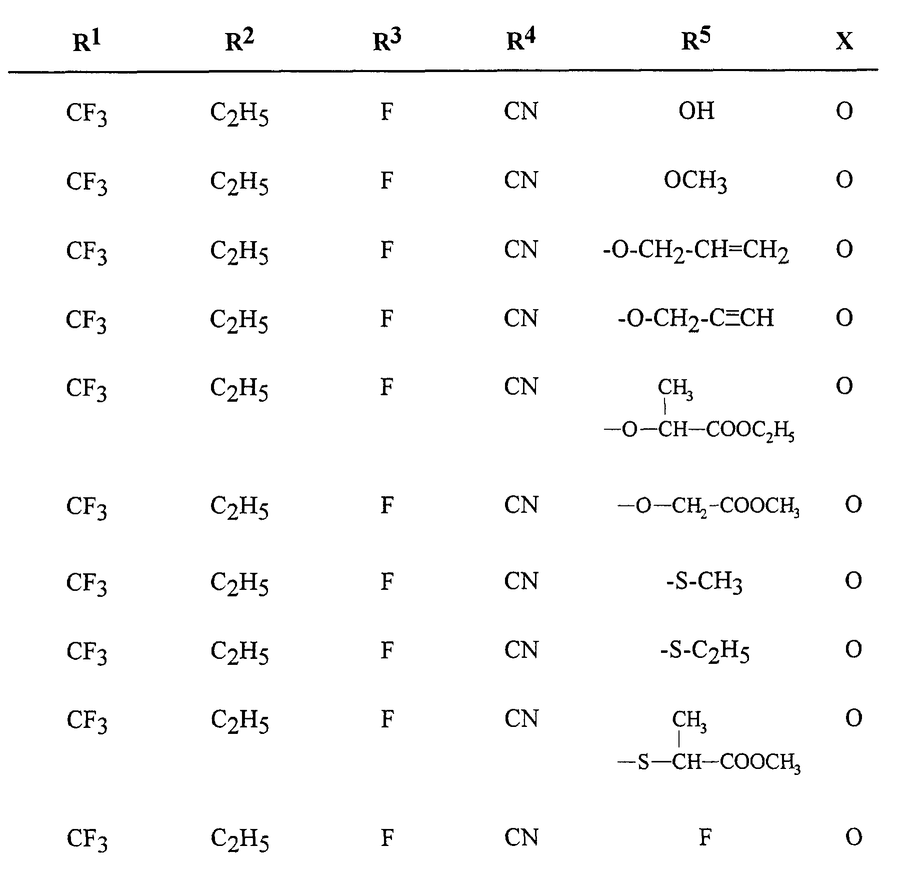

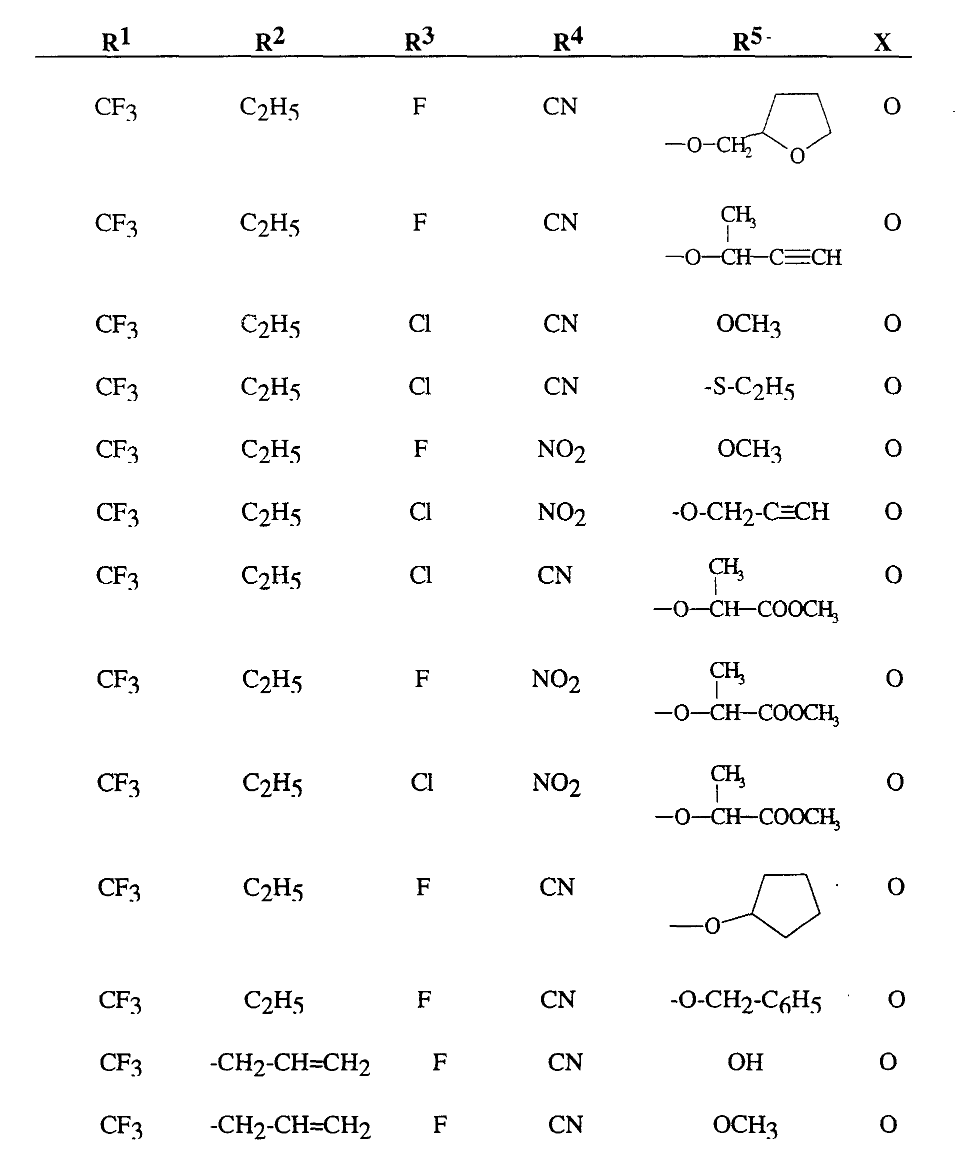

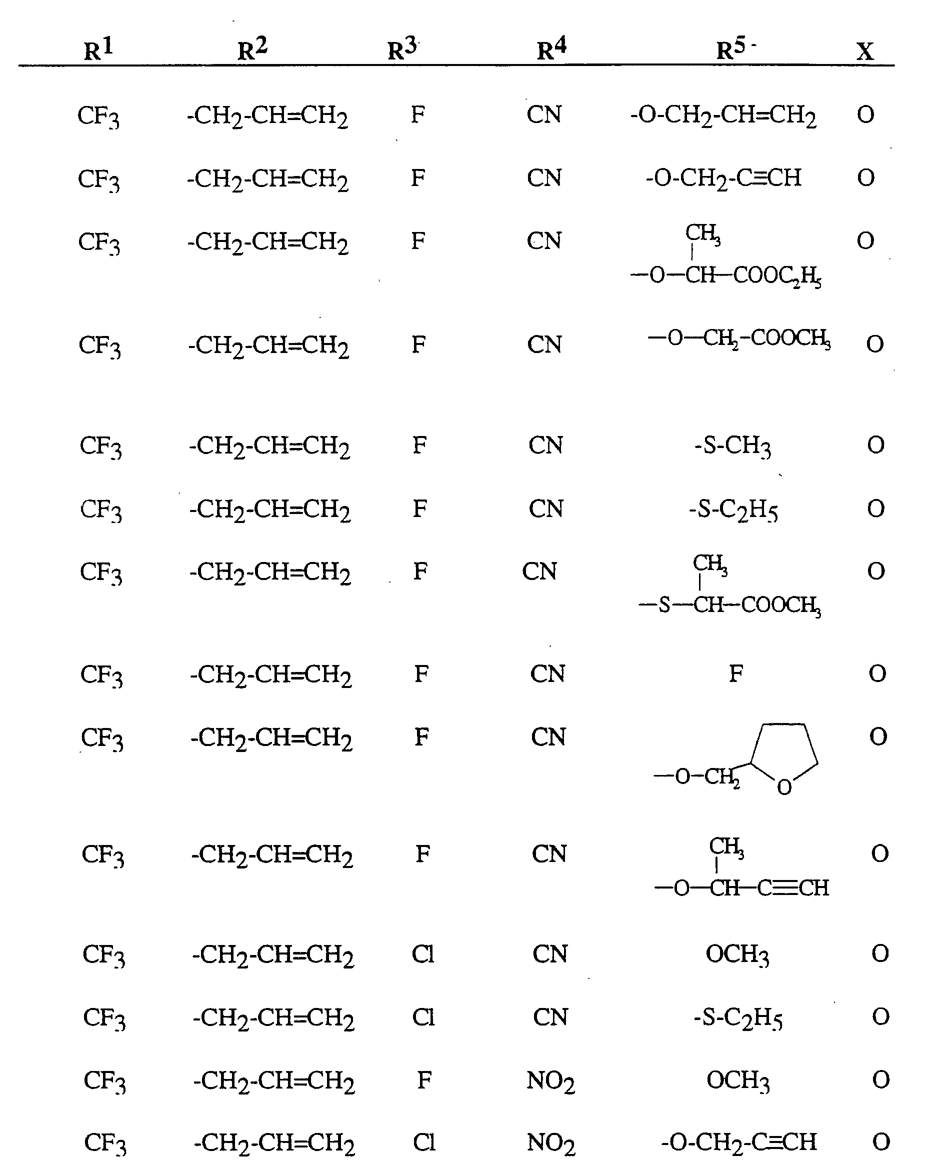

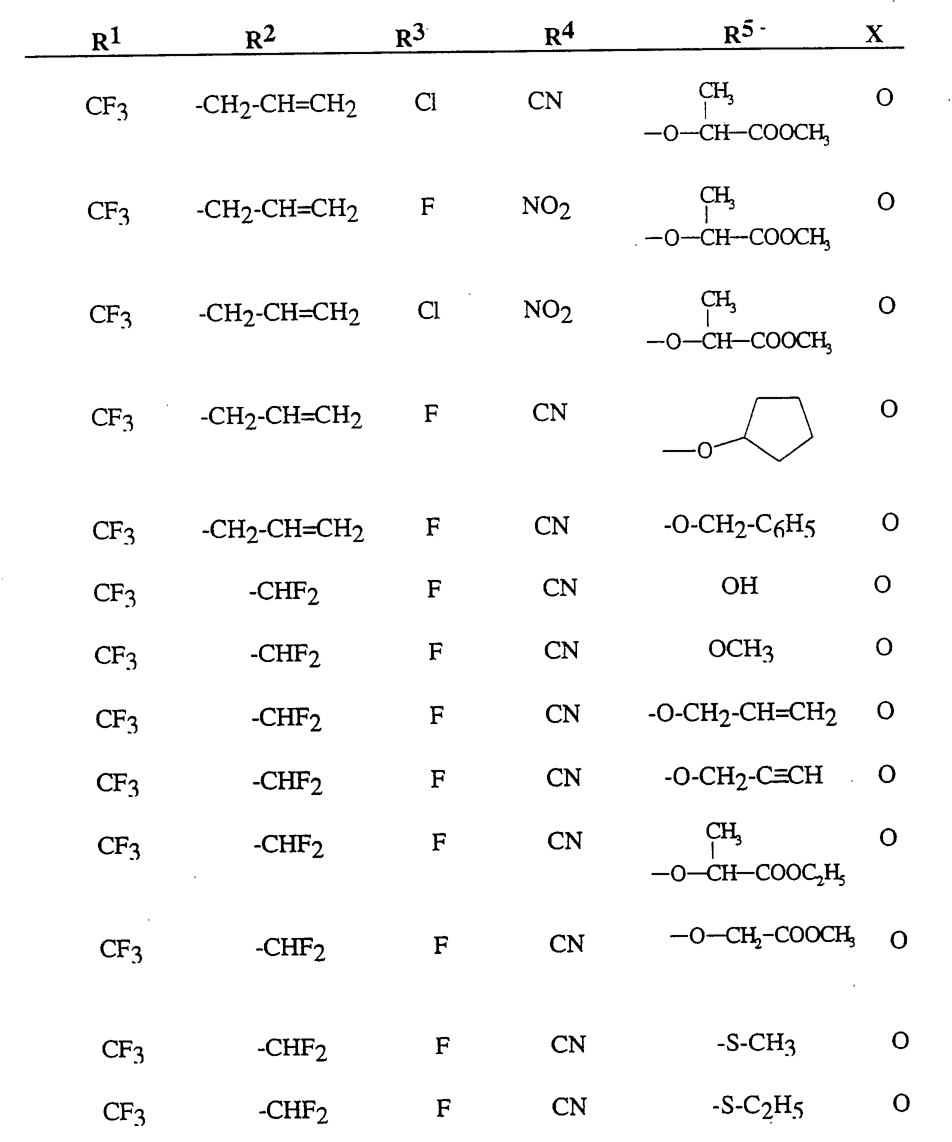

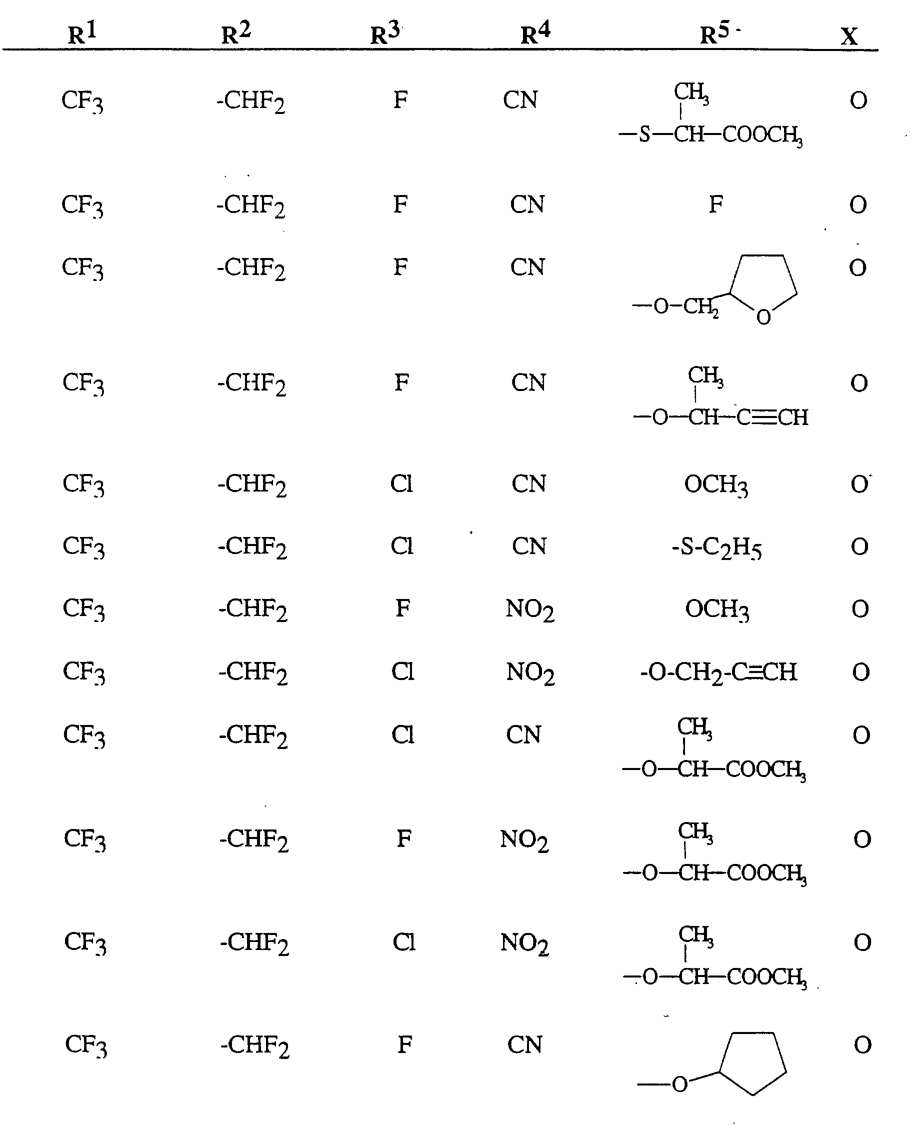

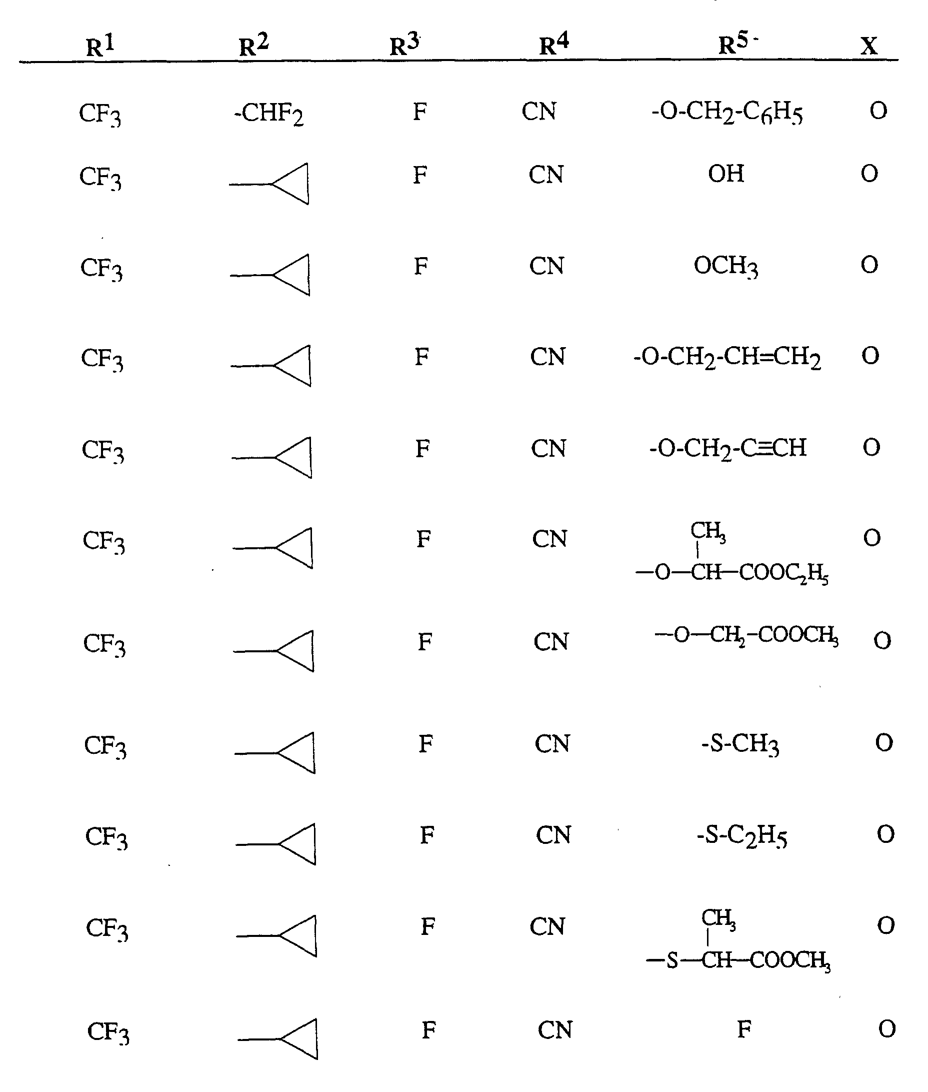

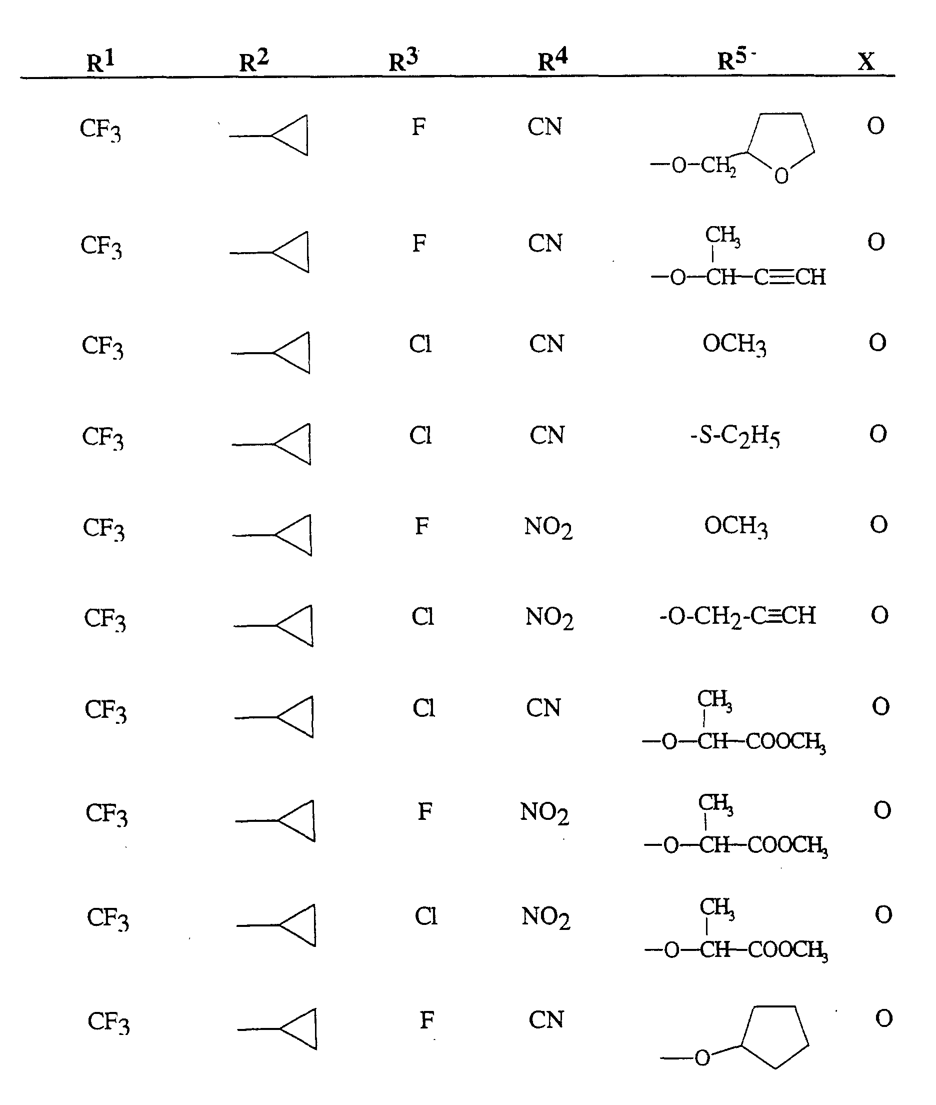

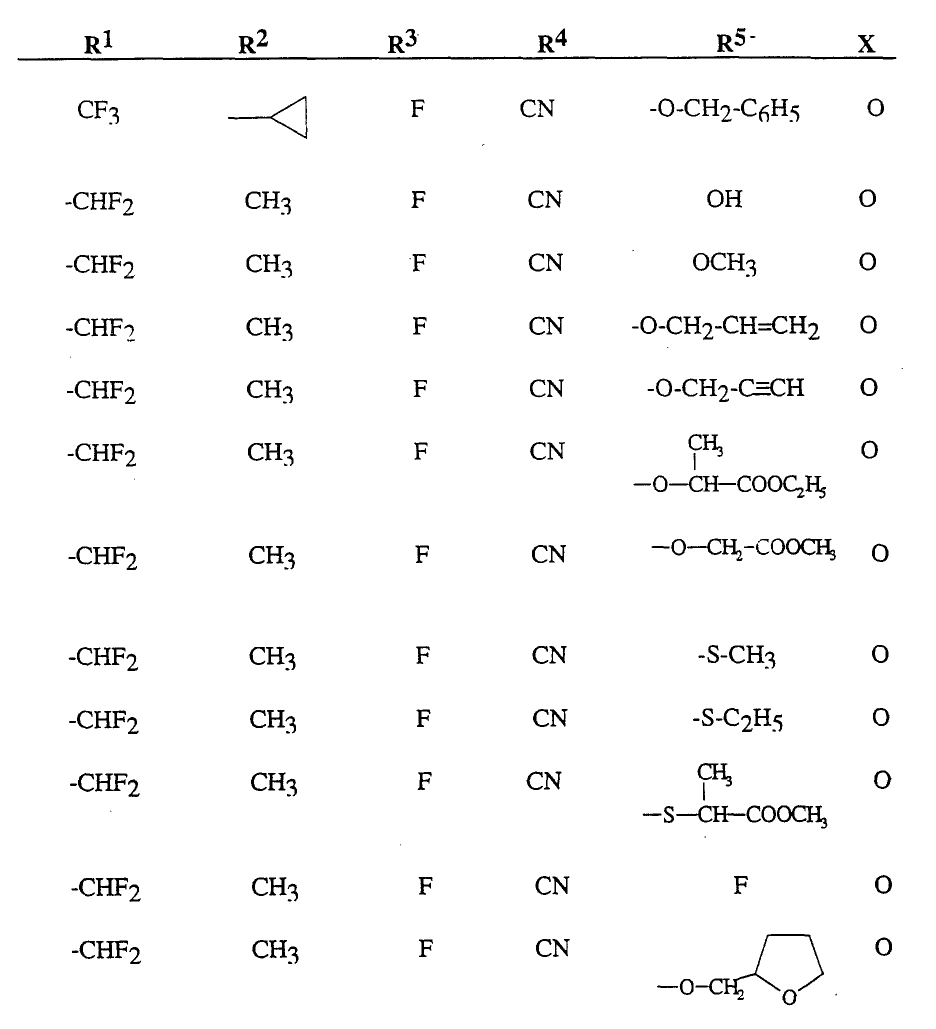

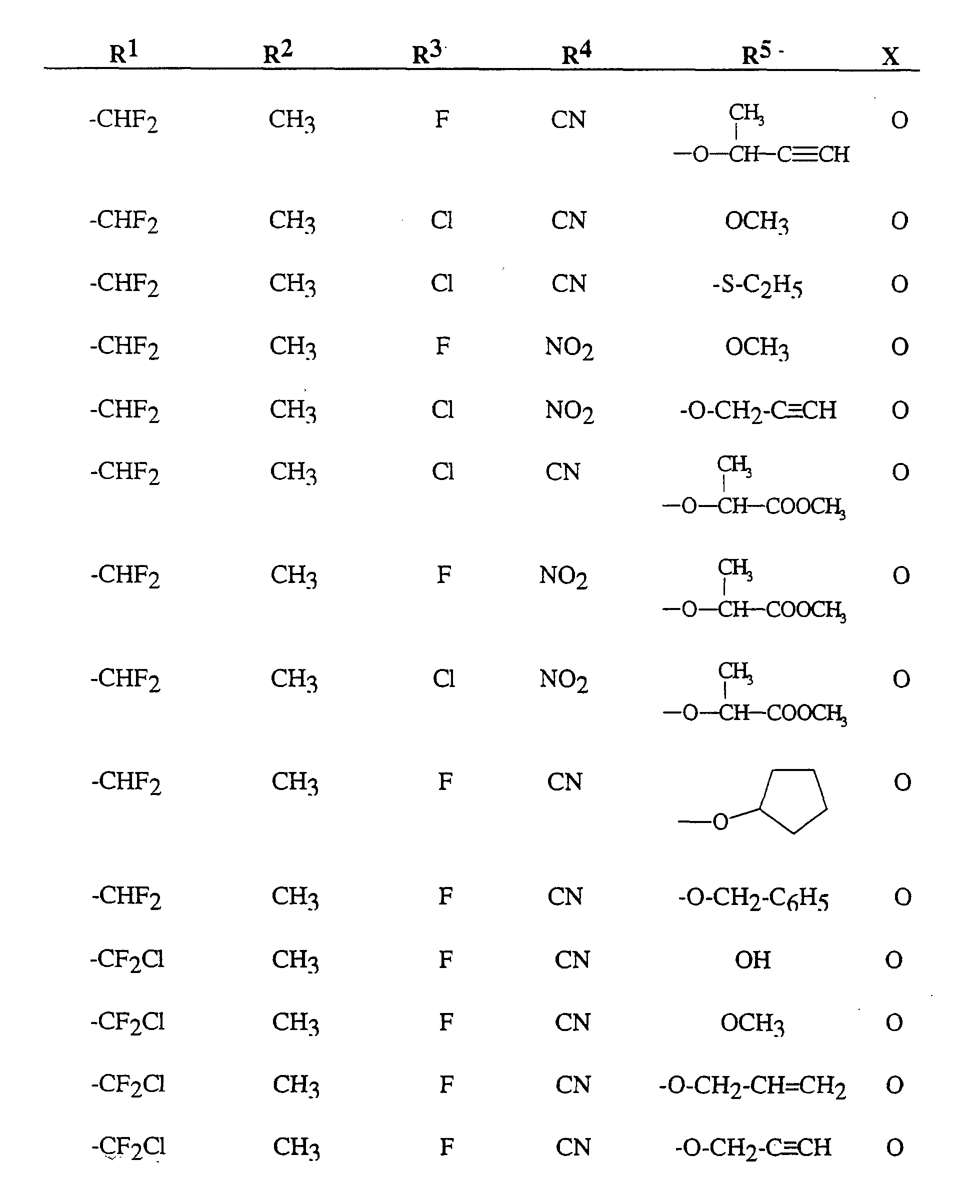

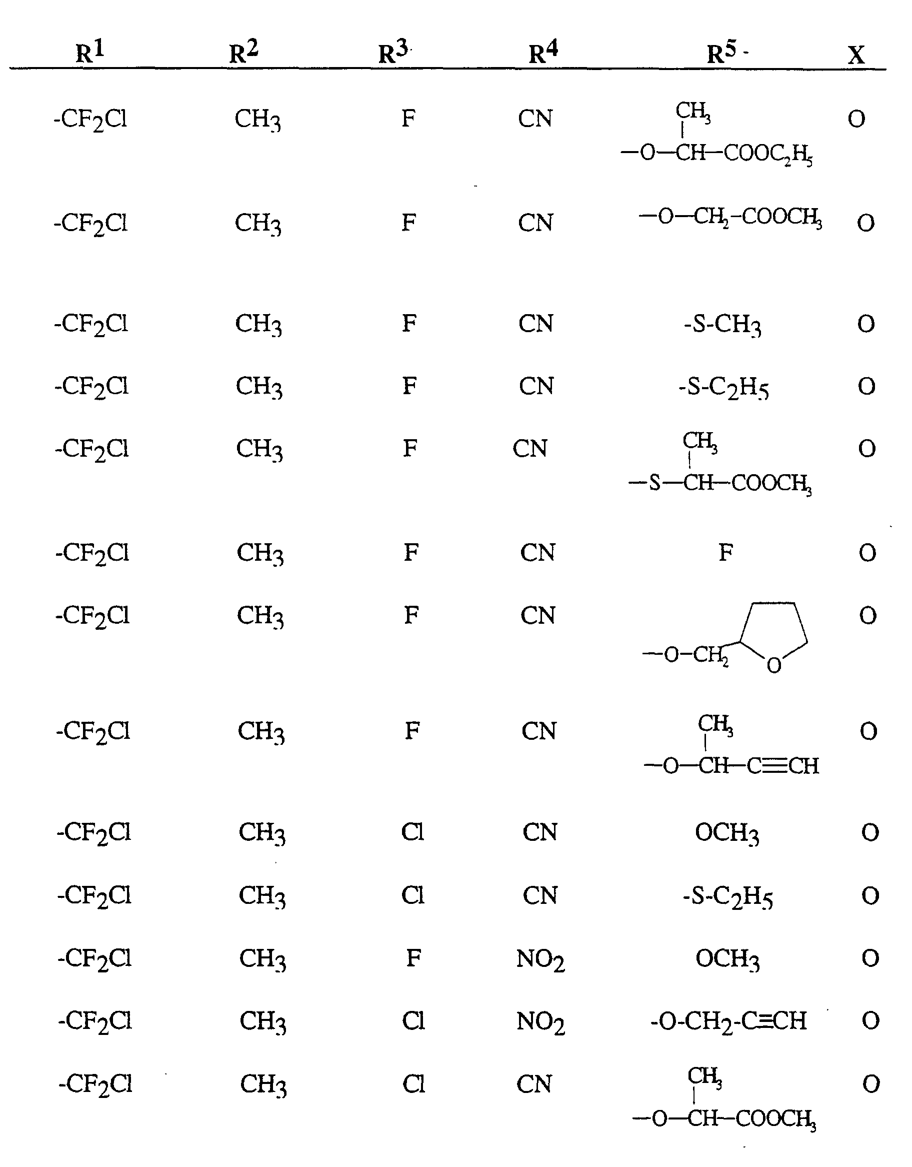

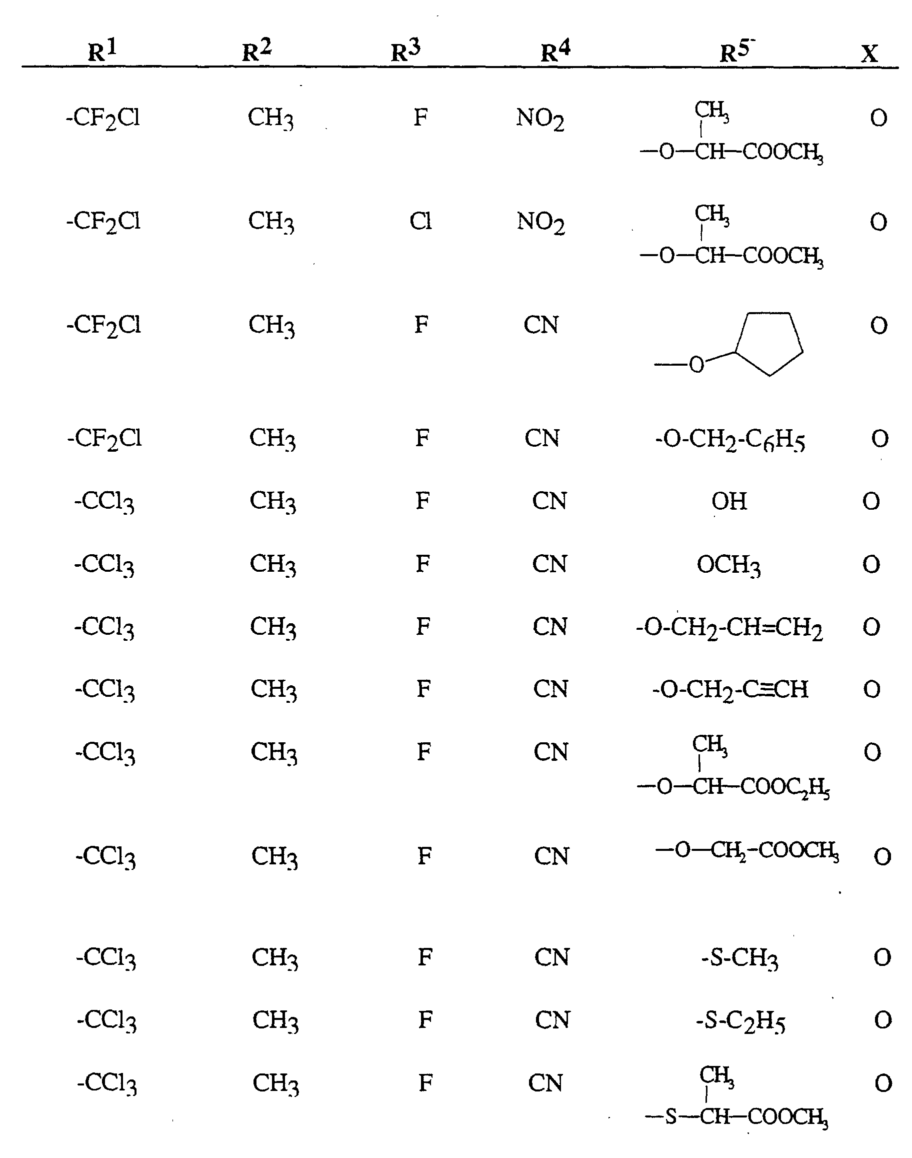

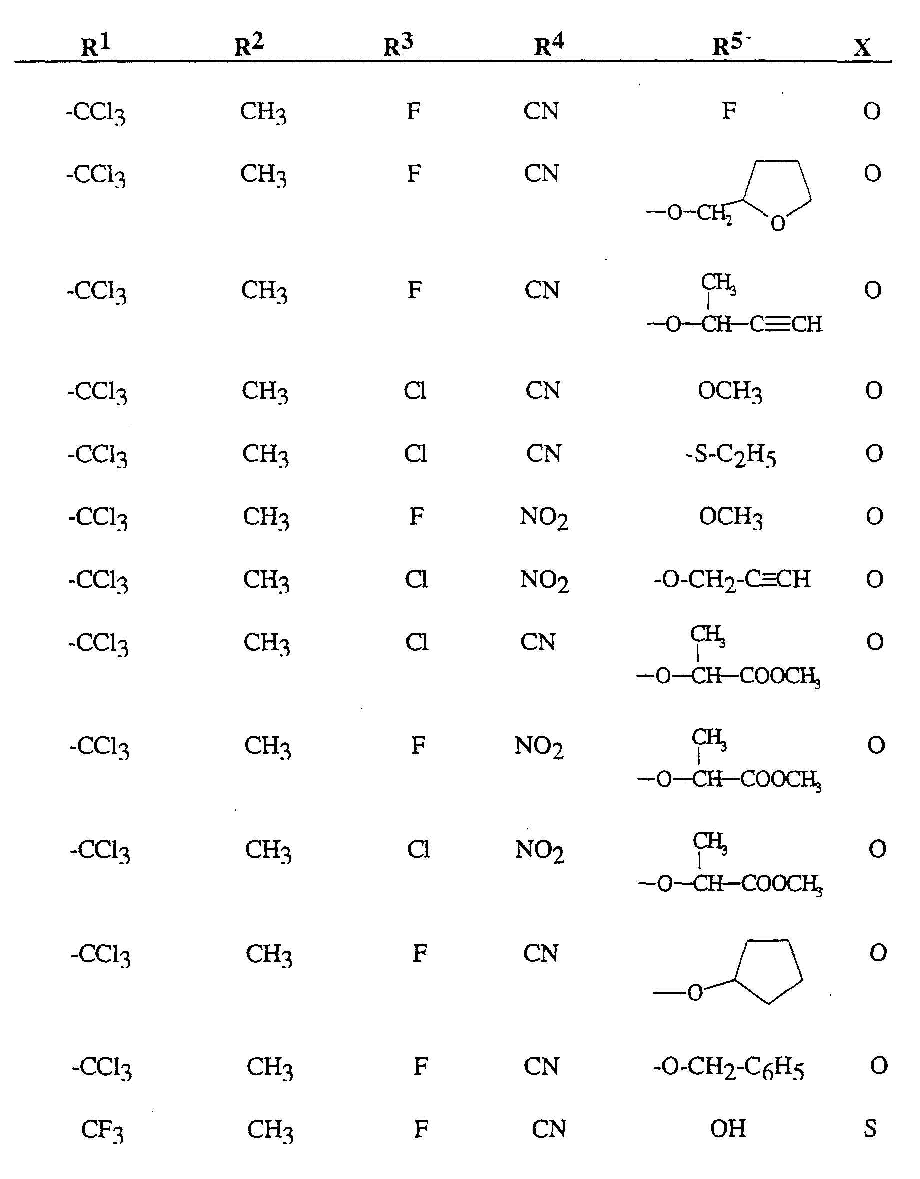

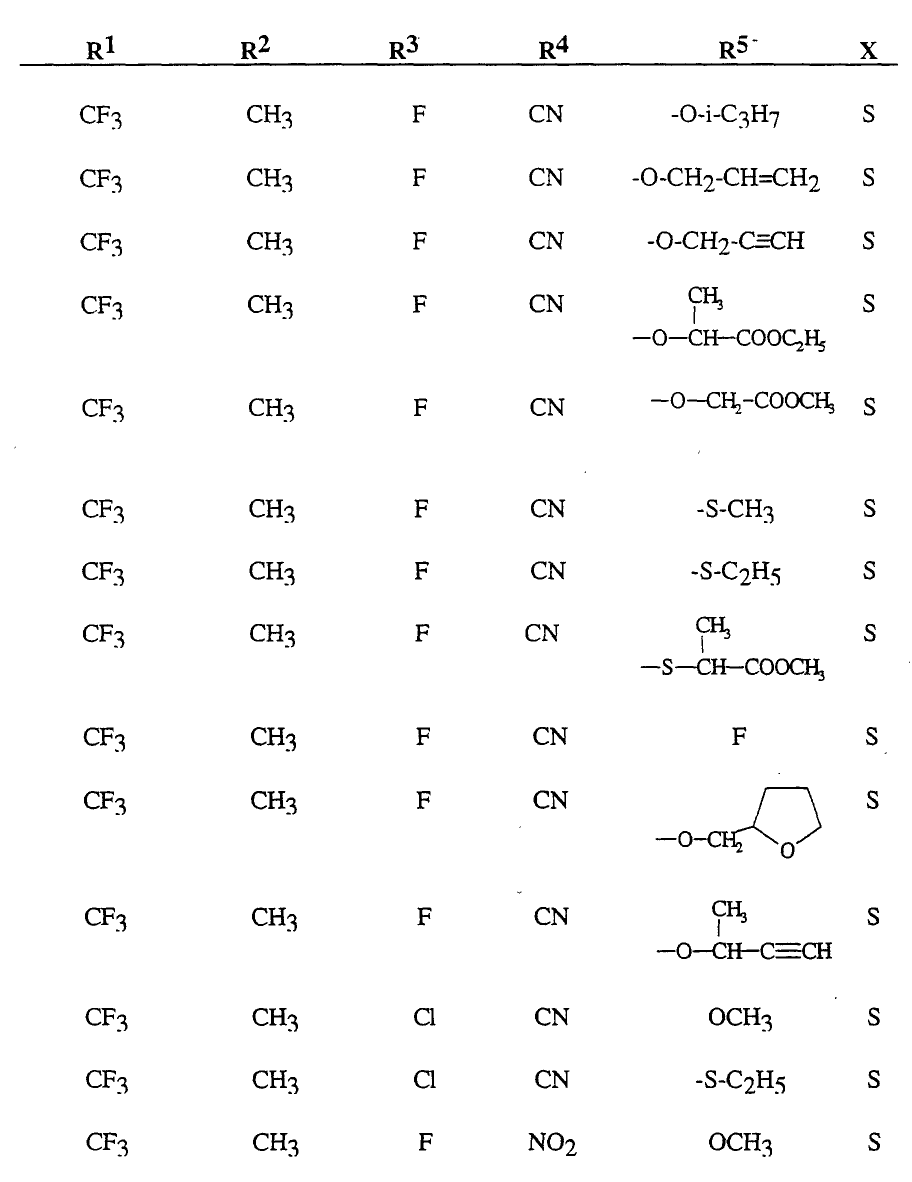

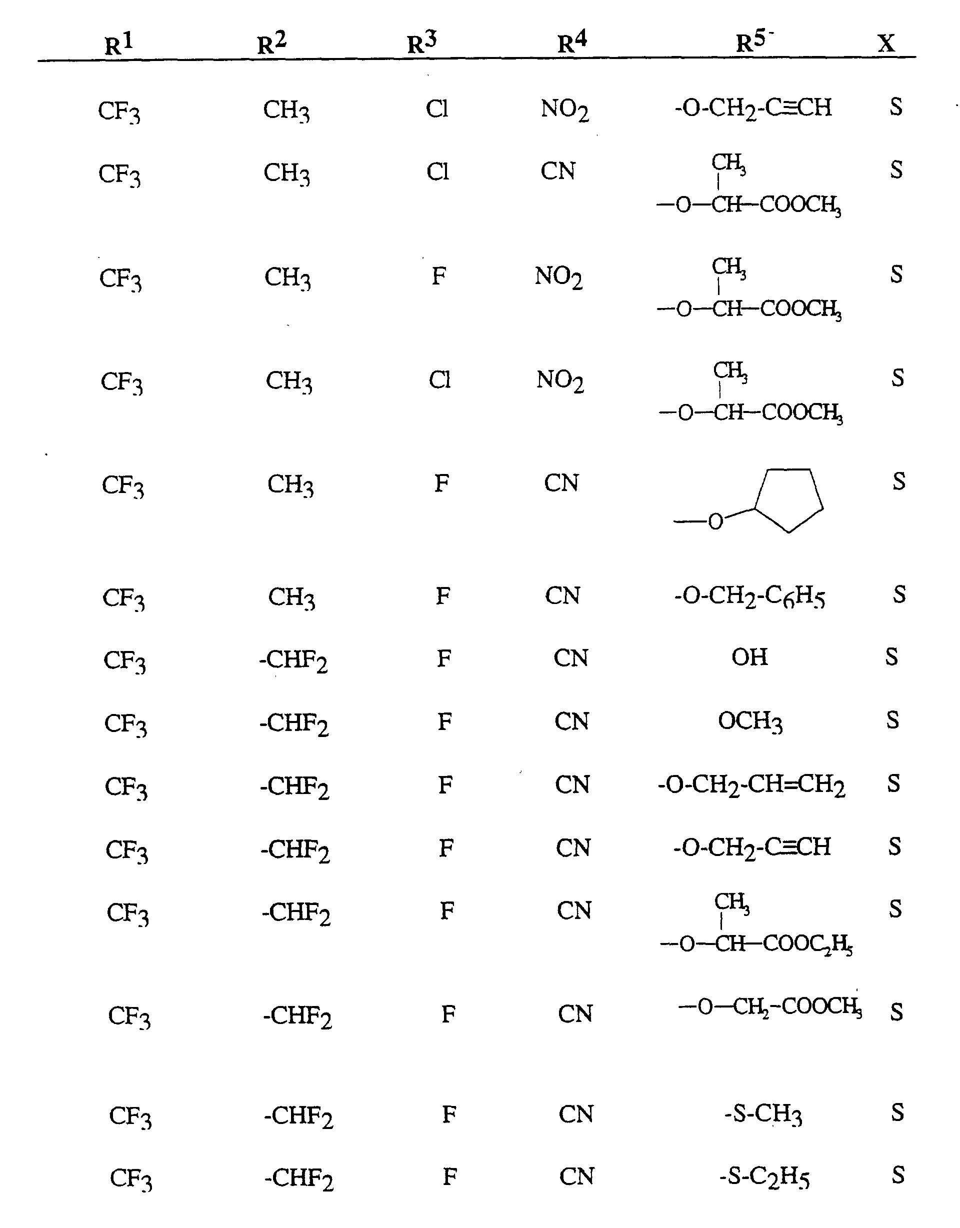

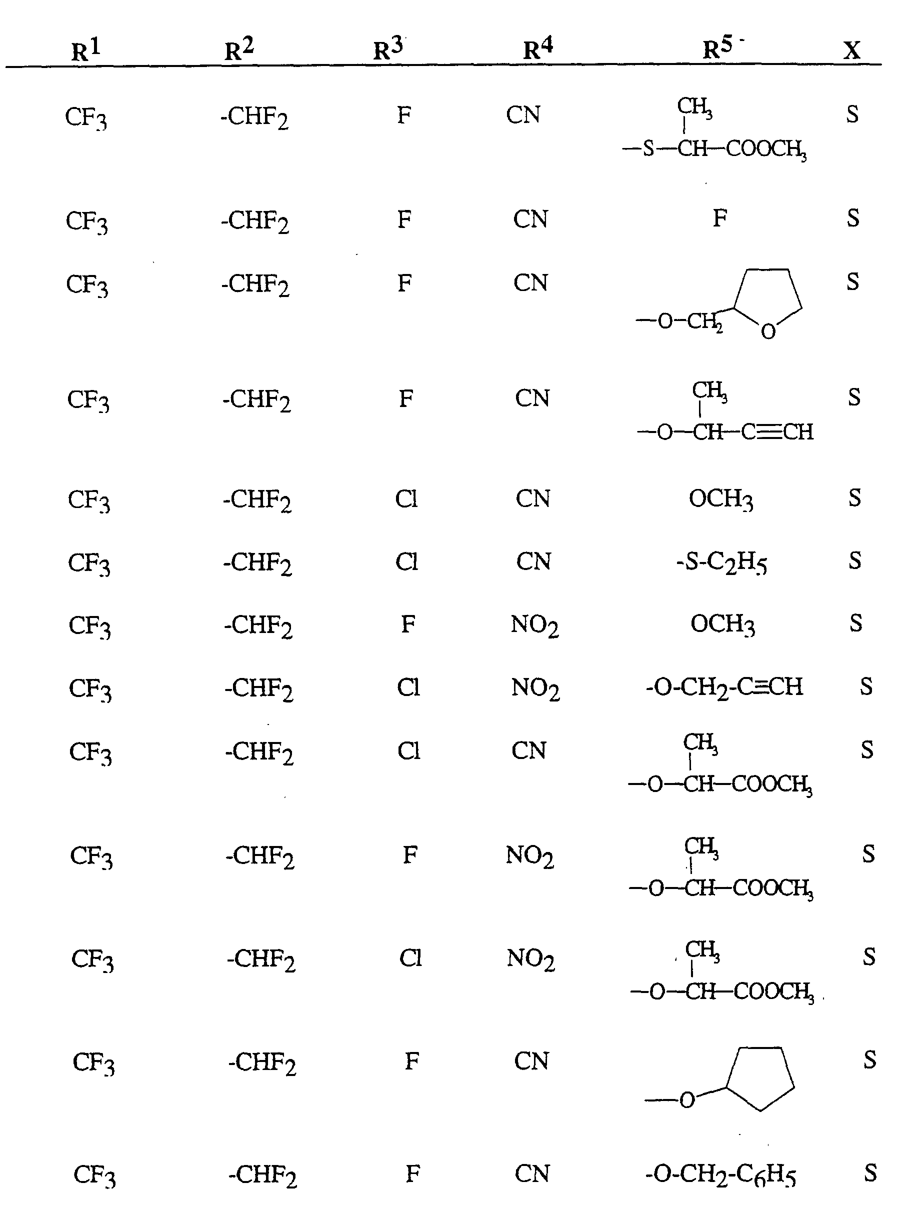

Verwendet man beispielsweise 4-Methyl-3-trifluormethyl-1,2,4-triazolin-5-on und 2,4,5-Trifluorbenzonitril als Ausgangsstoffe, so läßt sich der Reaktionsablauf des erfindungsgemäßen Verfahrens (a) durch das folgende Formelschema darstellen:

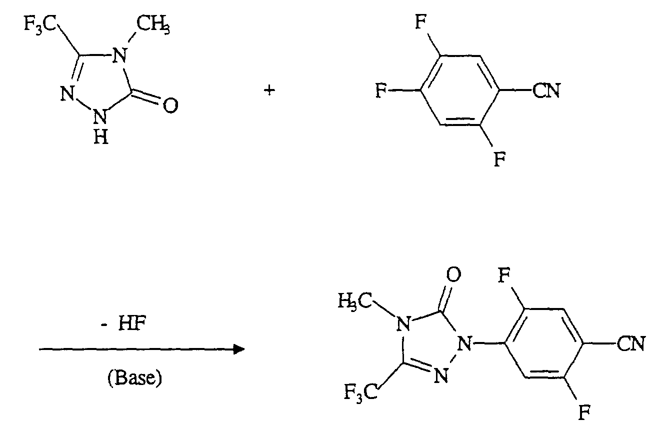

Verwendet man beispielsweise 1-(4-Cyano-2,5-difluorphenyl)-4-methyl-3-trifluormethyl-1,2,4-triazolin-5-on und 3-Butin-2-ol als Ausgangsstoffe, so läßt sich der Reaktionsablauf des erfindungsgemäßen Verfahrens (b) durch das folgende Formelschema darstellen:

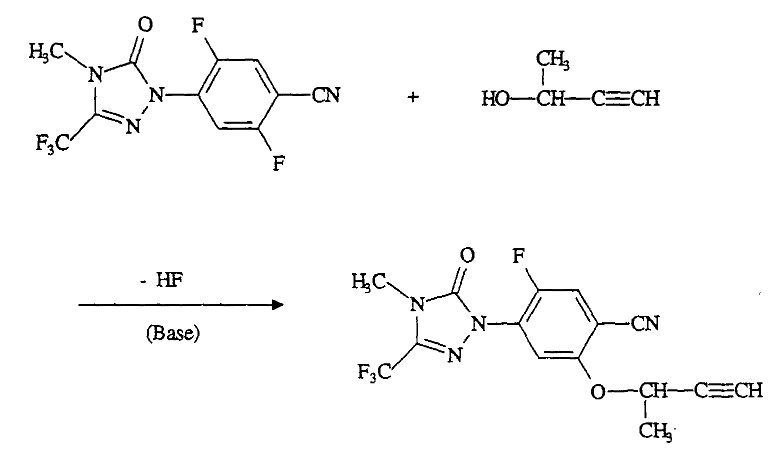

Verwendet man beispielsweise 1 -(4-Cyano-2,5-difluorphenyl)-4-amino-3-trifluormethyl-1,2,4-triazolin-5-on und Natriumnitrit als Ausgangsstoffe, so läßt sich der Reaktionsablauf des erfindungsgemäßen Verfahrens (c) durch das folgende Formelschema darstellen:

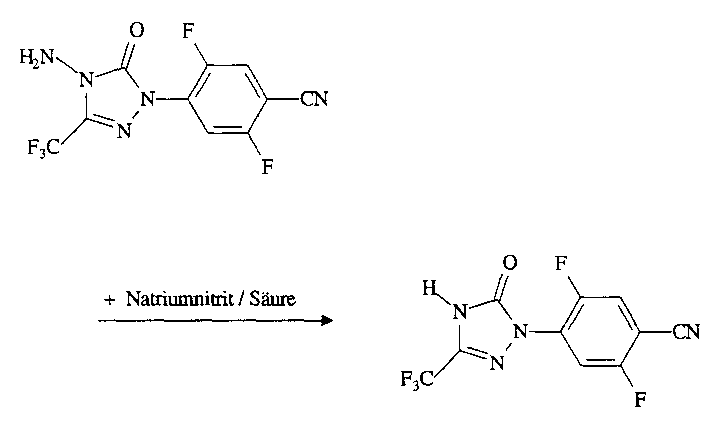

Verwendet man beispielsweise -(4-Cyano-2,5-difluorphenyl)-3-trifluormethyl-(4H)-1,2,4-triazolin-5-on und Chlordifluormethan als Ausgangsstoffe, so läßt sich der Reaktionsablauf des erfindungsgemäßen Verfahrens (d) durch das folgende Formelschema darstellen:

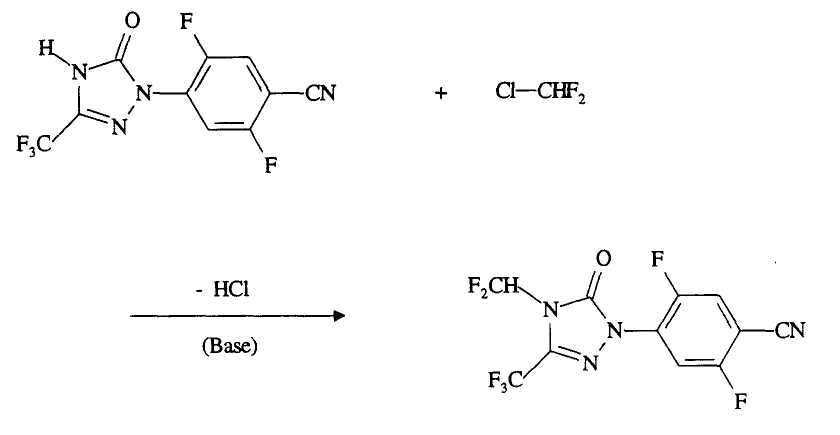

Die zur Durchführung des erfindungsgemäßen Verfahrens (a) als Ausgangsstoffe benötigten 1H-Triazolinone sind durch die Formel (II) allgemein definiert. In dieser Formel (II) stehen R¹, R² und X vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden.

Die 1H-Triazolinone der Formel (II) sind bekannt oder erhältlich in Analogie zu bekannten Verfahren (vergl. z.B. EP 399 294; US 4.477.459; DE 27 16 707; US 3.780.052; J. Med. Chem. 14, 335-338 [1971]; DE 20 29 375).

Die zur Durchführung des erfindungsgemäßen Verfahrens (a) weiterhin als Ausgangsstoffe benötigten Halogenbenzol-Derivate sind durch die Formel (III) allgemein definiert. In dieser Formel (III) stehen R³, R⁴ und R⁵ vorzugsweise und besonders bevorzugt für diejenigen Reste, die bereits im Zusammenhang mit der Beschreibung def erfindungsgemäßen Verbindungen der Formel (I) als bevorzugt und besonders bevorzugt für diese Substituenten genannt wurden. Hal steht vorzugsweise für Fluor, Chlor oder Brom, insbesondere für Fluor oder Chlor.

Die Halogenbenzol-Derivate der Formel (III) sind bekannt oder erhältlich in Analogie zu bekannten Verfahren (vergl. z.B. EP 441 004; EP 431 373).

Die zur Durchführung des erfindungsgemäßen Verfahren (b) als Edukte benötigten substituierten Triazolinone sind durch die Formel (I) R⁵⁻¹ = Halogen allgemein definiert. In dieser Formel (I) stehen R¹, R², R³, R⁴ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. R⁵⁻¹ steht vorzugsweise für Fluor, Chlor oder Brom, insbesondere für Fluor oder Chlor.

Die substituierten Triazolinone der Formel (I) mit R⁵⁻¹ = Halogen sind erfindungsgemäße Verbindungen und erhältlich mit Hilfe der erfindungsgemäßen Verfahren (a), (c) und/oder (d).

Die zur Durchführung des erfindungsgemäßen Verfahren (b) weiterhin als Edukte benötigten Nukleophile sind durch die Formel (IV) allgemein definiert. In dieser Formel (IV) steht Z vorzugsweise für Sauerstoff oder Schwefel. R⁶⁻¹ steht vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten R⁶ genannt wurden mit Ausnahme des Wasserstoff-Restes.

Die Nukleophile der Formel (IV) sind allgemein bekannte Verbindungen der organischen Chemie.

Die zur Durchführung des erfindungsgemäßen Verfahren (c) als Edukte benötigten substituierten Triazolinone sind durch die Formel (I) mit R²⁻¹ = Amino allgemein definiert. In dieser Formel (I) mit R²⁻¹ = Amino stehen R¹, R³, R⁴, R⁵ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. R²⁻¹ steht vorzugsweise für Amino.

Die substituierten Triazolinone der Formel (I) mit R²⁻¹ = Amino sind erfindungsgemäße Verbindungen und erhältlich mit Hilfe der erfindungsgemäßen Verfahren (a), (b) und/oder (d).

Die zur Durchführung des erfindungsgemäßen Verfahren (d) als Edukte benötigten substituierten Triazolinone sind durch die Formel (I) mit R²⁻² = Wasserstoff allgemein definiert. In dieser Formel (I) stehen R¹, R³, R⁴, R⁵ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.

Die substituierten Triazolinone der Formel (I) mit R²⁻² = Wasserstoff sind erfindungsgemäße Verbindungen und erhältlich mit Hilfe der erfindungsgemäßen Verfahren (a), (b) und/oder (c).

Die zur Durchführung des erfindungsgemäßen Verfahren (d) weiterhin als Edukte benötigten Alkylierungsmittel sind durch die Formel (V) allgemein definiert. In dieser Formel (V) steht R²⁻³ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten R² genannt wurden mit Ausnahme der Reste Wasserstoff, Amino, Cyano sowie Alkylidenimino. E steht vorzugsweise für einen bei Alkylierungsmitteln üblichen Abgangsrest, wie beispielsweise Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +180°C, vorzugsweise bei Temperaturen zwischen +20°C und +120°C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogenbenzol-Derivat der Formel (III) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach bekannten Verfahren (vergl. hierzu auch die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol substituiertem Triazolinon der Formel (I) mit R⁵⁻¹ = Fluor, Chlor, Brom, Iod im allgemeinen 1,0 bis 3,0 Mol, vorzugsweise 1,0 bis 1,5 Mol Nukleophil der Formel (IV) und gegebenenfalls 0,1 bis 3,0 Mol, vorzugsweise 1,0 bis 1,5 Mol Base als Reaktionshilfsmittel ein.

Die Reaktionsdurchführung, Aufarbeitung und Isolierung der Reaktionsprodukte erfolgt nach bekannten Verfahren (vergl. hierzu auch die Herstellungsbeispiele).

Das erfindungsgemäße Verfahren (c) wird üblicherweise in Gengenwart einer geeigneten Säure-durchgeführt. Als solche kommen insbesondere wässrige Mineralsäuren infrage. Mit besonderem Vorzug verwendet man verdünnte Salzsäure.

Als Verdünnungsmittel zur Durchführung des erfindungsgemäßen Verfahrens (c) kommen alle für derartigen Diazotierungsreaktionen üblichen Verdünnungsmittel in Betracht. Mit besonderem Vorzug verwendet man die als Reagenzien eingesetzten wässrigen Mineralsäuren, wie beispielsweise Salzsäure in entsprechendem Überschuß gleichzeitig als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +100°C, vorzugsweise bei Temperaturen zwischen -10°C und +80°C.

Das erfindungsgemäße Verfahren (c) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (c) setzt man pro Mol substituiertem Triazolinon der Formel (I) mit R²⁻¹ = Amino im allgemeinen 1,0 bis 3,0 Mol, vorzugsweise 1,0 bis 2,0 Mol Natriumnitrit und 1,0 bis 10,0 Mol, vorzugsweise 1,0 bis 5,0 Mol Säure ein.

Die Reaktionsdurchführung, Aufarbeitung und Isolierung der Reaktionsprodukte erfolgt nach bekannten Verfahren (vergl. hierzu auch die Herstellungsbeispiele).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Das erfindungsgemäße Verfahren (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (d) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (d) setzt man pro Mol substituiertem Triazolinon der Formel (Ic) im allgemeinen 1,0 bis 3,0 Mol, vorzugsweise 1,0 bis 2,0 Mol Alkylierungsmittel der Formel (V) und gegebenenfalls 1,0 bis 3,0 Mol, vorzugsweise 1,0 bis 2,0 Mol Base als Reaktionshilfsmittel ein.

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie (¹H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorg hum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern einsetzen.

Darüberhinaus eignen sich die Wirkstoffe auch zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Zu den oben erwähnten Schädlingen gehören:
Aus der Ordnung der Isopoda z.B. Oniscus asellus, Armadillidium vulgare, Porcellio scaber;
aus der Ordnung der Diplopoda z.B. Blaniulus guttulatus;
aus der Ordnung der Chilopoda z.B. Geophilus carpophagus, Scutigera spec.;
aus der Ordnung der Symphyla z.B. Scutigerella immaculata;
aus der Ordnung der Thysanura z.B. Lepisma saccharina;
aus der Ordnung der Collembola z.B. Onychiurus armatus;
aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria;
aus der Ordnung der Dermaptera z.B. Forficula auricularia;
aus der Ordnung der Isoptera z.B. Reticulitermes spp.;
aus der Ordnung der Anoplura z.B. Phylloxera vastatrix, Pemphigus spp., Pediculus humanus corporis, Haematopinus spp., Linognathus spp.;
aus der Ordnung der Mallophaga z.B. Trichodectes spp., Damalinea spp.;
aus der Ordnung der Thysanoptera z.B. Hercinothrips femoralis, Thrips tabaci;
aus der Ordnung der Heteroptera z.B. Eurigaster spp., Dysdercus intermedius, Piesma quadrata, Cimex lectularius, Rhodnius prolixus, Triatoma spp.;
aus der Ordnung der Homoptera z.B. Aleurodes brassicae, Bemisia tabaci, Trialeurodes vaporariorum, Aphis gossypü, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.;
aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana;
aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica;
aus der Ordnung der Hymenoptera z.B. Diprion spp., Hoplocampa spp., Lasius spp., Monomorium pharaonis, Vespa spp.;
aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa;
aus der Ordnung der Siphonaptera z.B. Xenopsylla cheopis. Ceratophyllus spp.;
aus der Ordnung der Arachnida z.B. Scorpio maurus, Latrodectus mactans;
aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus. Sie lassen sich mit besonders gutem Erfolg zur Bekämpfung der gemeinen Spinmilbe (Tetranychus urticae) einsetzen. Daneben besitzen die Wirkstoffe insbesondere blattinsektizide Eigenschaften.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können bei der Anwendung als Herbizide als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können bei der Anwendung als Herbizide sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die erfindungsgemäßen Wirkstoffe können bei der Anwendung als Insektizide und Akarizide ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können bei der Anwendung als Insektizide und Akarizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetze Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gewichtsprozent Wirkstoff, vorzugsweise zwischen 0,0001 und 1 Gewichtsprozent liegen.

Die Anwendung geschieht bei der Anwendung als Insektizide und Akarizide in einer den Anwendungsformen angepaßten üblichen Weise.

Die Herstellung und die Verwendung der erfindungsgemäßen Wirkstoffe geht aus den nachfolgenden Beispielen hervor.

### Herstellungsbeispiele:

### Beispiel 1:

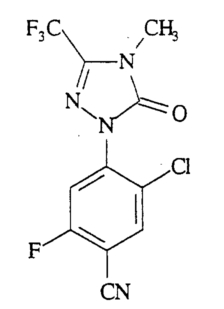

### (Verfahren a)

Zu 5,3 g (0,032 Mol) 4-Methyl-3-trifluormethyl-1,2,4-triazolin-5-on (vergl. z.B. US 3.780.052) und 5,5 g (0,032 Mol) 5-Chlor-2,4-difluorbenzonitril in 100 ml Dimethylsulfoxid gibt man bei Raumtemperatur 5,3 g (0,038 Mol) Kaliumcarbonat und erwärmt anschließend für 36 Stunden auf 100°C. Zur Aufarbeitung wird die abgekühlte Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet und im Vakuum eingeengt. Der Rückstand wird über Kieselgel (Laufmittel: Dichlormethan) chromatographiert.

Man erhält 1,8 g (18 % der Theorie) 1-(2-Chlor-4-cyano-5-fluor-phenyl)-4-methyl-3-trifluormethyl-1,2,4-triazolin-5-on vom Schmelzpunkt 105°C.

### Beispiel 2:

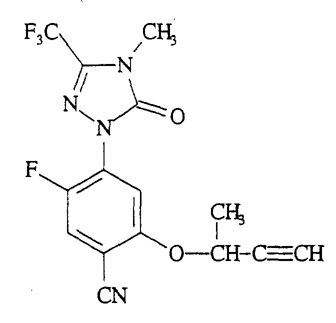

### Verfahren (b)

Zu 1,0 g (0,014 Mol) 3-Butin-2-ol in 50 ml Acetonitril gibt man bei Raumtemperatur unter Rühren 0,6 g (0,014 Mol) Natriumhydrid (60%-ig in Mineralöl), rührt 15 Minuten bei Raumtemperatur, gibt dann 2,9 g (0,01 Mol) 1-(2,5-Difluor-4-cyano-phenyl)-4-methyl-3-trifluormethyl-1,2,4-triazolin-5-on zu und rührt anschließend weitere 2 Stunden bei Raumtemperatur. Zur Aufarbeitung wird das Reaktionsgemisch im Vakuum eingeengt, der Rückstand zwischen Dichlormethan und Wasser verteilt, die organische Phase über Natriumsulfat getrocknet und im Vakuum vom Lösungsmittel befreit.

Man erhält 1,8 g (54 % der Theorie) 1-(2-Fluor-4-cyano-5-but-1-in-3-yl-oxy-phenyl)-4-methyl-3-trifluormethyl-1,2,4-triazolin-5-on vom Schmelzpunkt 41°C.

Beispiel 3:

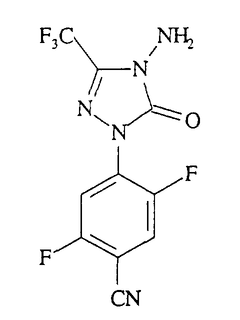

### Verfahren (a)

Zu 1,7 g (0,01 Mol) 4-Amino-3-trifluormethyl-1,2,4-triazolin-5-on und 1,6 g (0,01 Mol) 2,4,5-Trifluorbenzonitril in 30 ml Dimethylsulfoxid gibt man bei Raumtemperatur 1,7 g (0,012 Mol) Kaliumcarbonat und rührt anschließend weitere 14 Stunden bei Raumtemperatur. Zur Aufarbeitung wird die Reaktionsmischung in Wasser gegeben, mit verdünnter Salzsäure auf pH 2 gebracht und mehrfach mit Dichlormethan extrahiert. Die vereinigten organischen Phasen werden über Natriumsulfat getrocknet, im Vakuum eingeengt, der Rückstand mit Wasser verrührt, abgesaugt und getrocknet.

Man erhält 2,6 g (87 % der Theorie) 1-(2,5-Difluor-4-cyano-phenyl)-4-amino-3-trifluormethyl-1,2,4-triazolin-5-on vom Schmelzpunkt 141°C.

### Beispiel 5:

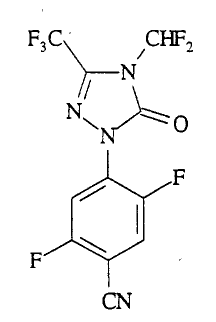

### Verfahren (d)

In eine Suspension von 2,5 g (0,009 Mol) 1-(2,5-Difluor-4-cyanophenyl)-3-trifluormethyl-4H-1,2,4-triazolin-5-on, 1,0 g (0,017 Mol) Kaliumhydroxid und 0,25 g Tetrabutylammoniumbromid in 50 ml Tetrahydrofuran leitet man bei 0°C bis 10°C innerhalb von 5 Stunden 15 g (0,17 Mol) Chlordifluormethan ein und kompensiert in dieser Zeit jeweils nach 1, 2 und 3 Stunden den Basen-Verbrauch durch Zugabe von jeweils weiteren 1,0 g (0,017 Mol) Kaliumhydroxid. Zur Aufarbeitung gibt man die Reaktionsmischung in Wasser, extrahiert mehrfach mit Essigester, trocknet die vereinigten organischen Phasen über Natriumsulfat und entfernt anschließend das Lösungsmittel im Vakuum. Der Rückstand wird über Kieselgel (Laufmittel: Dichlormethan) chromatographiert.

Man erhält 2,2 g (75 % der Theorie) 1-(2,5-Difluor-4-cyanophenyl)-3-trifluormethyl-4-difluormethyl-1,2,4-triazolin-5-on vom Schmelzpunkt 68°C.

### Herstellung der Ausgangsverbindungen:

### Beispiel II-1:

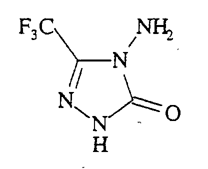

Zu 1.300 g (26 Mol) Hydrazinhydrat gibt man unter Rühren und Eiskühlung im Verlauf von 2 Stunden portionsweise 2.782 g (13 Mol) Diphenylcarbonat, so daß die Temperatur der Reaktionsmischung 30°C nicht übersteigt, anschließend rührt man 2 Stunden bei 80°C , kühlt dann wieder ab und gibt ebenfalls portionsweise 3.164 g (26 Mol) Trifluoressigsäure zu. danach wird abermals 2 Stunden bei 80°C gerührt und anschließend Wasser abdestilliert bis der Rückstand eine temperatur von 180°C erreicht hat. Nach dem Abkühlen gibt man 1.100 g (16,2 Mol) wässrigen Ammoniak (25%-ig) zu und erhitzt für 3 Stunden auf Rückflußtemperatur. Zur Aufarbeitung werden alle flüchtigen Bestandteile bis zu einer Rückstandstemperatur von 180°C bei allmählich vermindertem Druck (bis 20 mbar) abdestilliert und der Rückstand aus 2.000 ml Wasser umkristallisiert, abgesaugt und getrocknet.

Man erhält 702 g (32 % der Theorie) 3-Trifluormethyl-4-amino-1H-1,2,4-triazolin-5-on vom Schmelzpunkt 163°C.

### Beispiel 4:

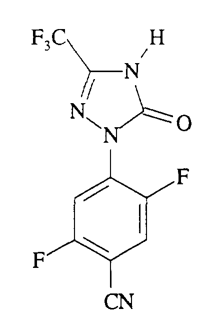

### Verfahren (c)

Zu 3,0 g (0,01 Mol) 1-(2,5-Difluor-4-cyano-phenyl)-4-amino-3-trifluormethyl-1,2,4-triazolin-5-on in 40 ml 10%-iger Salzsäure gibt man bei -5°C bis 0°C innerhalb von 15 Minuten unter Rühren eine gesättigte wässrige Lösung von 1,4 g (0,02 Mol) Natriumnitrit, entfernt anschließend das Kältebad, rührt eine Stunde bei Raumtemperatur, kühlt dann erneut auf -5°C bis 0°C ab, filtriert, wäscht den Rückstand mit Wasser und trocknet ihn.

Man erhält 1,8 g (62 % der Theorie) 1-(2,5-Difluor-4-cyano-phenyl)-3-trifluormethyl-1,2,4-triazolin-5-on vom Schmelzpunkt 51°C.

### Beispiel III-1:

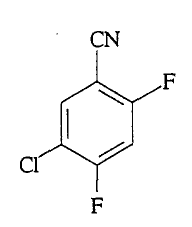

Zu 250 g (4,31 Mol) Kaliumfluorid in 400 ml destilliertem Tetramethylensulfon gibt man unter Rühren bei Raumtemperatur 220 g (1,06 Mol) 2,4,5-Trichlorbenzonitril (vergl. z.B. EP 441 004) und rührt anschließend 10 Stunden bei 195°C bis 200°C. Zur Aufarbeitung wird abgekühlt, 500 ml Wasser zugegeben und die Mischung einer Wasserdampfdestillation unterzogen. Der organische Anteil wird in Dichlormethan aufgenommen, über Natriumsulfat getrocknet, im Vakuum eingeengt und destilliert.

Man erhält 108 g (58 % der Theorie) 2,4-Dfliuor-5-chlorbenzonitril vom Siedepunkt 105-107°C bei 30 mbar und vom Schmelzpunkt 48-50°C.

In entsprechender Weise und gemäß den allgemeinen Angaben zur Herstellung erhält man die folgenden substituierten Triazolinone der allgemeinen Formel (I):

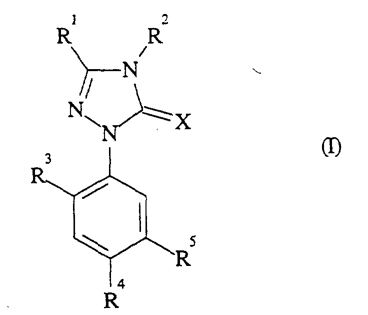

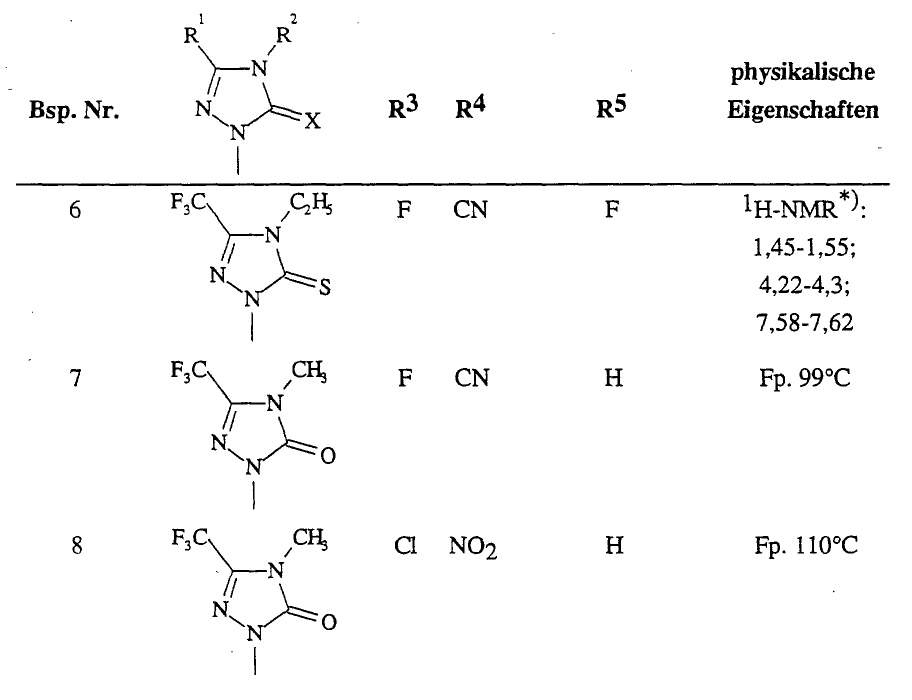

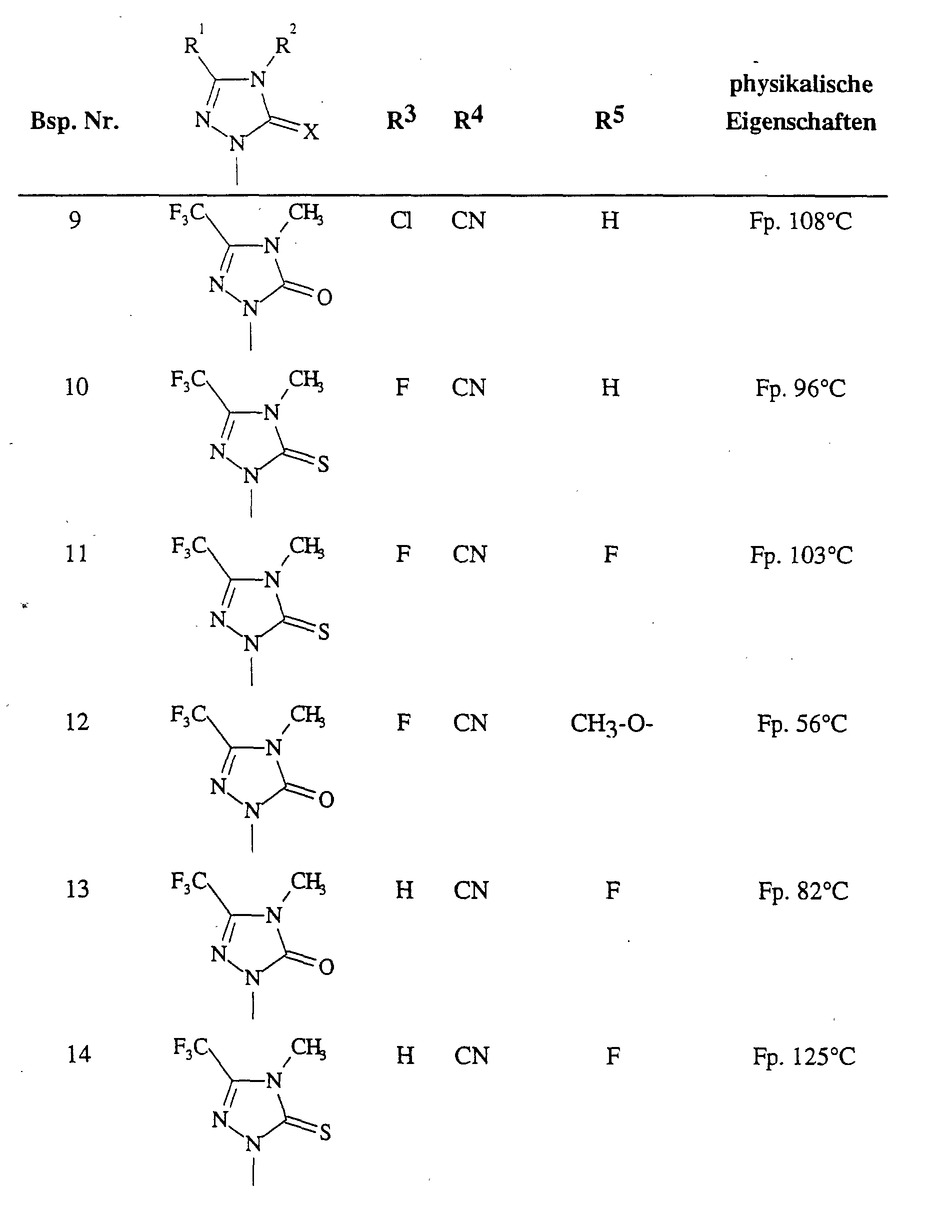

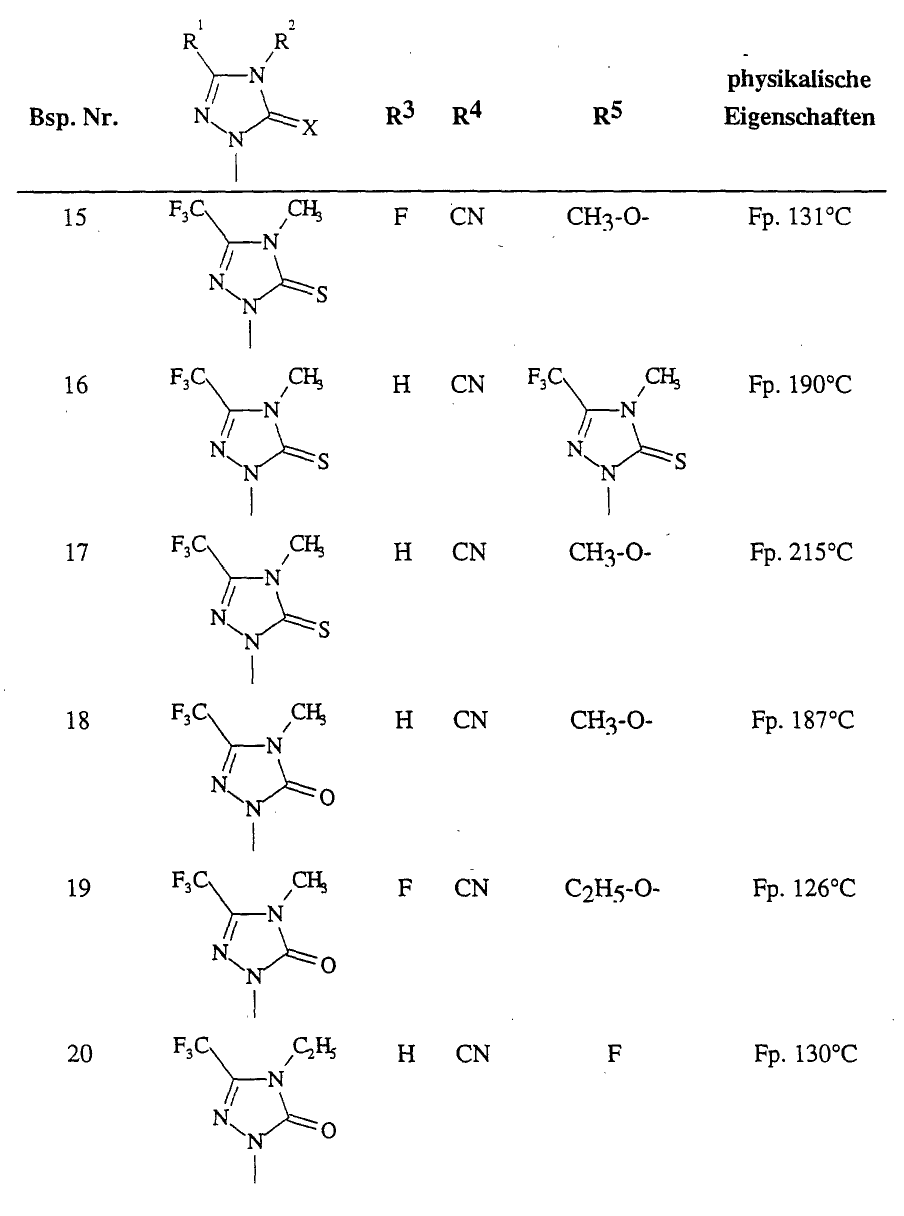

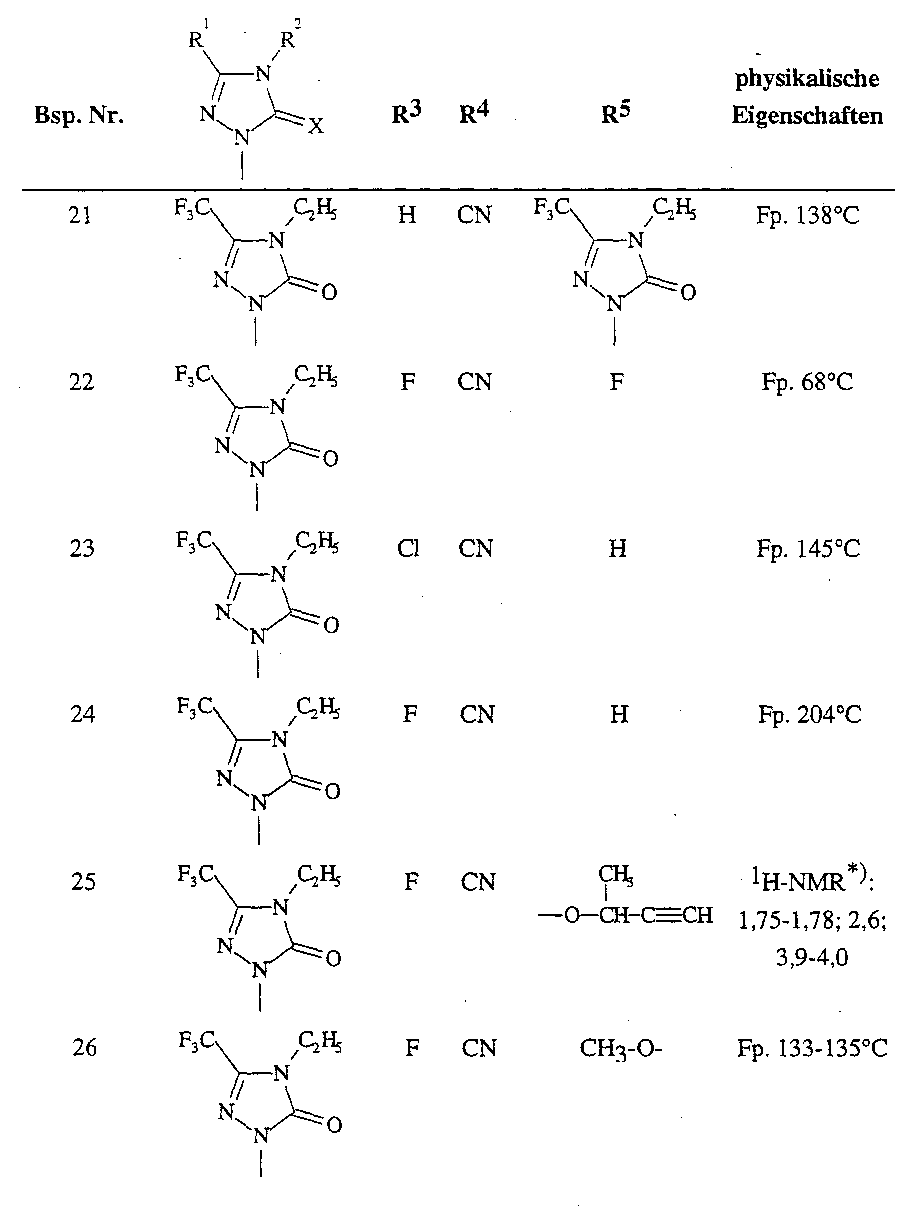

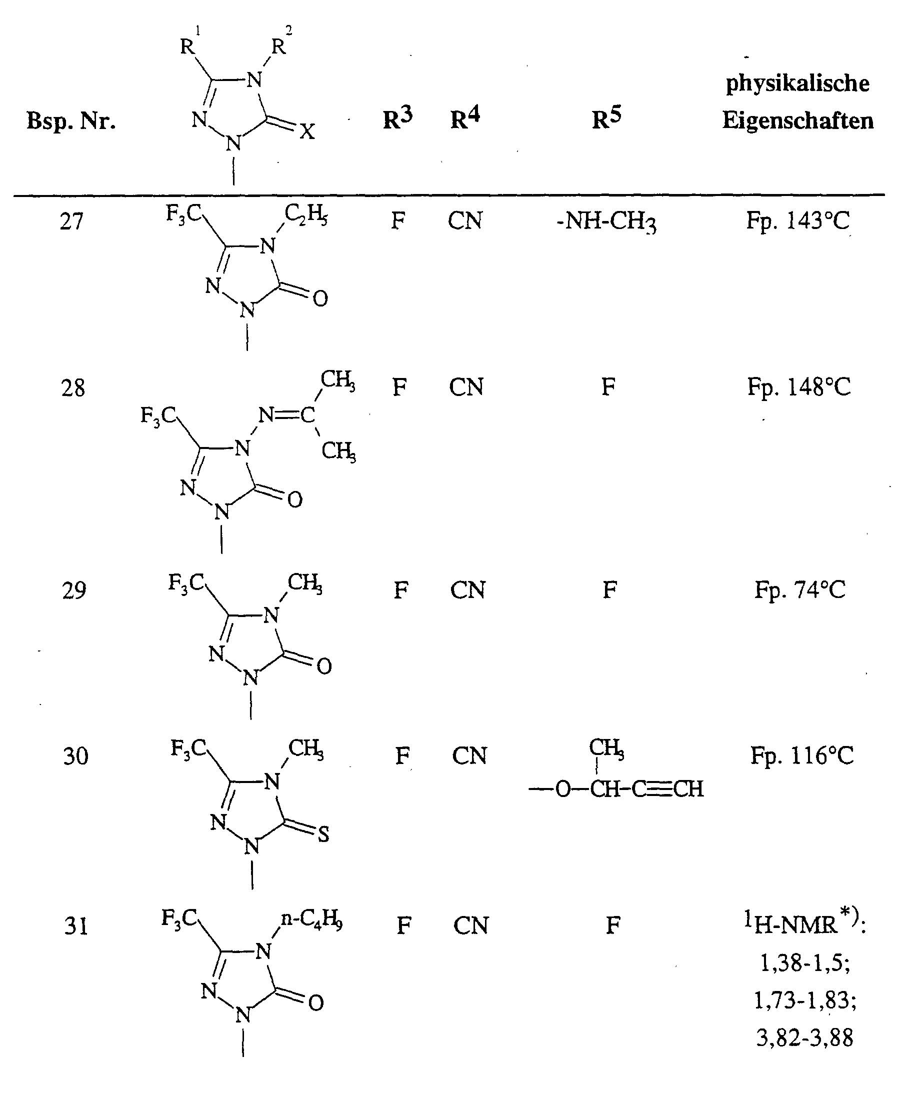

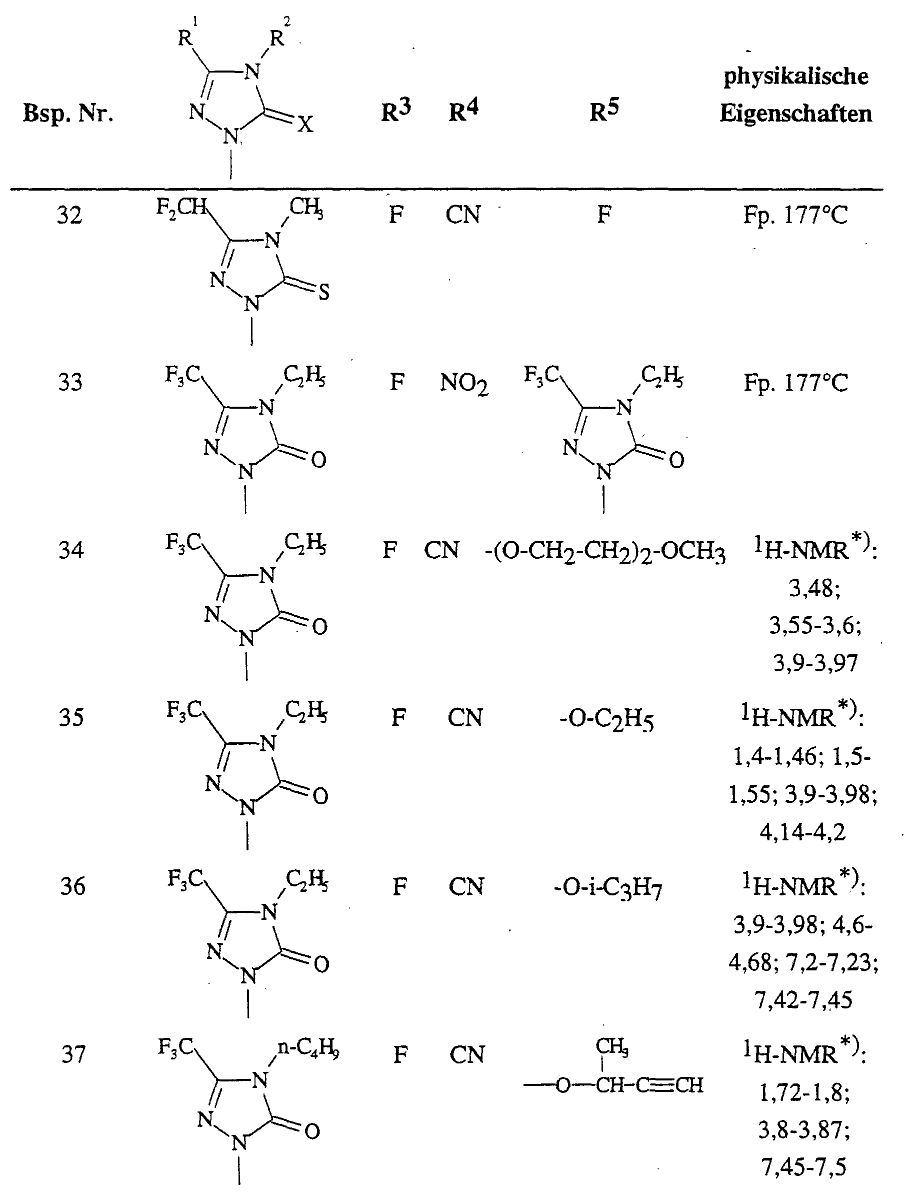

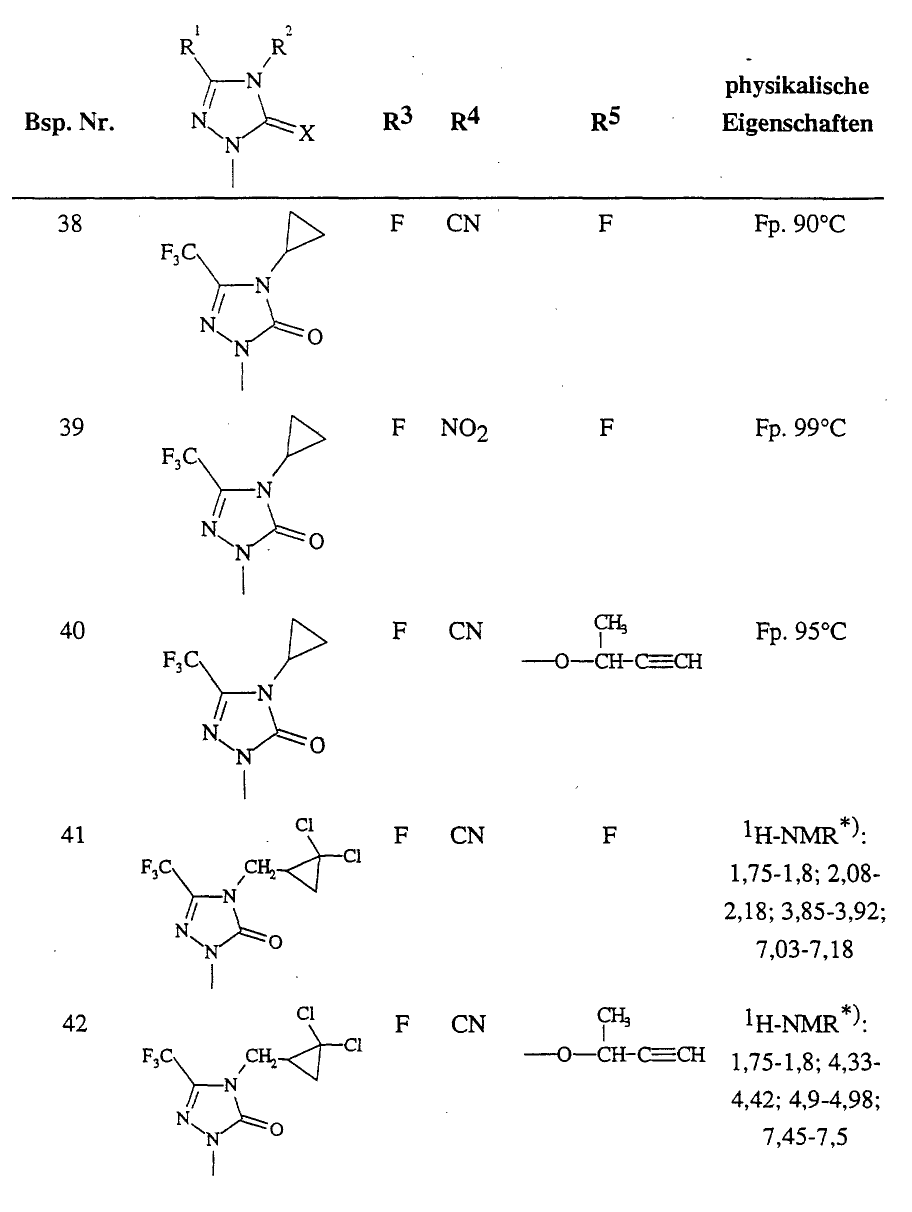

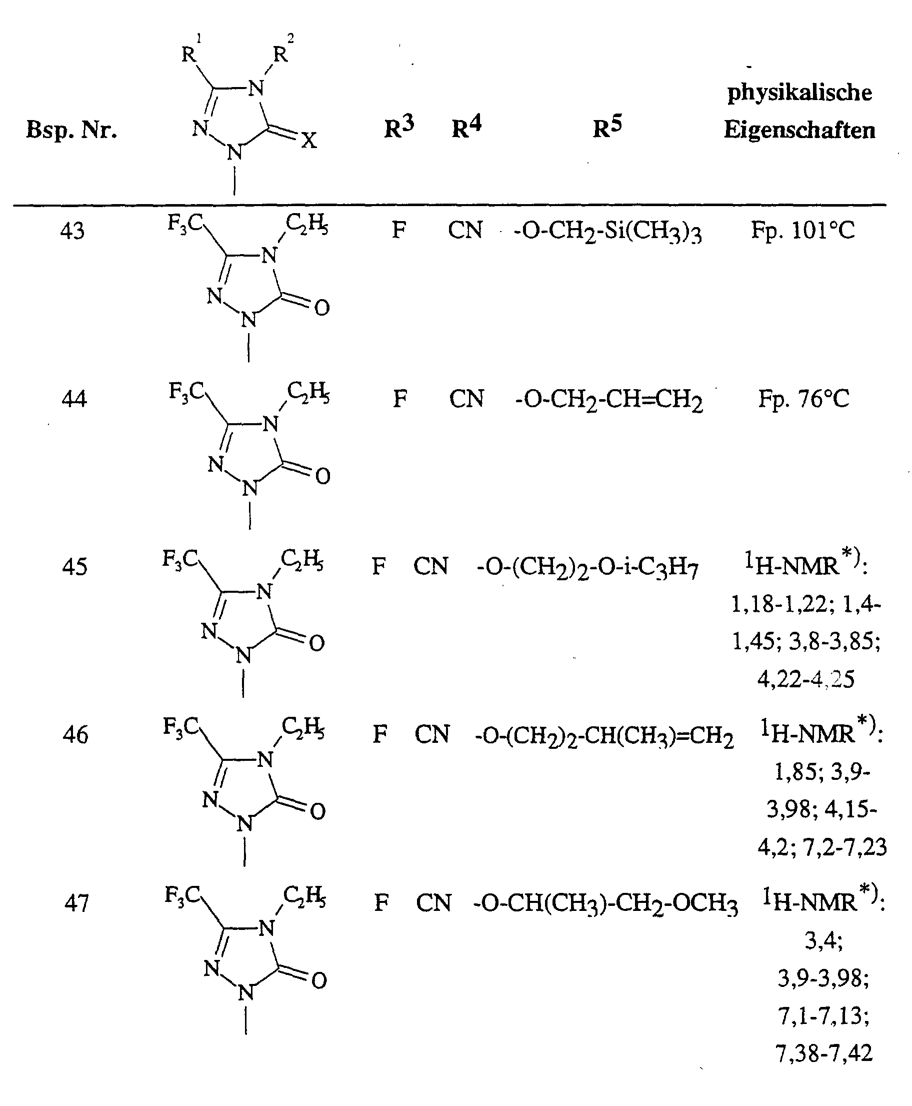

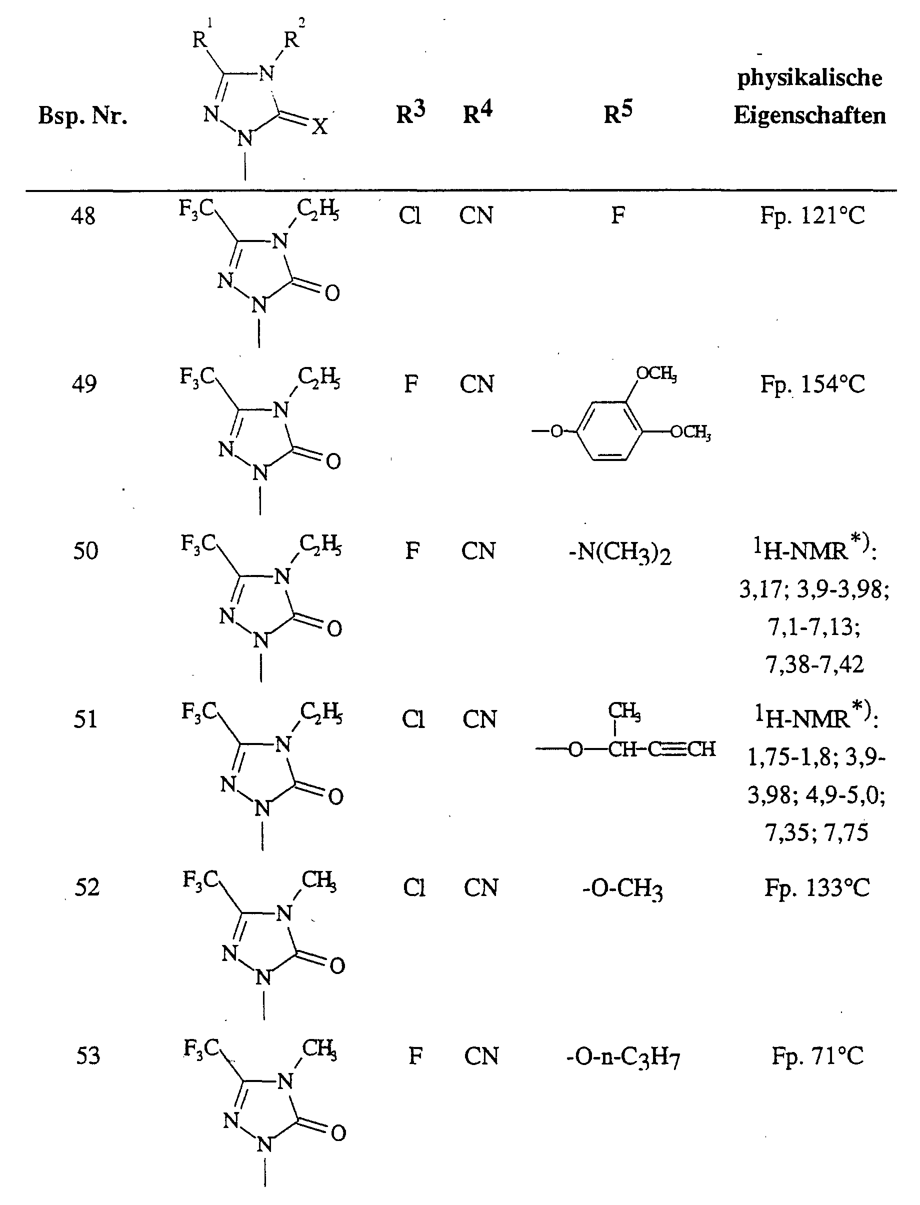

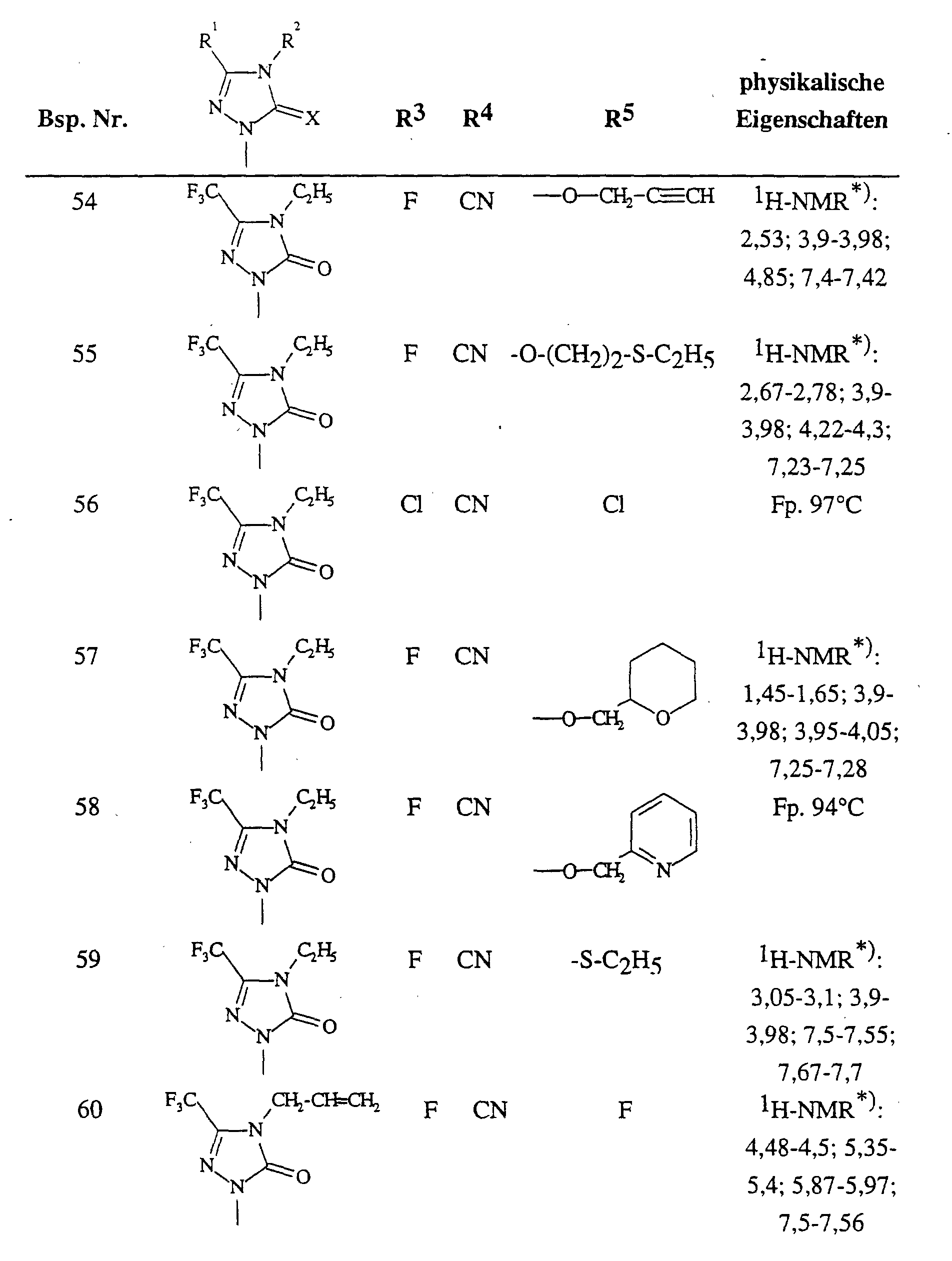

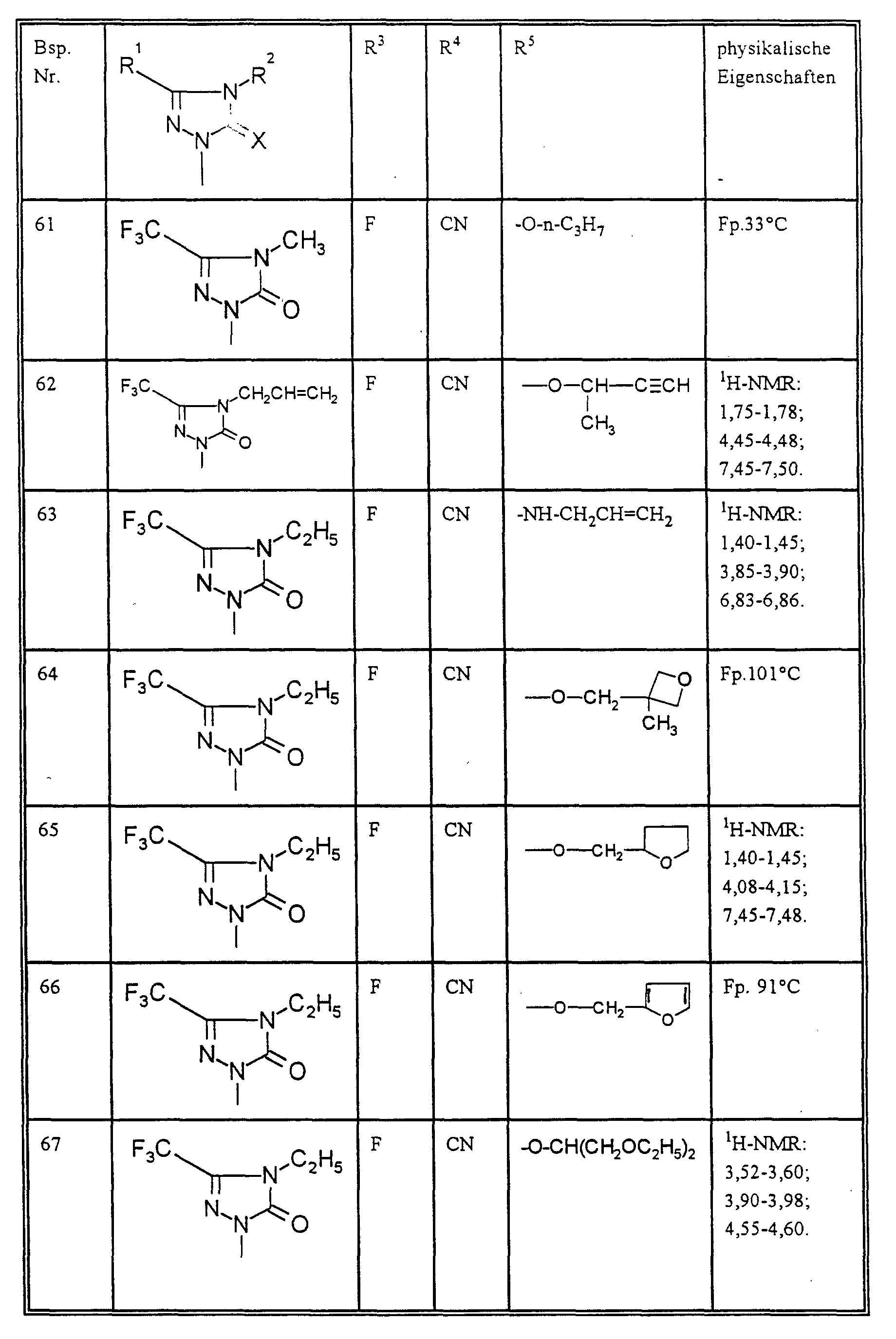

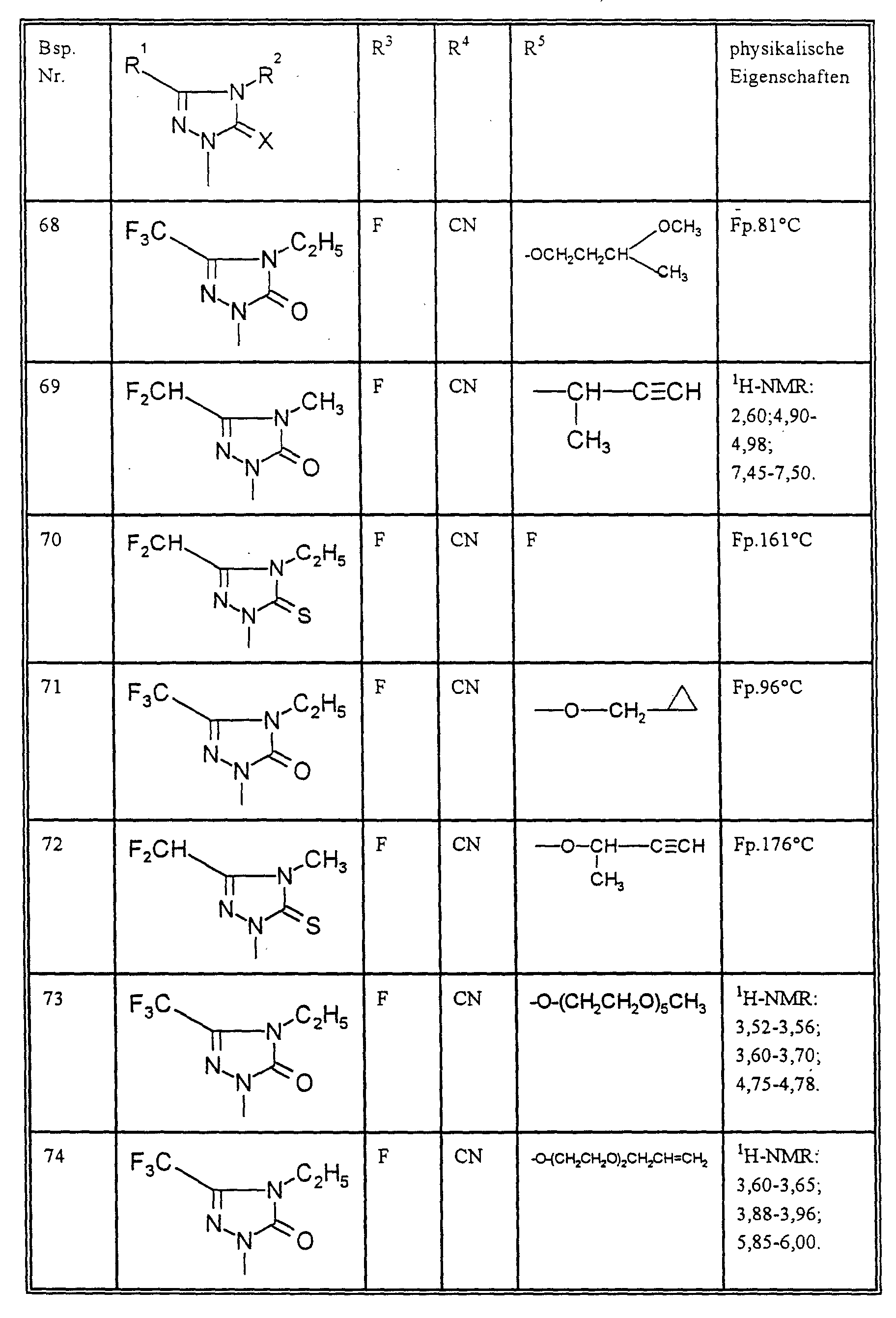

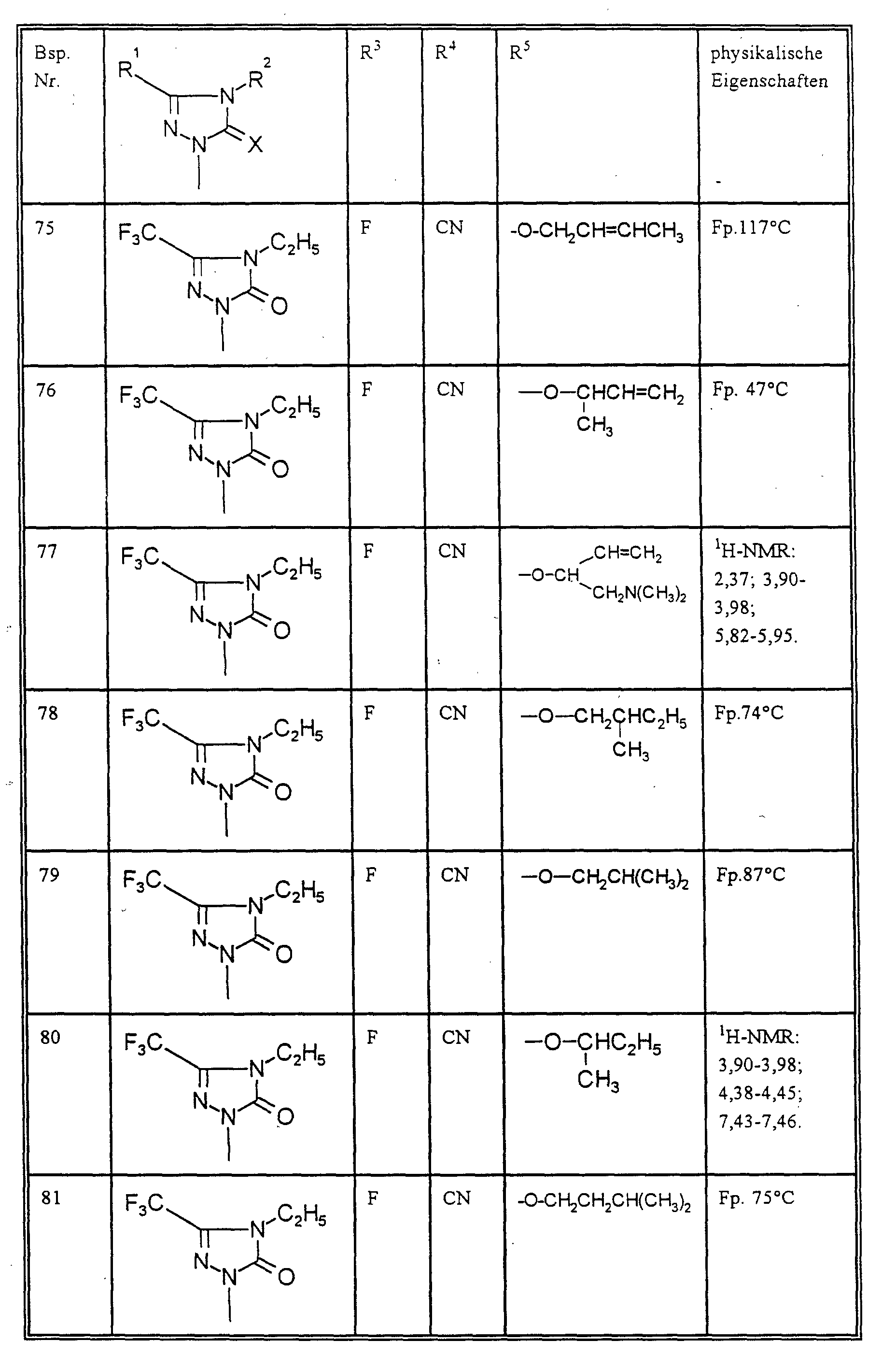

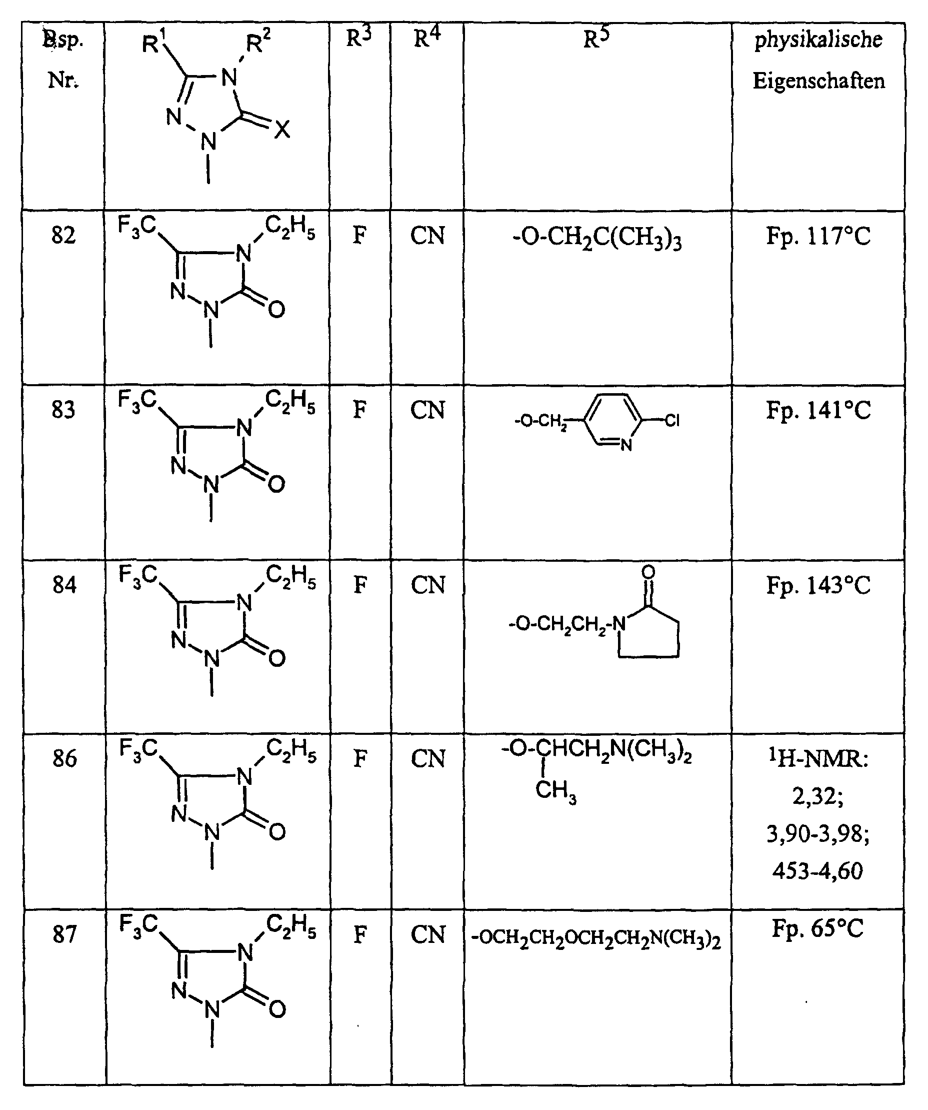

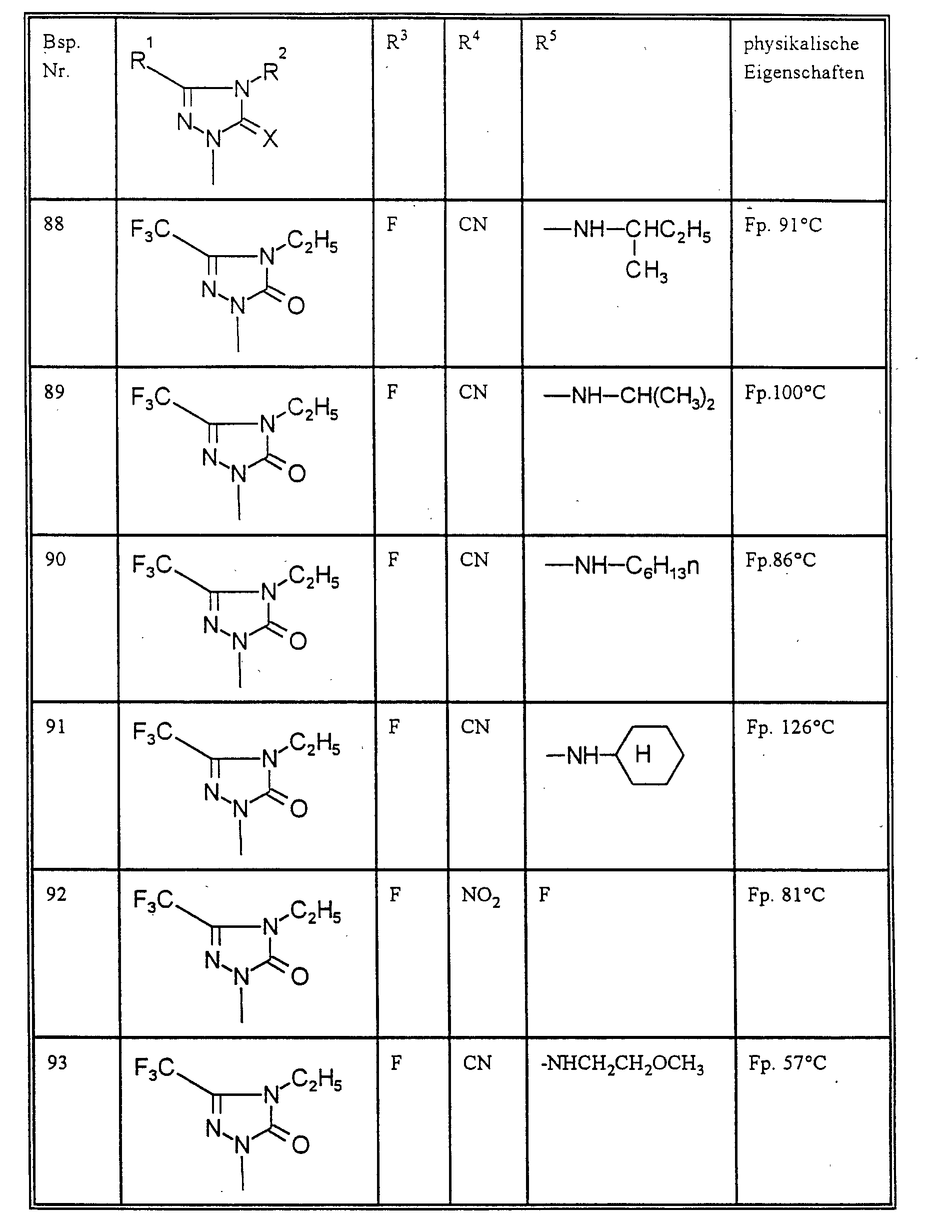

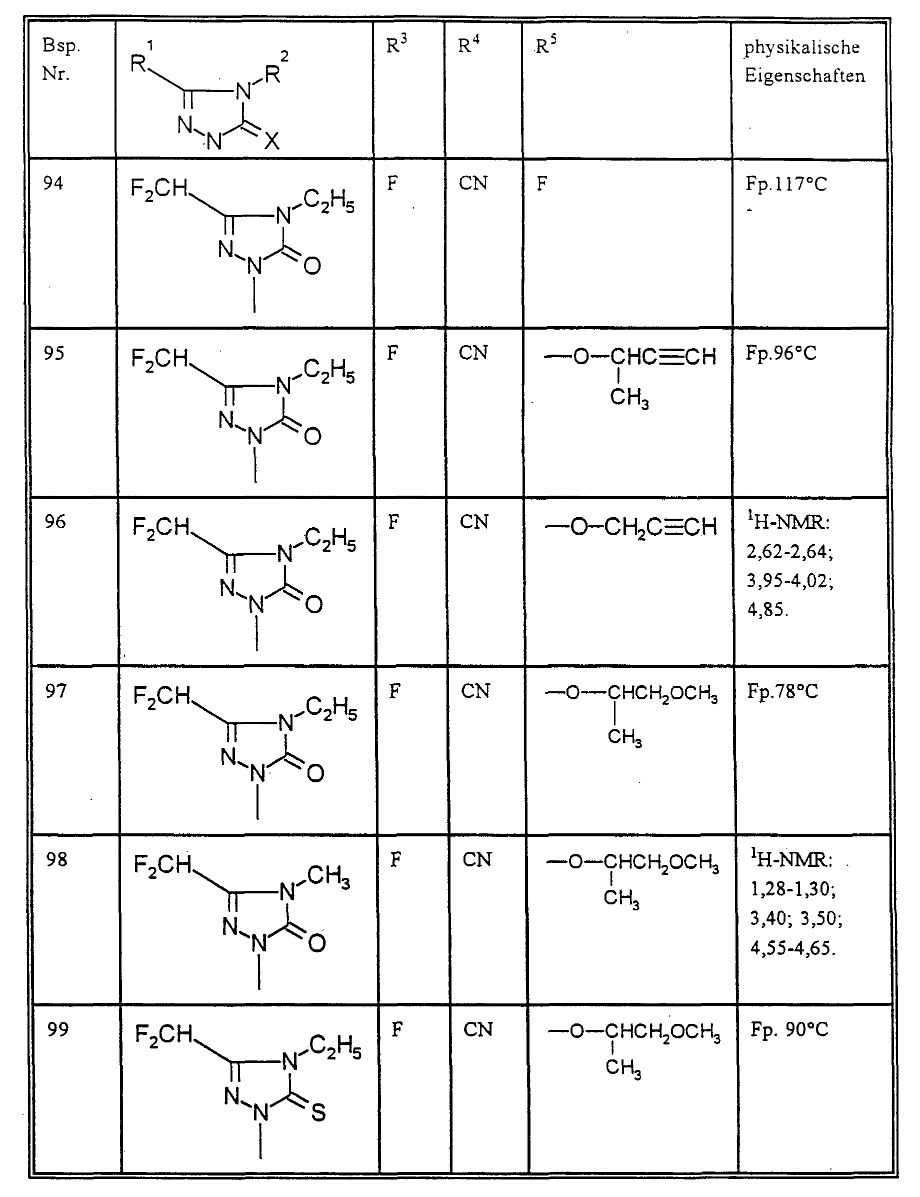

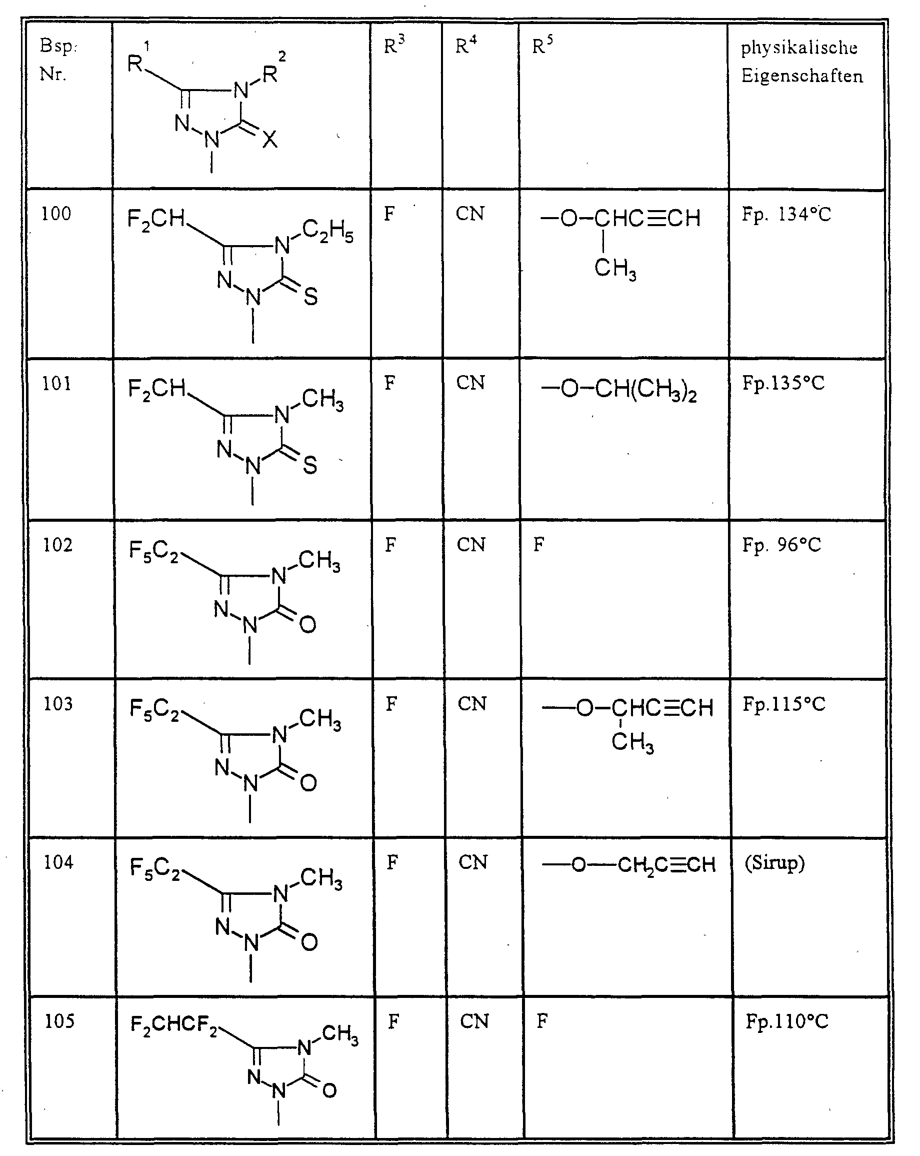

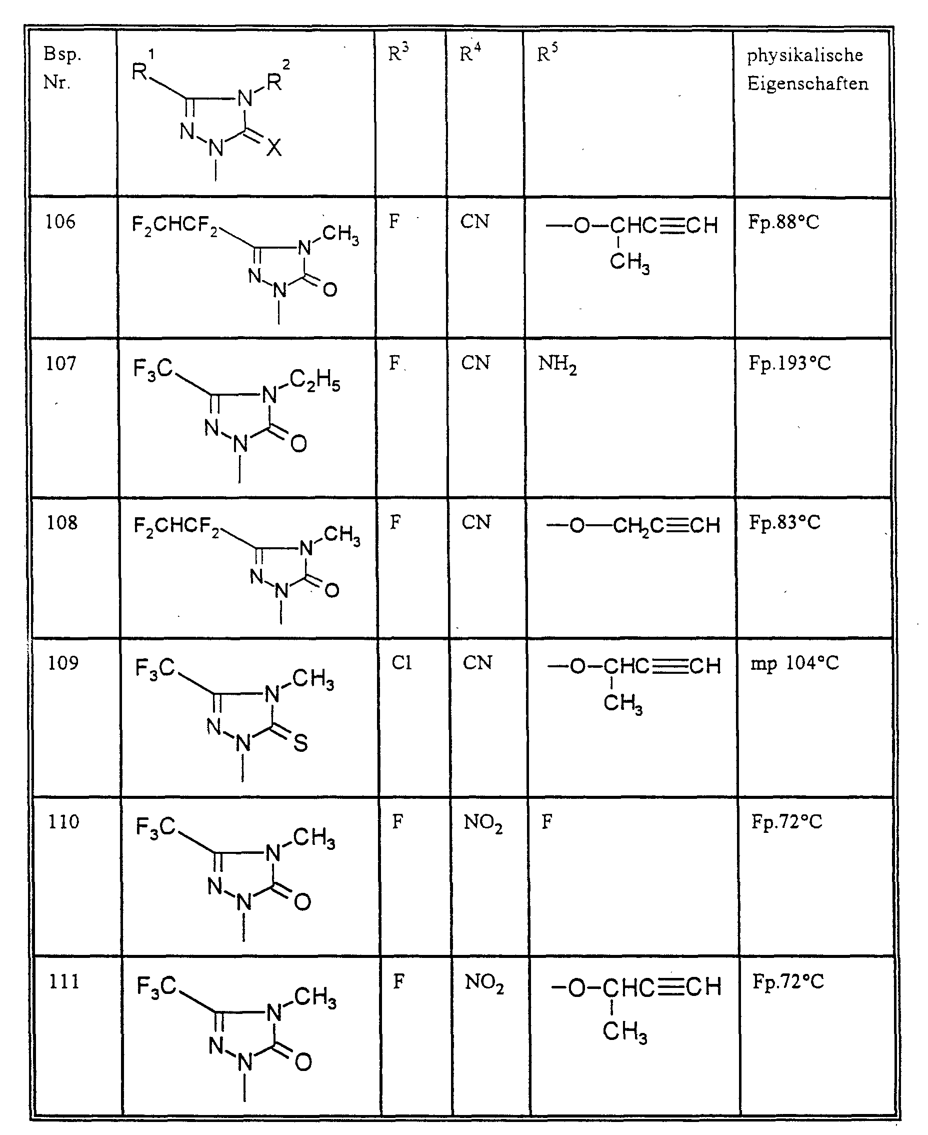

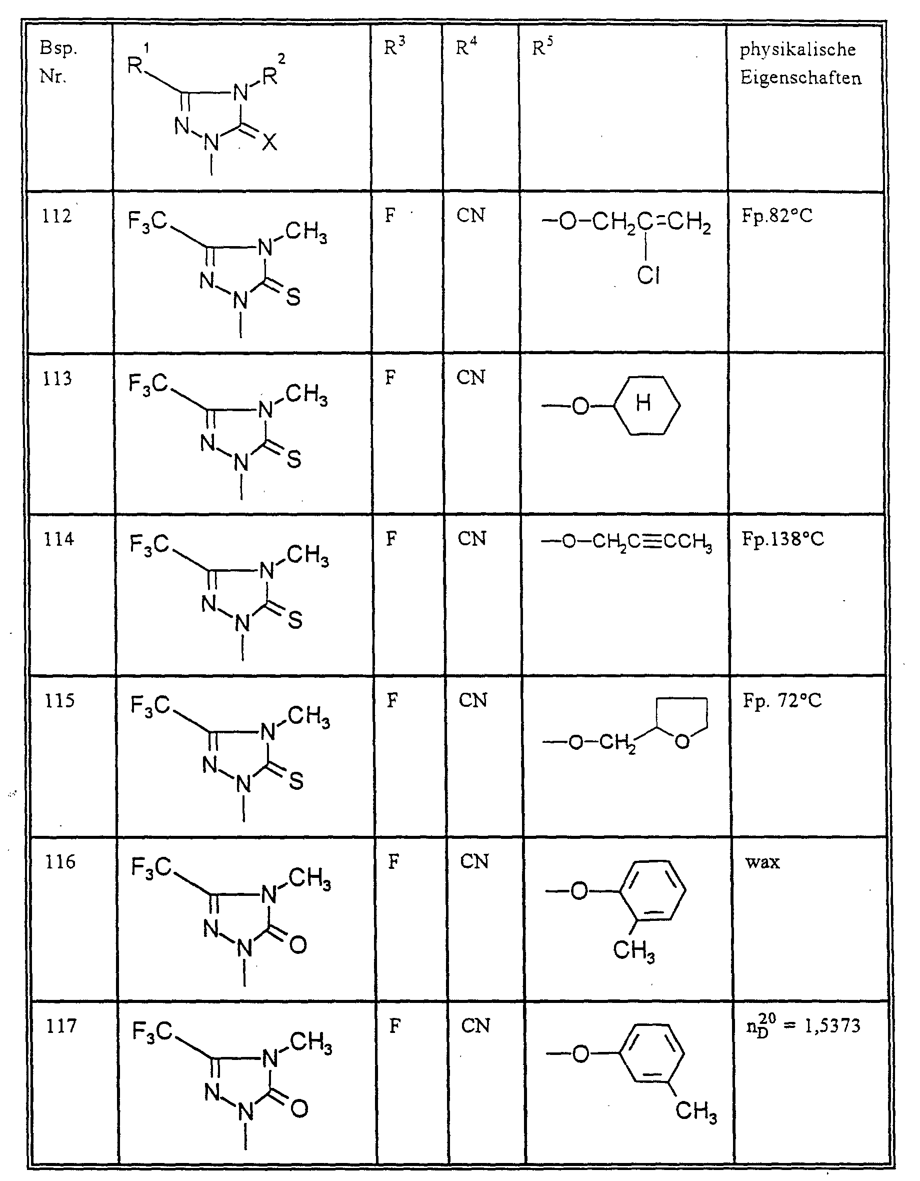

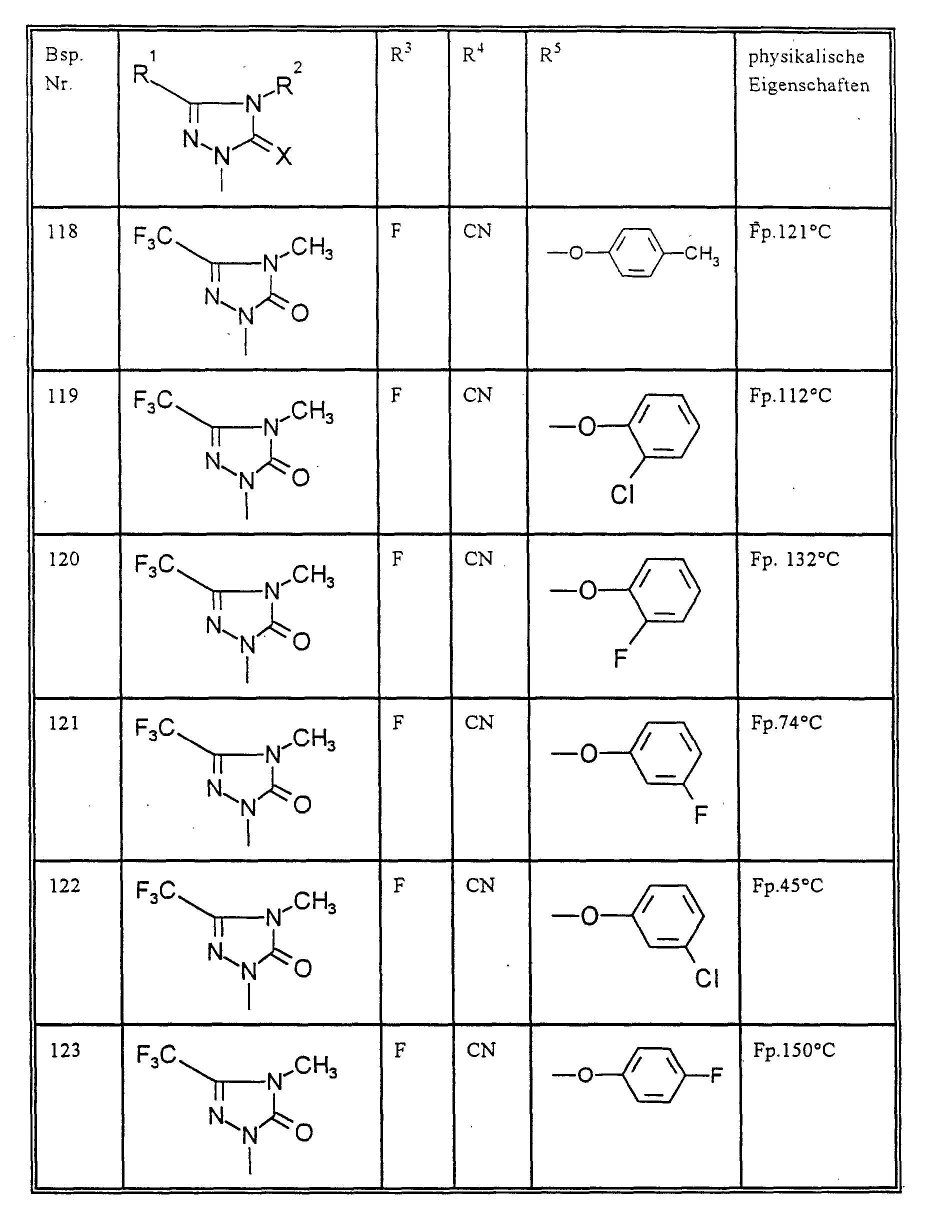

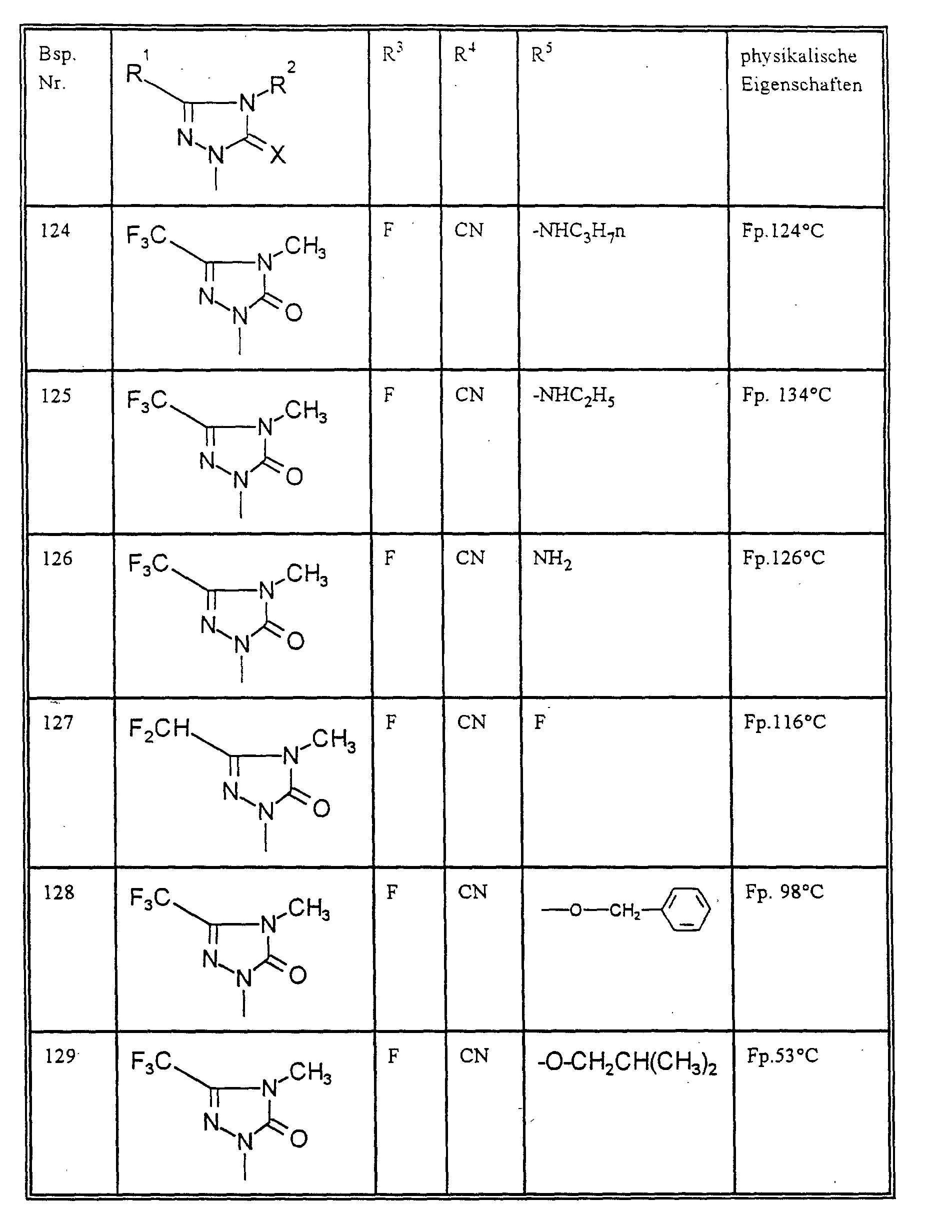

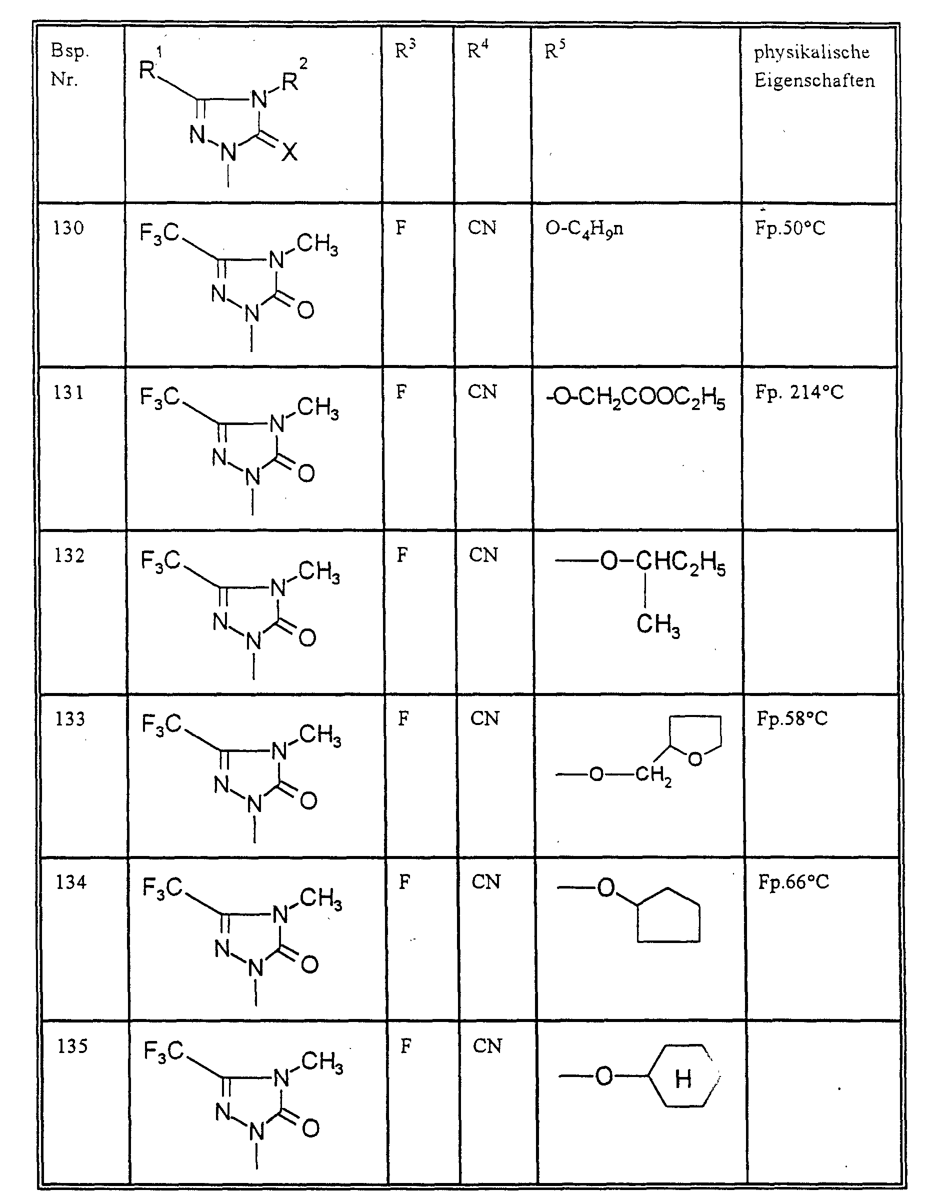

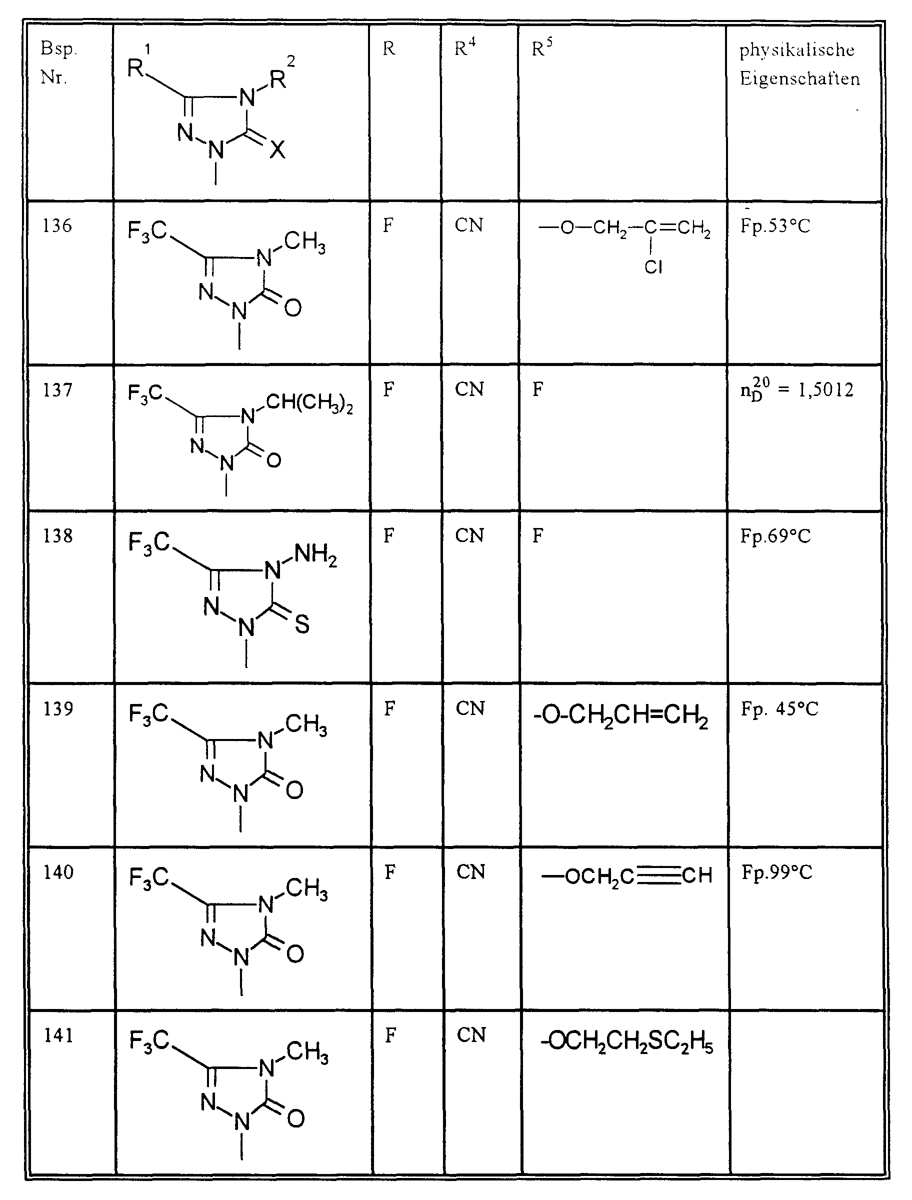

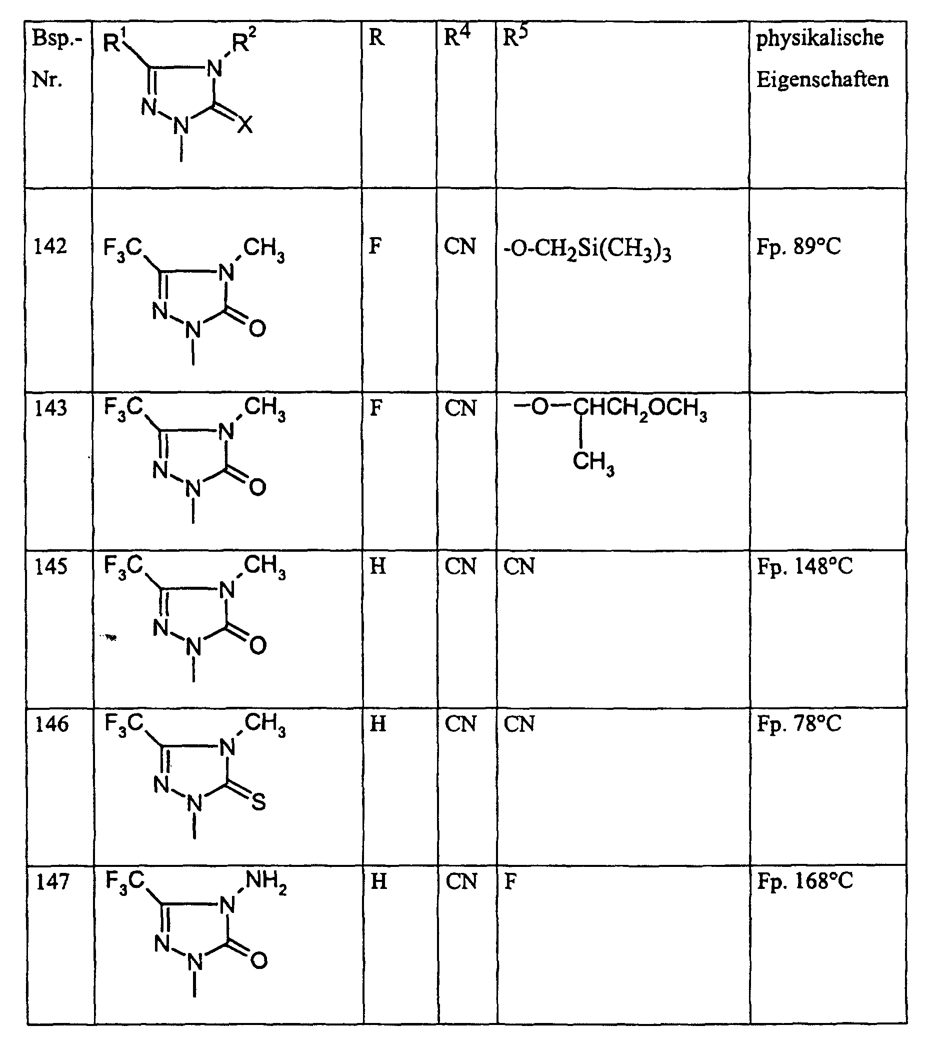

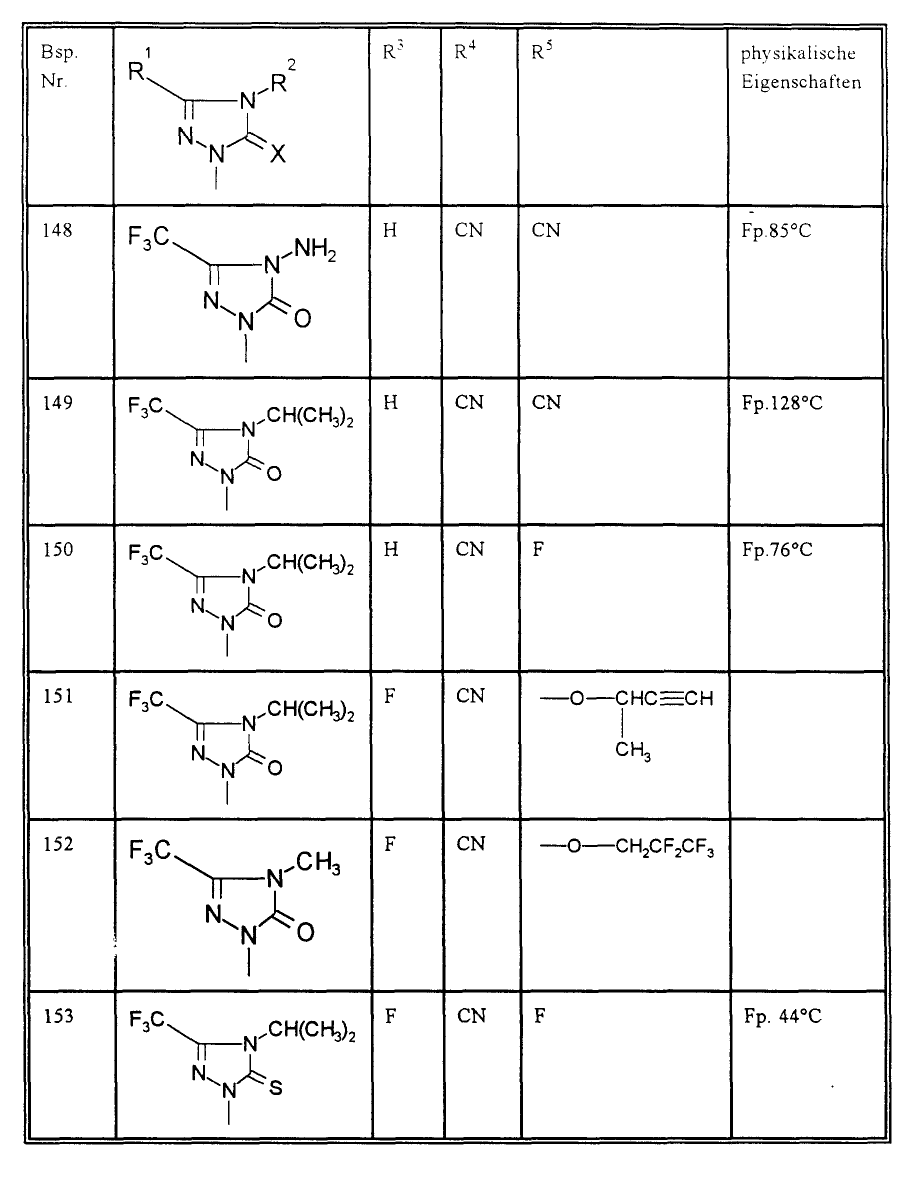

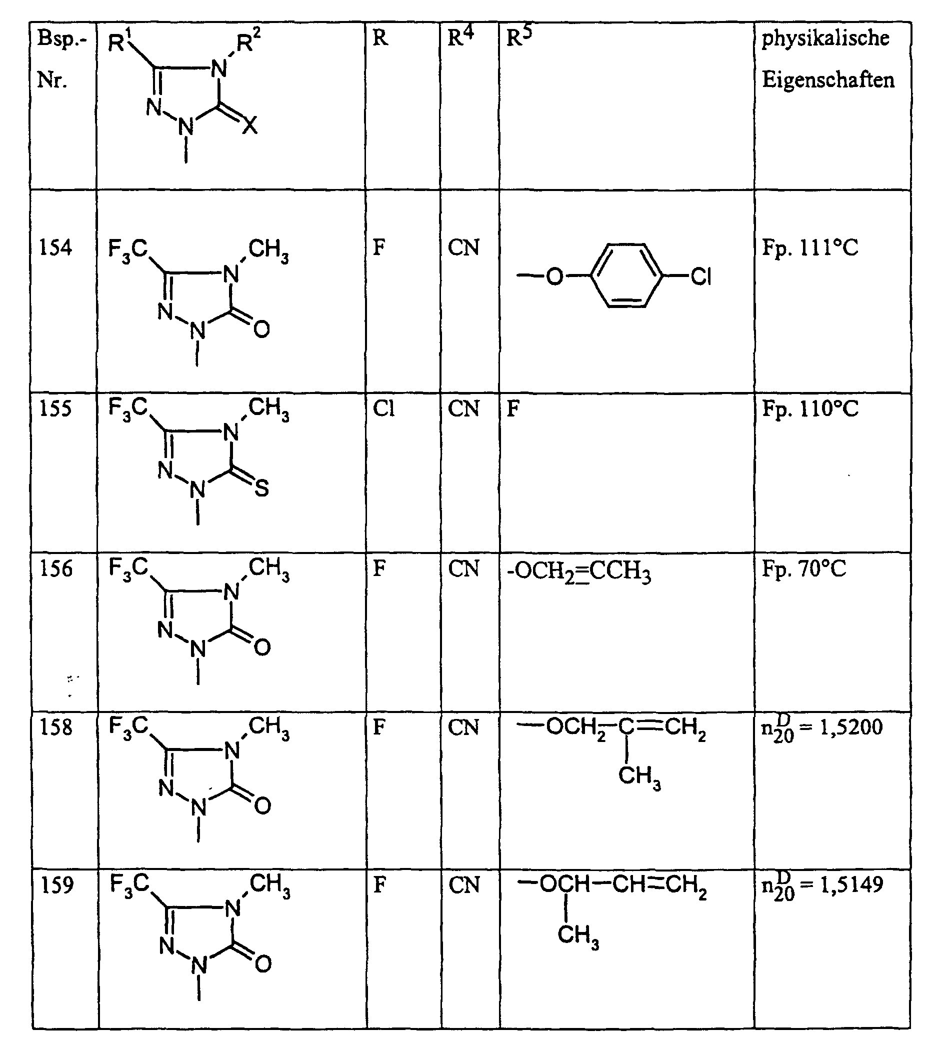

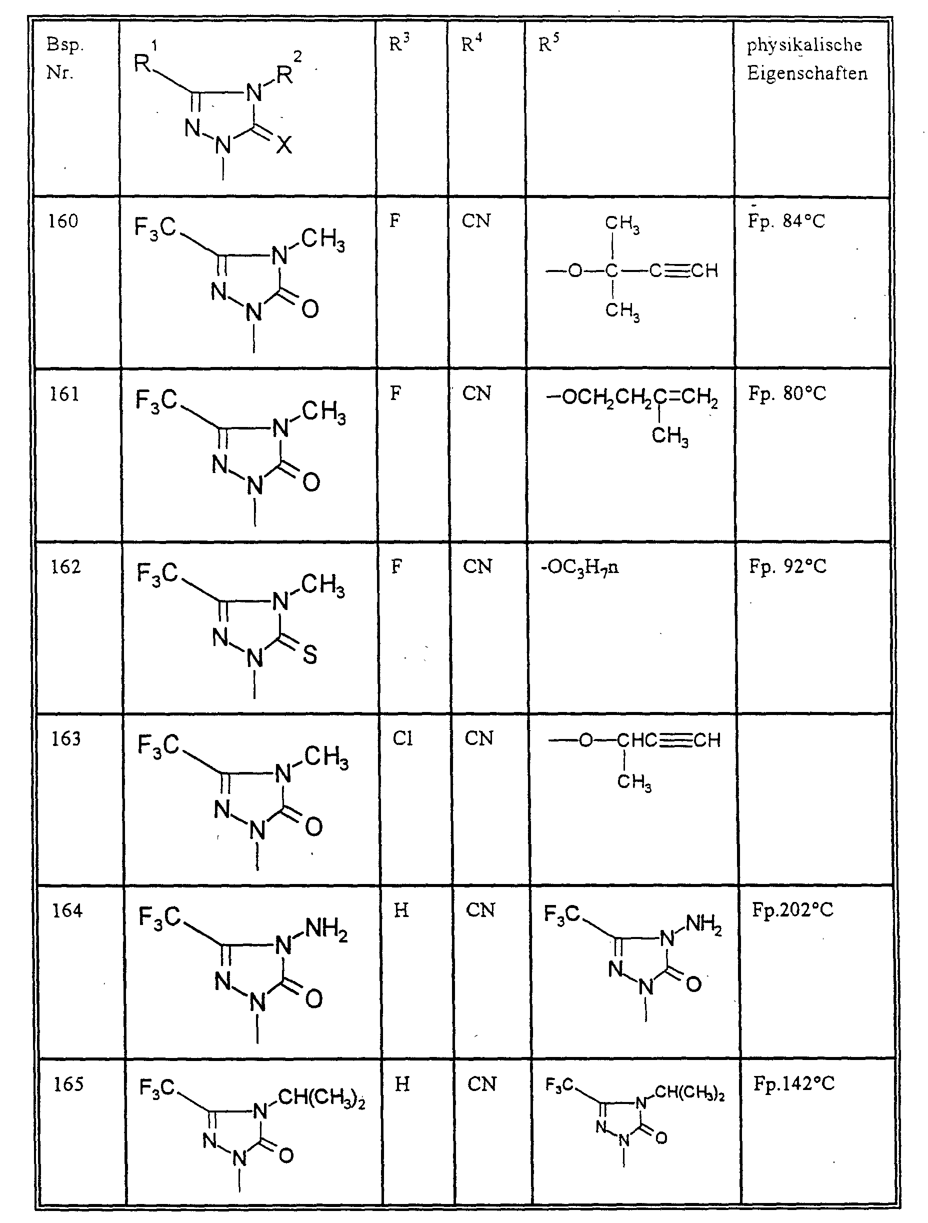

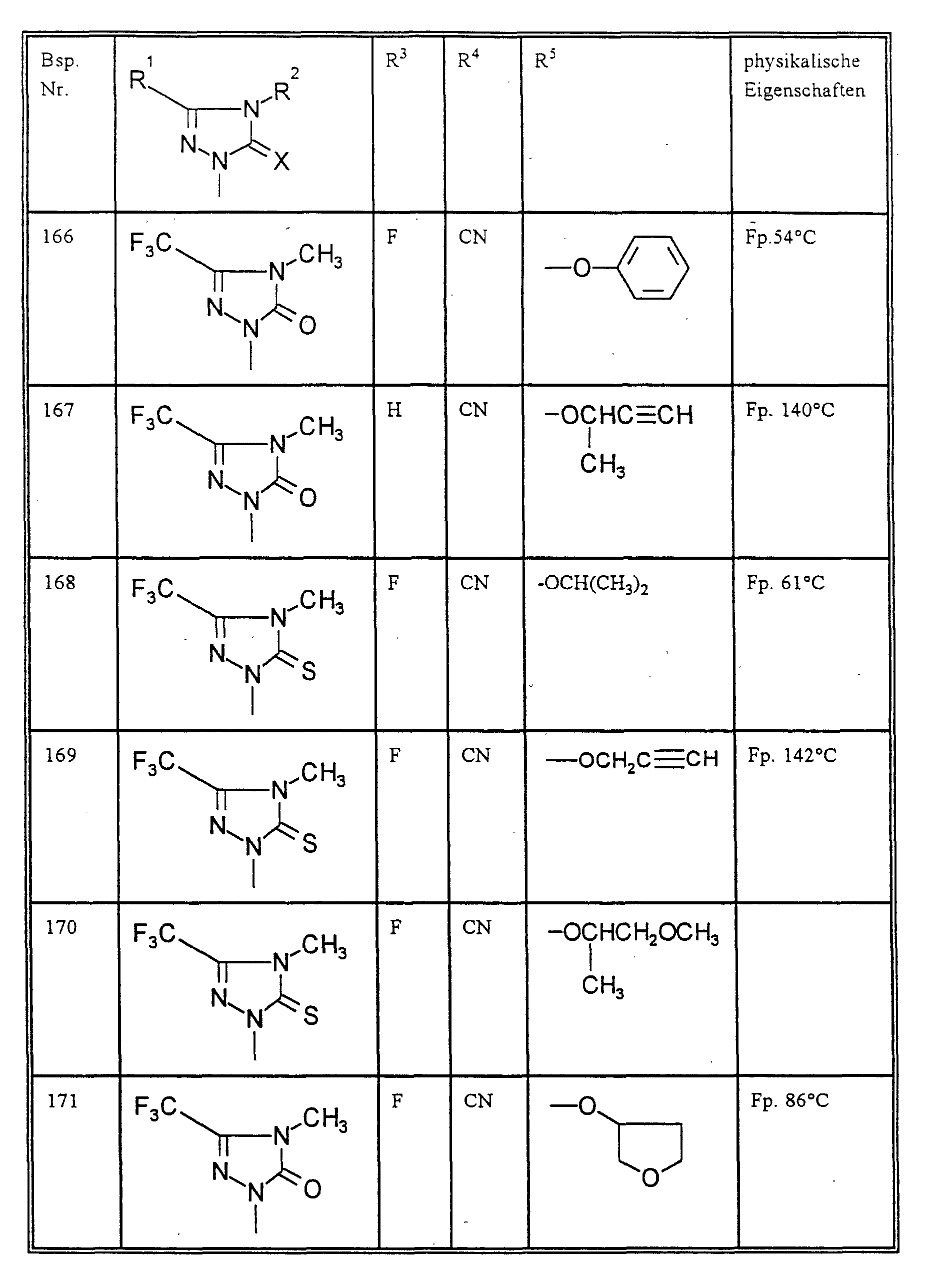

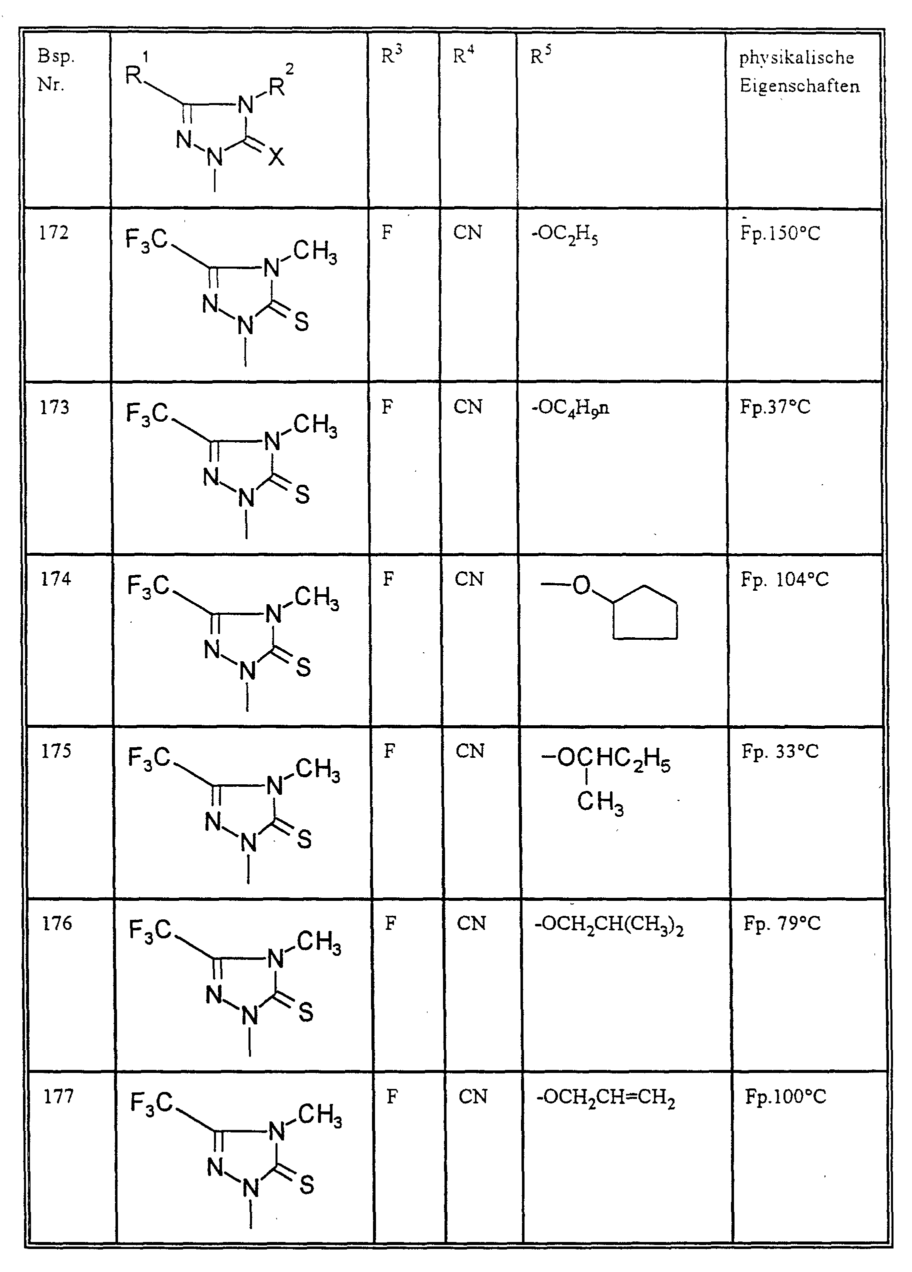

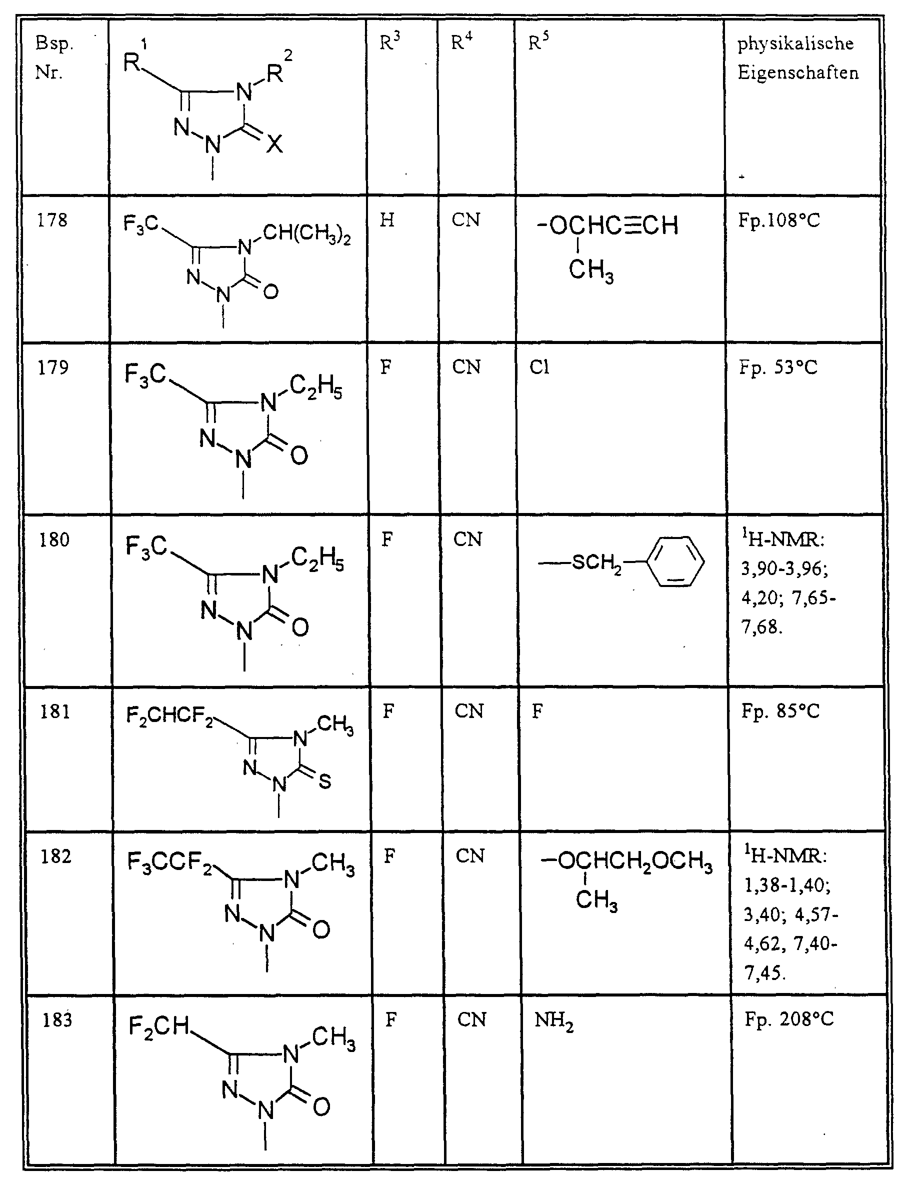

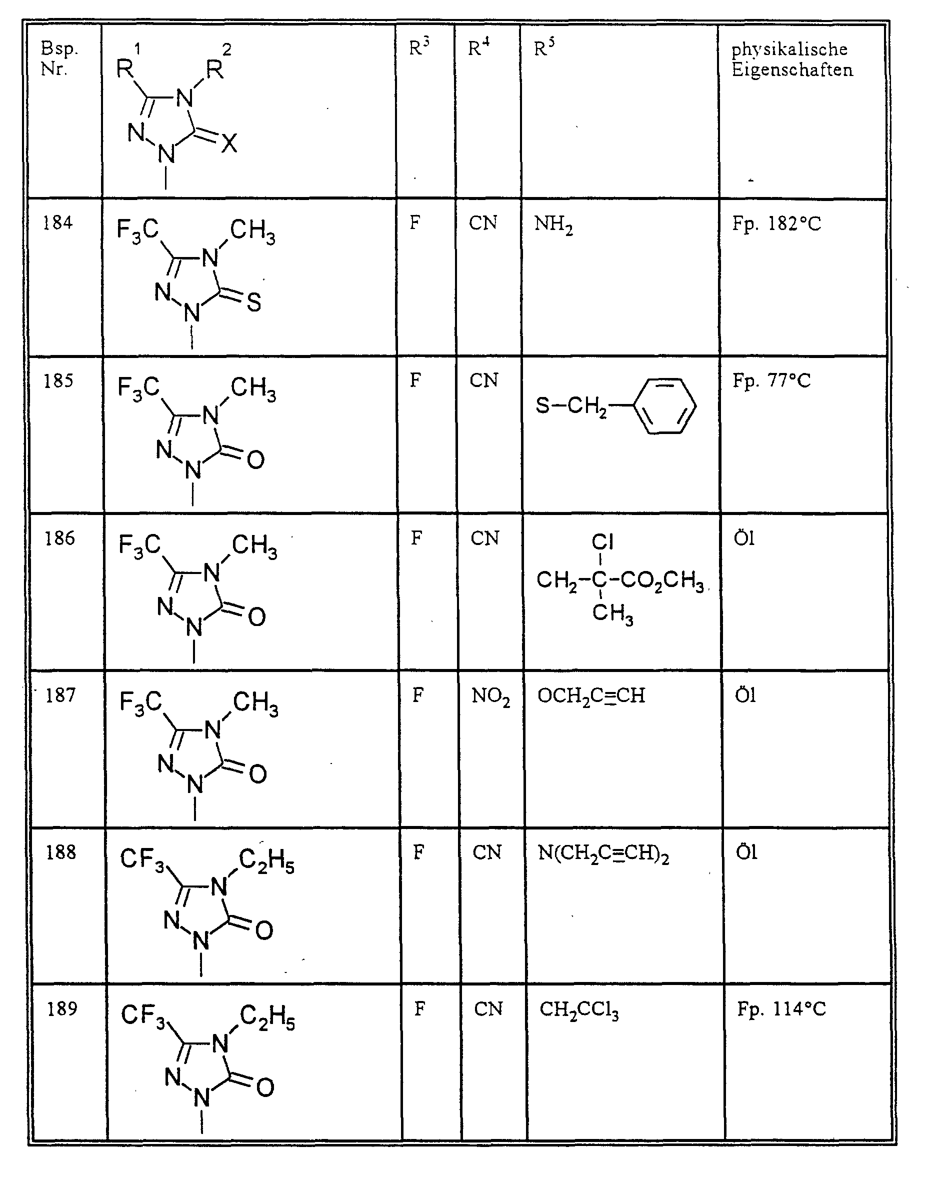

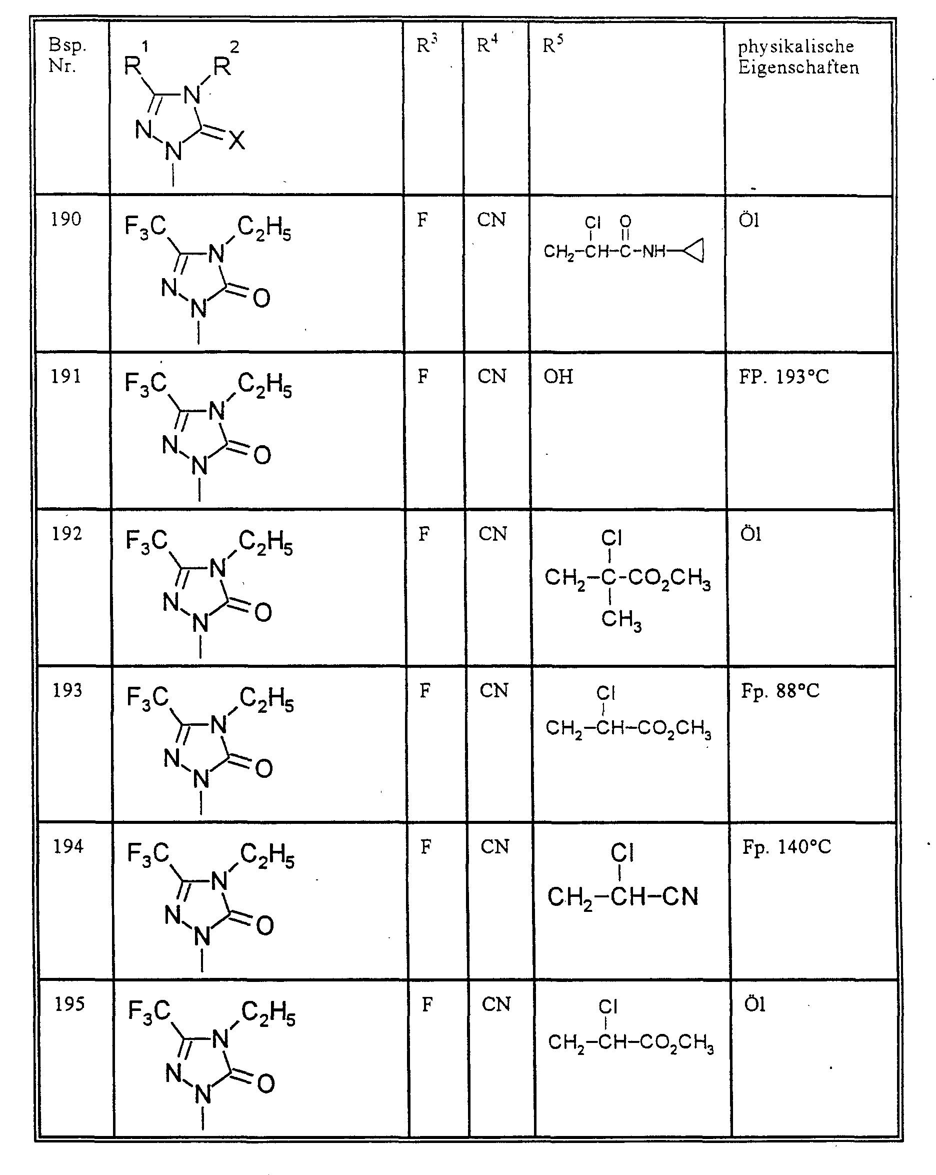

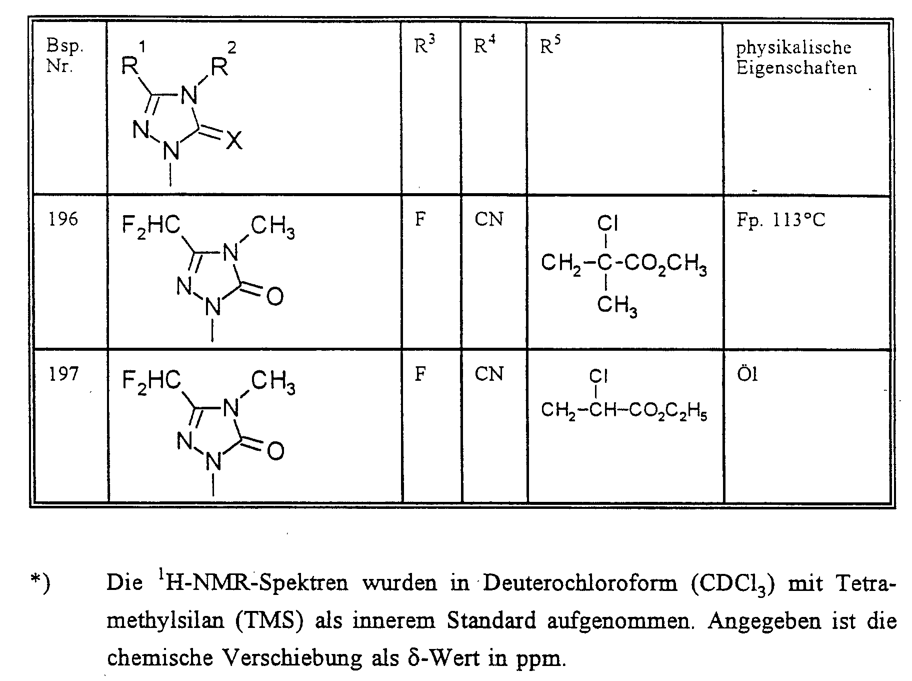

### Anwendungsbeispiele:

In den folgenden Anwendungsbeispielen wurden die nachstehend aufgeführten Verbindungen als Vergleichssubstanzen eingesetzt: 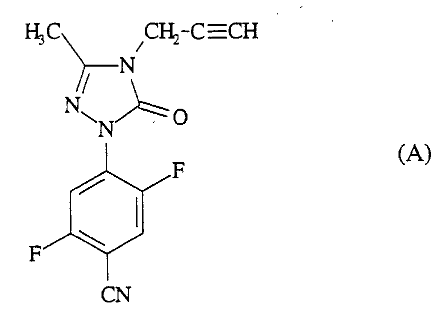
3-Methyl-4-propargyl-1 -(2,5-difluor-4-cyano-phenyl)-1,2,4-triazolin-5-on 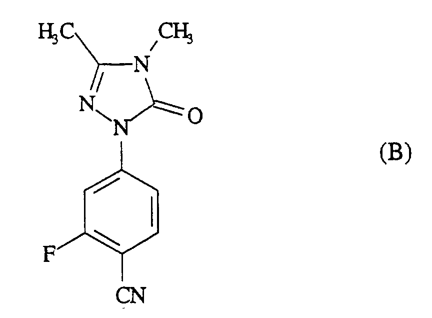
3,4-Dimethyl-1-(3-fluor-4-cyano-phenyl)-1,2,4-triazolin-5-on
   (beide bekannt aus DE 38 39 480)

### Beispiel A:

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Während das Vergleichsbeispiel (A) bei einer Aufwandmenge von 250 g/ha keinerlei herbizide Wirkung gegen Unkräuter wie Setaria, Amaranthus, Chenopodium, Galinsoga, Matricaria, Solanum und Viola aufweist, zeigen in diesem Test z.B. die Verbindungen gemäß den Herstellungsbeispielen 7, 9, 17 und 29 Wirkungen zwischen 40 und 100 % und die Verbindungen gemäß den Herstellungsbeispielen 10, 11, 12, 15 und 19 Wirkungen zwischen 95 und 100 %.

### Beispiel B:

### Tetranychus Test (OP-resistent)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Abtötung in Prozent bestimmt Dabei bedeutet 100 %, daß alle Spinnmilben abgetötet wurden; 0 % bedeutet, daß keine Spinnmilben abgetötet wurde.

Bei diesem Test zeigt z.B. die Verbindung 13 der Herstellungsbeispiele eine deutlich überlegene akarizide Wirksamkeit gegenüber dem aus dem Stand der Technik bekannten Beispiel (B).

### Beispiel C:

### Phaedon-Test

| | |
|---|---|
| Lösungsmittel | 31 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Kohlblätter (Brassica oleracea) werden mit der Wirkstoffzubereitung der gewünschten Konzentration behandelt. Ein behandeltes Blatt wird in eine Plastikdose gelegt und mit Larven (L2) des Meerrettichkäfers (Phaedon cochleariae) besetzt. Nach 3 Tagen wird jeweils ein unbehandeltes Blatt für die Nachfütterung verwendet.

Nach der gewünschten Zeit wird die Abtötung in Prozent bestimmt. Dabei bedeutet 100 %, daß alle Tiere abgetötet wurden; 0 % bedeutet, daß keine Tiere abgetötet wurden.

Bei diesem Test zeigen z.B. die Verbindungen (20) und (62) der Herstellungsbeispiele überlegene Wirksamkeit gegenüber dem aus dem Stand der Technik.

### Beispiel D:

### Myzus-Test

| | |
|---|---|
| Lösungsmittel | 31 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Keimlinge der Dicken Bohne (Vicia faba), welche mit der Grünen Pfirsichblattlaus (Myzus persicae) befallen sind, werden in die Wirkstoffzubereitung der gewünschten Konzentration getaucht und in eine Plastikdose gelegt

Nach der gewünschten Zeit wird die Abtötung in Prozent bestimmt Dabei bedeutet 100 %, daß alle Tiere abgetötet wurden; 0 % bedeutet, daß keine Tiere abgetötet wurden.

Bei diesem Test zeigen z.B. die Verbindungen (62) und (57) der Herstellungsbeispiele überlegene Wirksamkeit gegenüber dem aus dem Stand der Technik.

## Patentansprüche

1. Substituierte Triazolinone der allgemeinen Formel (I), 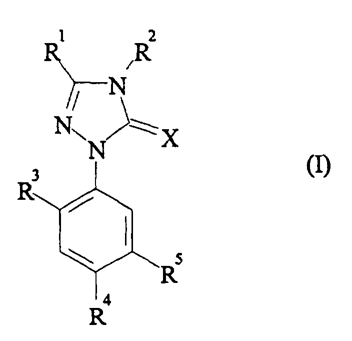 in welcher
R¹ für geradkettiges oder verzweigtes Halogenalkyl mit 1 bis 6 Kohlenstoffatomen und 1 bis 13 gleichen oder verschiedenen Halogenatomeninsbesondere Fluor, Chlor, Brom oder Iod - steht,
R² für Amino, für geradkettiges oder verzweigtes Alkyl mit 1 bis 8 Kohlenstoffatomen, für geradkettiges oder verzweigtes Alkenyl mit 2 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom oder Iod -, für geradkettiges oder verzweigtes Alkoxyalkyl mit jeweils 1 bis 4 Kohlenstoffatomen in den einzelnen Alkylteilen, für geradkettiges oder verzweigtes Alkylidenimino mit 1 bis 8 Kohlenstoffatomen oder für jeweils gegebenenfalls im Cycloalkylteil einfach oder mehrfach, gleich oder verschieden durch Halogen - insbesondere Fluor, Chlor, Brom und/oder Iod - substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im geradkettigen oder verzweigten Alkylteil steht,
R³ für Wasserstoff, Fluor, Chlor, Brom oder Iod steht,
R⁴ für Cyano oder Nitro steht,
R⁵ für Nitro, Cyano, Fluor, Chlor, Brom, Iod, R⁶, -O-R⁶, -S-R⁶, -NR⁶R⁷ oder für einen Rest der Formel 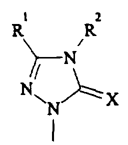 steht und
X für Sauerstoff oder Schwefel steht, wobei
R⁶ und R⁷ unabhängig voneinander jeweils für Wasserstoff oder für gegebenenfalls einfach oder mehrfach, gleich oder verschieden substituiertes, geradkettiges oder verzweigtes Alkyl mit 1 bis 8 Kohlenstoffatomen stehen, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, Alkoxycarbonylalkyl, N-Alkylaminocarbonyl, Cycloalkylaminocarbonyl, N,N-Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefelsteht;
R⁶ und R⁷ außerdem für jeweils gegebenenfalls einfach oder mehrfach, gleich oder verschieden durch Halogen - insbesondere Fluor, Chlor, Brom und/oder Iod -substituiertes Alkenyl oder Alkinyl mit jeweils 2 bis 8 Kohlenstoffatomen stehen;
R⁶ und R⁷ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen oder für C₃-C₇-Cycloalkyl-C₁-C₃-alkyl stehen oder
R⁶ und R⁷ für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, wobei als Arylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

2. Substituierte Triazolinone gemäß Anspruch 1, **dadurch gekennzeichnet, daß**
R¹ für geradkettiges oder verzweigtes Halogenalkyl mit 1 bis 4 Kohlenstoffatomen und 1 bis 9 gleichen oder verschiedenen Halogenatomeninsbesondere Fluor, Chlor oder Brom - steht,
R² für Amino, für geradkettiges oder verzweigtes Alkyl mit 1 bis 6 Kohlenstoffatomen, für geradkettiges oder verzweigtes Alkenyl mit 2 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom -, für geradkettiges oder verzweigtes Alkoxyalkyl mit jeweils 1 bis 3 Kohlenstoffatomen in den einzelnen Alkylteilen, für geradkettiges oder verzweigtes Alkylidenimino mit 1 bis 6 Kohlenstoffatomen oder für jeweils gegebenenfalls im Cycloalkylteil einfach bis vierfach, gleich oder verschieden durch Halogen - insbesondere Fluor, Chlor und/ oder Brom - substituiertes Cycloalkyl oder Cycloalkylalkyl mit jeweils 3 bis 7 Kohlenstoffatomen im Cycloalkylteil und gegebenenfalls 1 bis 3 Kohlenstoffatomen im geradkettigen oder verzweigten Alkylteil steht,
R³ für Wasserstoff, Fluor, Chlor oder Brom steht,
R⁴ für Cyano oder Nitro steht,
R⁵ für Nitro, Cyano, Fluor, Chlor, Brom, R⁶, -O-R⁶, -S-R⁶, -NR⁶R⁷, oder für einen Rest der Formel 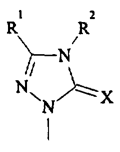 steht und
X für Sauerstoff oder Schwefel steht, wobei
R⁶ und R⁷ unabhängig voneinander jeweils für Wasserstoff oder für gegebenenfalls einfach substituiertes geradkettiges oder verzweigtes Alkyl mit 1 bis 6 Kohlenstoffatomen stehen, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, Alkoxycarbonylalkyl, N-Alkylaminocarbonyl, N,N-Dialkylaminocarbonyl, Trialkylsilyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
R⁶ und R⁷ außerdem für geradkettiges oder verzweigtes und gegebenenfalls neben Halogen zusätzlich substituiertes Halogenalkyl mit 1 bis 4 Kohlenstoffatomen und 1 bis 9 gleichen oder verschiedenen Halogenatomeninsbesondere Fluor, Chlor oder Brom - stehen, wobei als Substituenten C₁₋₂-Alkoxycarbonyl, C₁₋₆-Cycloalkylaminocarbonyl oder Cyano infrage kommen;
R⁶ und R⁷ außerdem für jeweils gegebenenfalls einfach bis dreifach, gleich oder verschieden durch Halogen - insbesondere Fluor, Chlor oder Bromsubstituiertes Alkenyl oder Alkinyl mit jeweils 2 bis 6 Kohlenstoffatomen stehen;
R⁶ und R⁷ außerdem für gegebenenfalls einfach bis vierfach, gleich oder verschieden durch Halogen - insbesondere Fluor, Chlor oder Bromund/oder geradkettiges oder verzweigtes Alkyl mit 1 bis 3 Kohlenstoffatomen substituiertes Cycloalkyl mit 3 bis 6 Kohlenstoffatomen oder für C₃-C₆-Cycloalkyl-C₁-C₂-alkyl stehen oder
für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil stehen, wobei als Phenylsubstituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl.

3. Verfahren zur Herstellung neuer substituierter Triazolinone der allgemeinen Formel (I) 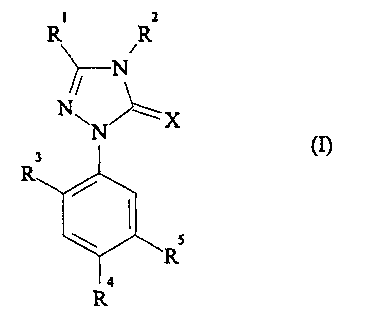 in welcher
R¹, R², R³, R⁴, R⁵ und X die in Anspruch 1 genannten Bedeutungen haben,
**dadurch gekennzeichnet, daß** man
a) 1H-Triazolinone der Formel (II), 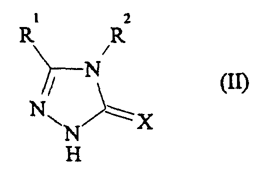 in welcher
R¹, R² und X die in Anspruch 1 angegebenen Bedeutungen haben,
mit Halogenbenzol-Derivaten der Formel (III), 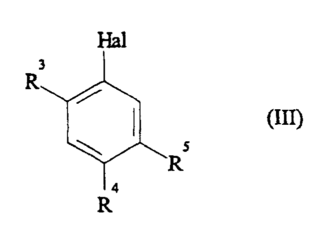 in welcher
R³, R⁴ und R⁵ die in Anspruch 1 angegebenen Bedeutungen haben und
Hal für Halogen steht,
gegebenenfalls in Gegenwart eines Verdünnungsmittels und gegebebenfalls in Gegenwart eines Reaktionshilfsmittels umsetzt,
oder daß man
b) substituierte Triazolinone der Formel (Ia), 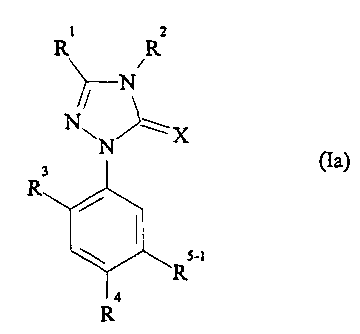 in welcher
R¹, R², R³, R⁴ und X die in Anspruch 1 angegebenen Bedeutungen haben und
R⁵⁻¹ für Halogen steht,
mit Nukleophilen der Formel (IV),
R⁶⁻¹-Z-H (IV)
in welcher
Z für Sauerstoff oder Schwefel steht und
R⁶⁻¹ für jeweils geradkettiges oder verzweigtes, gegebenenfalls substituiertes Alkyl, Alkenyl, Alkinyl, Cycloalkyl oder Aryl steht und außerdem für den Fall, daß Z für Sauerstoff steht, auch für Heterocyclyl steht,
gegebenenfalls in Gegenwart eines Verdünnungsmittels und gegebenenfalls in Gegenwart eines Reaktionshilfsmittels umsetzt oder daß man
c) substituierte Triazolinone der Formel (Ib), 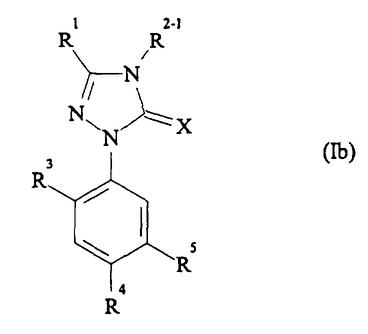 in welcher
R¹, R³, R⁴, R⁵ und X die in Anspruch 1 angegebenen Bedeutungen haben und
R²⁻¹ für Amino steht,
mit Natriumnitrit in Gegenwart einer Säure und gegebenenfalls in Gegenwart eines Verdünnungsmittels umsetzt oder daß man
d) substituierte Triazolinone der Formel (Ic), 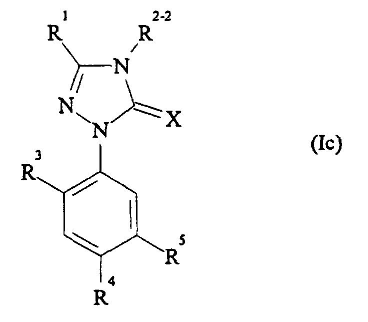 in welcher
R¹, R³, R⁴, R⁵ und X die in Anspruch 1 angegebenen Bedeutungen haben und
R²⁻² : für Wasserstoff steht,
mit Alkylierungsmitteln der Formel (V),
R²⁻³-E (V)
in welcher
R²⁻³ für Alkyl, Alkenyl, Alkinyl, Halogenalkyl, Halogenalkenyl, Halogenalkinyl, Alkoxyalkyl oder für gegebenenfalls substituiertes Cycloalkyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt.

4. Verfahren zur Bekämpfung von unerwünschten Pflanzen, **dadurch gekennzeichnet, daß** man substituierte Triazolinone gemäß Anspruch 1 oder 2 auf Pflanzen und/oder ihren Lebensraum einwirken läßt.

5. Verwendung von substituierten Triazolinonen gemäß Anspruch 1 oder 2 zur Bekämpfung von unerwünschten Pflanzen.

6. Verfahren zu Herstellung von herbiziden und akariziden Mitteln, **dadurch gekennzeichnet, daß** man substituierte Triazolinone gemäß Anspruch 1 oder 2 mit Streckmitteln und/oder oberflächenaktiven Mitteln vermischt.

7. Verfahren zur Bekämpfung von Arachniden, **dadurch gekennzeichnet, daß** man substituierte Triazolinone gemäß Anspruch 1 oder 2 auf Arachnide und/oder ihren Lebensraum einwirken läßt.

8. Verwendung von substituierten Triazolinonen gemäß Anspruch 1 oder 2 zur Bekämpfung von Arachniden.

9. Substituierte Triazolinone gemäß Anspruch I**dadurch gekennzeichnet, daß**
- R¹, R², R³, R⁴ und X die in Anspruch 1 angegebenen Bedeutungen haben und
R⁵ für Fluor, Chlor, Brom oder Iod steht.

10. Substituierte Triazolinone gemäß Anspruch 1, **dadurch gekennzeichnet, daß**
R¹,R³,R⁴R⁵ und X die in Anspruch 1 angegebenen Bedeutungen haben und
R² für Amino steht.

11. Substituierte Triazolinone der Formel (Ic) 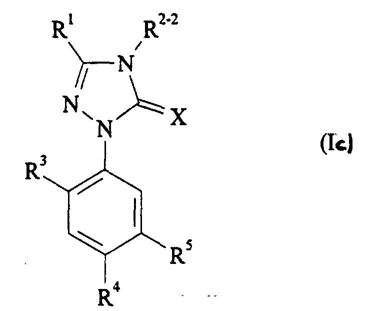 **dadurch gekennzeichnet, daß**
R¹, R³, R⁴, R⁵ und X die in Anspruch 1 genannten Bedeutungen haben und
R²⁻² für Wasserstoff steht.

## Claims

1. Substituted triazolinones of the general formula (I) 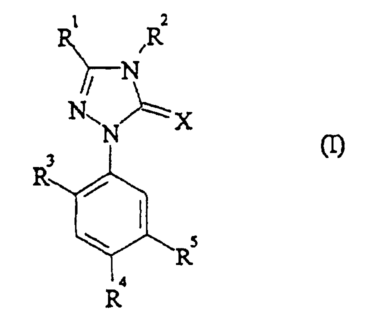 in which
R¹ represents straight-chain or branched halogenoalkyl having 1 to 6 carbon atoms and 1 to 13 identical or different halogen atoms, in particular fluorine, chlorine, bromine or iodine,
R² represents amino, straight-chain or branched alkyl having 1 to 8 carbon atoms, straight-chain or branched alkenyl having 2 to 6 carbon atoms, straight-chain or branched halogenoalkyl having 1 to 6 carbon atoms and 1 to 13 identical or different halogen atoms, in particular fluorine, chlorine, bromine or iodine, straight-chain or branched alkoxyalkyl having 1 to 4 carbon atoms in each of the individual alkyl moieties, straight-chain or branched alkylideneimino having 1 to 8 carbon atoms, or cycloalkyl or cycloalkylalkyl, each of which has 3 to 8 carbon atoms in the cycloalkyl moiety and, if appropriate, 1 to 4 carbon atoms in the straight-chain or branched alkyl moiety, and each of which is optionally monosubstituted or polysubstituted in the cycloalkyl moiety by identical or different halogen substituents, in particular fluorine, chlorine, bromine and/or iodine,
R³ represents hydrogen, fluorine, chlorine, bromine or iodine,
R⁴ represents cyano or nitro,
R⁵ represents nitro, cyano, fluorine, chlorine, bromine, iodine or a radical of the formula 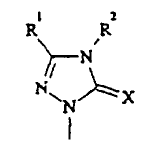 and
X represents oxygen or sulphur, where
R⁶ and R⁷ independently of one another in each case represent hydrogen or straight-chain or branched alkyl which has 1 to 8 carbon atoms and which is optionally monosubstituted or polysubstituted by identical or different substituents, suitable substituents being:
halogen, in particular fluorine, chlorine, bromine and/or iodine, cyano, carboxyl, carbamoyl, in each case straight-chain or branched alkoxy, alkoxyalkoxy, alkylthio, alkylsulphinyl, alkylsulphonyl, alkoxycarbonyl, alkoxycarbonylalkyl, N-alkylaminocarbonyl, cycloalkylaminocarbonyl, N,N-dialkylaminocarbonyl, trialkylsilyl or alkylsulphonylaminocarbonyl, each of which has 1 to 8 carbon atoms in the individual alkyl moieties, or heterocyclyl, the heterocyclyl being represented by a five- to seven-membered, optionally benzo-fused, saturated or unsaturated heterocycle having 1 to 3 identical or different hetero atoms, in particular nitrogen, oxygen and/ or sulphur;
R⁶ and R⁷ furthermore represent alkenyl or alkinyl, each of which has 2 to 8 carbon atoms and each of which is optionally monosubstituted or polysubstituted by identical or different halogen substituents, in particular fluorine, chlorine, bromine and/or iodine;
R⁶ and R⁷ furthermore represent cycloalkyl which has 3 to 7 carbon atoms and which is optionally monosubstituted or polysubstituted by identical or different halogen substituents, in particular fluorine, chlorine, bromine and/or iodine, and/or by straight-chain or branched alkyl having 1 to 4 carbon atoms or C₃-C₇-cycloalkyl-C₁-C₃-alkyl, or
R⁶ and R⁷ represent arylalkyl or aryl, each of which has 6 to 10 carbon atoms in the aryl moiety and, if appropriate, 1 to 4 carbon atoms in the straight-chain or branched alkyl moiety, and each of which is optionally monosubstituted or polysubstituted in the aryl moiety by identical or different substituents, suitable aryl substituents in each case being:
halogen, cyano, nitro, in each case straight-chain or branched alkyl, alkoxy, alkylthio, alkylsulphinyl or alkylsulphonyl, each of which has 1 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched alkoxycarbonyl or alkoximinoalkyl, each of which has 1 to 6 carbon atoms in the individual alkyl moieties, and phenyl which is optionally monosubstituted or polysubstituted by identical or different halogen substituents and/or by straight-chain or branched alkyl or alkoxy, each of which has 1 to 6 carbon atoms, and/or by straight-chain or branched halogenoalkyl or halogenoalkoxy, each of which has 1 to 6 carbon atoms and 1 to 13 identical or different halogen atoms.

2. Substituted triazolinones according to Claim 1, **characterised in that**
R¹ represents straight-chain or branched halogenoalkyl having 1 to 4 carbon atoms and 1 to 9 identical or different halogen atoms, in particular fluorine, chlorine or bromine,
R² represents amino, straight-chain or branched alkyl having 1 to 6 carbon atoms, straight-chain or branched alkenyl having 2 to 4 carbon atoms, straight-chain or branched halogenoalkyl having 1 to 4 carbon atoms and 1 to 9 identical or different halogen atoms, in particular fluorine, chlorine or bromine, straight-chain or branched alkoxyalkyl having 1 to 3 carbon atoms in each of the individual alkyl moieties, straight-chain or branched alkylideneimino having 1 to 6 carbon atoms, or cycloalkyl or cycloalkylalkyl, each of which has 3 to 7 carbon atoms in the cycloalkyl moiety and, if appropriate, 1 to 3 carbon atoms in the straight-chain or branched alkyl moiety, and each of which is optionally monosubstituted to tetrasubstituted in the cycloalkyl moiety by identical or different halogen substituents, in particular fluorine, chlorine and/or bromine,
R³ represents hydrogen, fluorine, chlorine or bromine,
R⁴ represents cyano or nitro,
R⁵ represents nitro, cyano, fluorine, chlorine, bromine R⁶, -O-R⁶, -S-R⁶, -NR⁶R⁷ or a radical of the formula 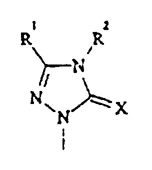 and
X represents oxygen or sulphur, where
R⁶ and R⁷ independently of one another in each case represent hydrogen or straight-chain or branched alkyl which has 1 to 6 carbon atoms and which is optionally monosubstituted, suitable substituents being:
cyano, carboxyl, carbamoyl, in each case straight-chain or branched alkoxy, alkoxyalkoxy, alkylthio, alkylsulphinyl, alkylsulphonyl, alkoxycarbonyl, alkoxycarbonylalkyl, N-alkylaminocarbonyl, N,N-dialkylaminocarbonyl, trialkylsilyl or alkylsulphonylaminocarbonyl, each of which has 1 to 6 carbon atoms in the individual alkyl moieties, or heterocyclyl, the heterocyclyl radical being represented by a five- or six-membered, saturated or unsaturated heterocycle having 1 to 3 identical or different hetero atoms, in particular nitrogen, oxygen and/or sulphur;
R⁶ and R⁷ furthermore represent straight-chain or branched halogenoalkyl having 1 to 4 carbon atoms and 1 to 9 identical or different halogen atoms, in particular fluorine, chlorine or bromine, which may optionally carry other substituents in addition to halogen, suitable substituents being C₁₋₂-alkoxycarbonyl, C₁₋₆-cycloalkylamino or cyano;
R⁶ and R⁷ furthermore represent alkenyl or alkinyl, each of which has 2 to 6 carbon atoms and each of which is optionally monosubstituted to trisubstituted by identical or different halogen substituents, in particular fluorine, chlorine or bromine;
R⁶ and R⁷ furthermore represent cycloalkyl which has 3 to 6 carbon atoms and which is optionally monosubstituted to tetrasubstituted by identical or different halogen substituents, in particular fluorine, chlorine or bromine, and/or by straight-chain or branched alkyl having 1 to 3 carbon atoms or C₃-C₆-cycloalkyl-C₁-C₂-alkyl, or
represent phenylalkyl or phenyl, each of which has 1 to 3 carbon atoms in the straight-chain or branched alkyl moiety and each of which is optionally monosubstituted to trisubstituted in the phenyl moiety by identical or different substituents, suitable phenyl substituents in each case being:
halogen, cyano, nitro, in each case straight-chain or branched alkyl, alkoxy, alkylthio, alkylsulphinyl or alkylsulphonyl, each of which has 1 to 4 carbon atoms, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, and phenyl which is optionally monosubstituted or polysubstituted by identical or different halogen substituents and/or by straight-chain or branched alkyl or alkoxy, each of which has 1 to 4 carbon atoms, and/or by straight-chain or branched halogenoalkyl or halogenoalkoxy, each of which has 1 to 4 carbon atoms and 1 to 9 identical or different halogen atoms.

3. Process for the preparation of new substituted triazolinones of the general formula (I) 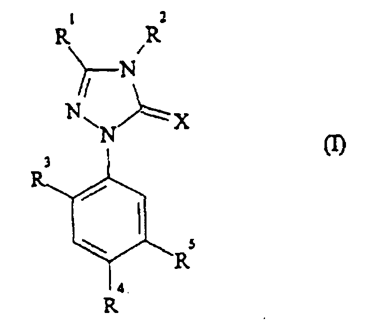 in which
R¹, R², R³, R⁴, R⁵ and X have the meanings given in Claim 1,
**characterised in that**
a) 1H-triazolinones of the formula (II) 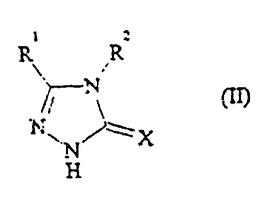 in which
R¹, R² and X have the meanings given in Claim 1,
are reacted with halogenobenzene derivatives of the formula (III) 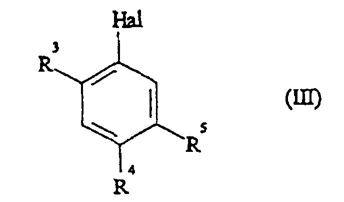 in which
R³, R⁴ and R⁵ ' have the meanings given in Claim 1 and
Hal represents halogen,
if appropriate in the presence of a diluent and if appropriate in the presence of a reaction auxiliary, or **in that**
b) substituted triazolinones of the formula (Ia) 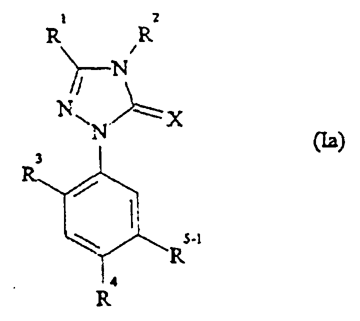 in which
R¹, R², R³, R⁴ and X have the meanings given in Claim 1 and
R⁵⁻¹ represents halogen,
are reacted with nucleophiles of the formula (IV)
R⁶⁻¹-Z-H (IV)
in which
Z represents oxygen or sulphur and
R⁶⁻¹ represents in each case straight-chain or branched, optionally substituted alkyl, alkenyl, alkinyl, cycloalkyl or aryl, and furthermore, in the event that Z represents oxygen, also represents heterocyclyl,
if appropriate in the presence of a diluent and if appropriate in the presence of a reaction auxiliary, or **in that**
c) substituted triazolinones of the formula (Ib) 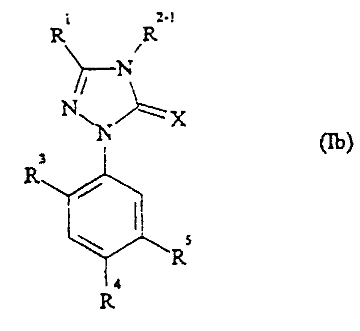 in which
R¹, R³, R⁴, R⁵ and X have the meanings given in Claim 1 and
R²⁻¹ represents amino,
are reacted with sodium nitrite in the presence of an acid and, if appropriate, in the presence of a diluent, or **in that**
d) substituted triazolinones of the formula (Ic) 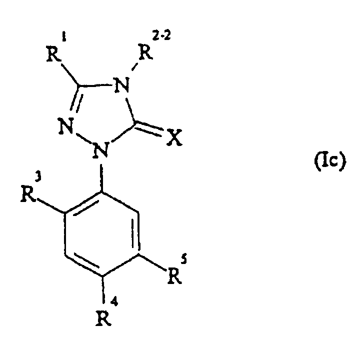 in which
R¹, R³, R⁴, R⁵ and X have the meanings given in Claim 1 and
R²⁻² represents hydrogen,
are reacted with alkylating agents of the formula (V)
R²⁻³-E (V)
in which
R²⁻³ represents alkyl, alkenyl, alkinyl, halogenoalkyl, halogenoalkenyl, halogenoalkinyl, alkoxyalkyl or optionally substituted cycloalkyl and
E represents an electron-attracting leaving group,
if appropriate in the presence of a diluent and if appropriate in the presence of a reaction auxiliary.

4. Method of combating undesired plants, **characterised in that** substituted triazolinones according to Claim 1 or 2 are allowed to act on plants and/or their environment.

5. Use of substituted triazolinones according to Claim 1 or 2 for combating undesired plants.

6. Process for the preparation of herbicidal and acaricidal agents, **characterised in that** substituted triazolinones according to Claim 1 or 2 are mixed with extenders and/or surface-active agents.

7. Method of combating arachnids, **characterised in that** substituted triazolinones according to Claim 1 or 2 are allowed to act on arachnids and/or their environment.

8. Use of substituted triazolinones according to Claim 1 or 2 for combating arachnids.

9. Substituted triazolinones according to Claim 1, **characterised in that**
R¹, R², R³, R⁴ and X have the meanings given in Claim 1, and
R⁵ represents fluorine, chlorine, bromine or iodine.

10. Substituted triazolinones according to Claim 1,
**characterised in that**
R¹, R³, R⁴, R⁵ and X have the meanings given in Claim 1, and
R² represents amino.

11. Substituted triazolinones of the formula (Ic) 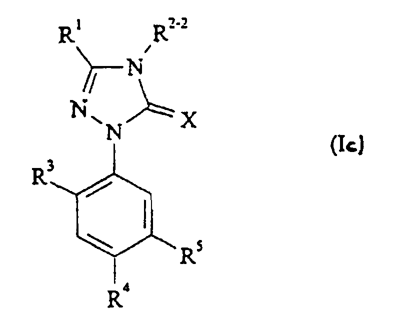 **characterised in that**
R¹, R³, R⁴, R⁵ and X have the meanings given in Claim 1, and
R²⁻² represents hydrogen.

## Revendications

1. Triazolinones substituées de formule générale (I) : 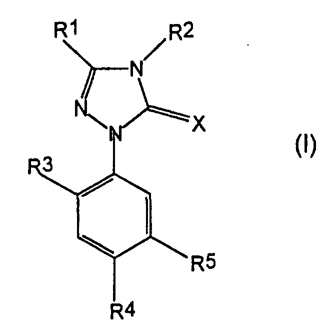 dans laquelle :
R¹ représente un halogénoalcoyle linéaire ou ramifié ayant 1 à 6 atomes de carbone et 1 à 13 atomes d'halogène identiques ou différents, en particulier les atomes de fluor, de chlore, de brome ou d'iode ;
R² représente un radical amino, un alcoyle linéaire ou ramifié ayant 1 à 8 atomes de carbone, un alcényle linéaire ou ramifié ayant 2 à 6 atomes de carbone, un halogénoalcoyle linéaire ou ramifié ayant 1 à 6 atomes de carbone et 1 à 13 atomes d'halogène identiques ou différents, en particulier les atomes de fluor, de chlore, de brome ou d'iode, un alcoxyalcoyle linéaire ou ramifié ayant chaque fois, 1 à 4 atomes de carbone dans les parties alcoyle individuelles, un alcoylidèneimino linéaire ou ramifié ayant 1 à 8 atomes de carbone ou un cycloalcoyle ou cycloalcoylalcoyle, substitué chaque fois facultativement dans la partie cycloalcoyle, une ou plusieurs fois, de manière identique ou différente par des atomes d'halogène, en particulier les atomes de fluor, de chlore, de brome et/ou d'iode, ayant chaque fois 3 à 8 atomes de carbone dans la partie cycloalcoyle et facultativement, 1 à 4 atomes de carbone dans la partie alcoyle linéaire ou ramifiée ;
R³ représente les atomes d'hydrogène, de fluor, de chlore, de brome ou d'iode ;
R⁴ représente lés radicaux cyano ou nitro ;
R⁵ représente les radicaux nitro, cyano, les atomes de fluor, de chlore, de brome, d'iode, les radicaux -R⁶, -O-R⁶, -S-R⁶, -NR⁶R⁷, ou un radical de formule : 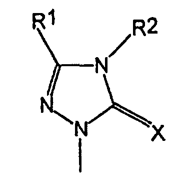 et
X représente l'atome d'oxygène ou de soufre, où
R⁶ et R⁷ représentent indépendamment l'un de l'autre, chaque fois l'atome d'hydrogène ou un alcoyle linéaire ou ramifié, facultativement une ou plusieurs fois substitués de manière identique ou différente, ayant 1 à 8 atomes de carbone, où comme substituant, on considère :
les atomes d'halogène, en particulier les atomes de fluor, de chlore, de brome et/ou d'iode, les radicaux cyano, carboxyle, carbamoyle, les radicaux alcoxy, alcoxyalcoxy, alcoylthio, alcoylsulfinyle, alcoylsulfonyle, alcoxycarbonyle, alcoxycarbonylalcoyle, N-alcoylaminocarbonyle, cycloalcoylaminocarbonyle, N,N-dialcoylaminocarbonyle, trialcoylsilyle ou alcoylsulfonylaminocarbonyle chaque fois linéaires ou ramifiés, ayant chaque fois 1 à 8 atomes de carbone dans les parties alcoyle individuelles ou un radical hétérocyclyle, où comme reste hétérocyclyle, on considère un hétérocycle de 5 à 7 membres, facultativement condensé à un radical benzène, saturé ou insaturé, ayant 1 à 3 hétéroatomes identiques ou différents, en particulier les atomes d'oxygène, d'azote et/ou de soufre ;
R⁶ et R⁷ représentent en outre, un alcényle ou alcynyle substitué chaque fois facultativement une ou plusieurs fois, de manière identique ou différente par des atomes d'halogène, en particulier les atomes de fluor, de chlore, de brome et/ou d'iode, ayant chaque fois 2 à 8 atomes de carbone ;
R⁶ et R⁷ représentent en outre, un cycloalcoyle ayant 3 à 7 atomes de carbone, facultativement substitué une ou plusieurs fois, de manière identique ou différente, par des atomes d'halogène, en particulier les atomes de fluor, de chlore, de brome et/ou d'iode, et/ou par un alcoyle linéaire ou ramifié ayant 1 à 4 atomes de carbone ou représentent un (cycloalcoyle en C₃-C₇)alcoyle en C₁-C₃, ou
R⁶ et R⁷ représentent un arylalcoyle ou aryle chaque fois facultativement substitué sur la partie aryle, une ou plusieurs fois, de manière identique ou différente, ayant 6 à 10 atomes de carbone dans la partie aryle et facultativement, 1 à 4 atomes de carbone dans la partie alcoyle linéaire ou ramifiée, où comme substituants du reste aryle, on considère chaque fois :
les atomes d'halogène, les radicaux cyano, nitro, les radicaux alcoyle, alcoxy, alcoylthio, alcoylsulfinyle ou alcoylsulfonyle chaque fois linéaires ou ramifiés, ayant chaque fois 1 à 6 atomes de carbone, les radicaux halogénoalcoyle, halogénoalcoxy, halogénoalcoylthio, halogénoalcoylsulfinyle ou halogénoalcoylsulfonyle chaque fois linéaires ou ramifiés, ayant chaque fois, 1 à 6 atomes de carbone et 1 à 13 atomes d'halogène identiques ou différents, les radicaux alcoxycarbonyle ou alcoxyiminoalcoyle chaque fois linéaires ou ramifiés, ayant chaque fois 1 à 6 atomes de carbone dans les parties alcoyle individuelles, ainsi que le radical phényle facultativement substitués une ou plusieurs fois, de manière identique ou différente par un atome d'halogène et/ou un alcoyle ou alcoxy linéaire ou ramifié, ayant chaque fois 1 à 6 atomes de carbone et/ou par un halogénoalcoyle ou halogénoalcoxy ayant chaque fois 1 à 6 atomes de carbone et 1 à 13 atomes d'halogène identiques ou différents.

2. Triazolinones substituées suivant la revendication 1, **caractérisées en ce que** :
R¹ représente un halogénoalcoyle linéaire ou ramifié ayant 1 à 4 atomes de carbone et 1 à 9 atomes d'halogène identiques ou différents, en particulier les atomes de fluor, de chlore ou de brome;
R² représente le radical amino, un alcoyle linéaire ou ramifié ayant 1 à 6 atomes de carbone, un alcényle linéaire ou ramifié ayant 2 à 4 atomes de carbone, un halogénoalcoyle linéaire ou ramifié ayant 1 à 4 atomes de carbone et 1 à 9 atomes d'halogène identiques ou différents, en particulier les atomes de fluor, de chlore ou de brome, un alcoxyalcoyle linéaire ou ramifié ayant chaque fois, 1 à 3 atomes de carbone dans les parties alcoyle individuelles, un alcoylidèneimino linéaire ou ramifié ayant 1 à 6 atomes de carbone ou un cycloalcoyle ou cycloalcoylalcoyle, substitué chaque fois facultativement dans la partie cycloalcoyle, une à quatre fois, de manière identique ou différente par des atomes d'halogène, en particulier les atomes de fluor, de chlore et/ou de brome, ayant chaque fois 3 à 7 atomes de carbone dans la partie cycloalcoyle et facultativement, 1 à 3 atomes de carbone dans la partie alcoyle linéaire ou ramifiée ;
R³ représente les atomes d'hydrogène, de fluor, de chlore ou de brome ;
R⁴ représente les radicaux cyano ou nitro ;
R⁵ représente les radicaux nitro, cyano, les atomes de fluor, de chlore, de brome, les radicaux -R⁶, -O-R⁶, -S-R⁶, -NR⁶R⁷, ou un radical de formule : 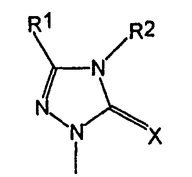 et
X représente l'atome d'oxygène ou de soufre, où
R⁶ et R⁷ représentent indépendamment l'un de l'autre, l'atome d'hydrogène ou un alcoyle linéaire ou ramifié, facultativement une fois substitué, ayant 1 à 6 atomes de carbone, où comme substituant, on considère :
les radicaux cyano, carboxyle, carbamoyle, les radicaux alcoxy, alcoxyalcoxy, alcoylthio, alcoylsulfinyle, alcoylsulfonyle, alcoxycarbonyle, alcoxycarbonylalcoyle, N-alcoylaminocarbonyle, N,N-dialcoylaminocarbonyle, trialcoylsilyle ou alcoylsulfonylaminocarbonyle chaque fois linéaires ou ramifiés, ayant chaque fois 1 à 6 atomes de carbone dans les parties alcoyle individuelles ou un radical hétérocyclyle, où comme reste hétérocyclyle, on considère un hétérocycle de 5 ou 6 membres, saturé ou insaturé, ayant 1 à 3 hétéroatomes identiques ou différents, en particulier les atomes d'oxygène, d'azote et/ou de soufre ;
R⁶ et R⁷ représentent en outre, un halogénoalcoyle linéaire ou ramifié, facultativement substitué en plus des atomes d'halogène, ayant 1 à 4 atomes de carbone et 1 à 9 atomes d'halogène identiques ou différents, en particulier les atomes de fluor, de chlore ou de brome, où comme substituant, on considère un (alcoxy en C₁-C₂)carbonyle, un (cycloalcoyl en C₁-C₆)aminocarbonyle ou le radical cyano ;
R⁶ et R⁷ représentent en outre, un alcényle ou alcynyle chaque fois facultativement substitué une à trois fois, de manière identique ou différente par des atomes d'halogène, en particulier les atomes de fluor, de chlore ou de brome, ayant chaque fois 2 à 6 atomes de carbone ;
R⁶ et R⁷ représentent en outre, un cycloalcoyle ayant 3 à 6 atomes de carbone, facultativement substitué une à quatre fois, de manière identique ou différente, par des atomes d'halogène, en particulier les atomes de fluor, de chlore ou de brome, et/ou par un alcoyle linéaire ou ramifié ayant 1 à 3 atomes de carbone ou représentent un (cycloalcoyle en C₃-C₆) alcoyle en C₁-C₂, ou
R⁶ et R⁷ représentent un phénylalcoyle ou le radical phényle chaque fois facultativement substitué sur la partie phényle, une à trois fois, de manière identique ou différente, ayant facultativement, 1 à 3 atomes de carbone dans la partie alcoyle linéaire ou ramifiée, où comme substituants du reste phényle, on considère chaque fois :
les atomes d'halogène, les radicaux cyano, nitro, les radicaux alcoyle, alcoxy, alcoylthio, alcoylsulfinyle ou alcoylsulfonyle chaque fois linéaires ou ramifiés, ayant chaque fois 1 à 4 atomes de carbone, les radicaux halogénoalcoyle, halogénoalcoxy, halogénoalcoylthio, halogénoalcoylsulfinyle ou halogénoalcoylsulfonyle chaque fois linéaires ou ramifiés, ayant chaque fois, 1 à 4 atomes de carbone et 1 à 9 atomes d'halogène identiques ou différents, les radicaux alcoxycarbonyle ou alcoxyiminoalcoyle chaque fois linéaires ou ramifiés, ayant chaque fois 1 à 4 atomes de carbone dans les parties alcoyle individuelles, ainsi que le radical phényle facultativement substitué une ou plusieurs fois, de manière identique ou différente par un atome d'halogène et/ou un alcoyle ou alcoxy linéaire ou ramifié, ayant chaque fois 1 à 4 atomes de carbone et/ou par un halogénoalcoyle ou halogénoalcoxy linéaire ou ramifié ayant chaque fois 1 à 4 atomes de carbone et 1 à 9 atomes d'halogène identiques ou différents.

3. Procédé de préparation des nouvelles triazolinones substituées de formule générale (I) : 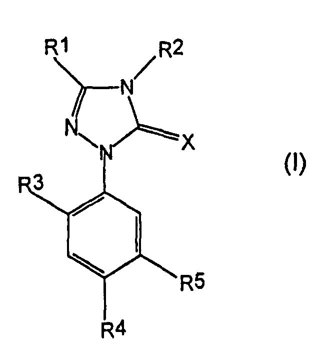 dans laquelle :
R¹, R², R³, R⁴, R⁵ et X ont les significations indiquées à la revendication 1,
**caractérisé en ce que** l'on :
a) fait réagir une 1H-triazolinone de formule (II) : 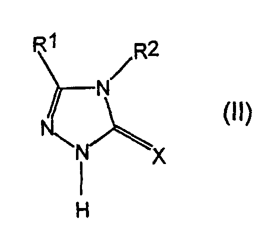 dans laquelle :
R¹, R² et X ont les significations indiquées à la revendication 1,
avec un dérivé halogénobenzène de formule (III) : 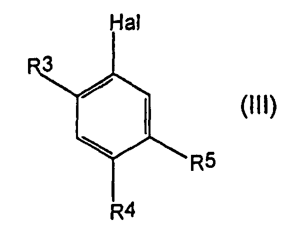 dans laquelle :
R³, R⁴ et R⁵ ont les significations indiquées à la revendication 1, et
Hal représente un atome d'halogène,
facultativement en présence d'un agent de dilution et facultativement, en présence d'un auxiliaire de réaction, ou **en ce que** l'on
b) fait réagir une triazolinone substituée de formule (Ia) : 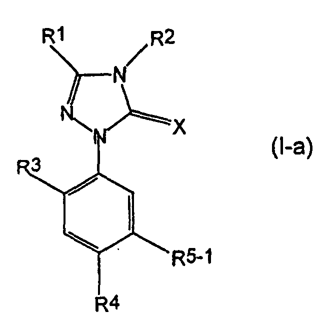 dans laquelle :
R¹, R², R³, R⁴ et X ont les significations indiquées à la revendication 1,
R⁵⁻¹ représente un atome d'halogène,
avec un nucléophile de formule (IV) :
R⁶⁻¹ - Z - H (IV)
dans laquelle:
Z représente l'atome d'oxygène ou de soufre, et
R⁶⁻¹ représente un alcoyle, alcényle, alcynyle, cycloalcoyle ou aryle chaque fois linéaire ou ramifié, facultativement substitué, et dans le cas où Z représente l'atome d'oxygène, il représente également un hétérocyclyle, facultativement en présence d'un agent de dilution et facultativement, en présence d'un auxiliaire de réaction, ou **en ce que** l'on
c) fait réagir une triazolinone substituée de formule (Ib) : 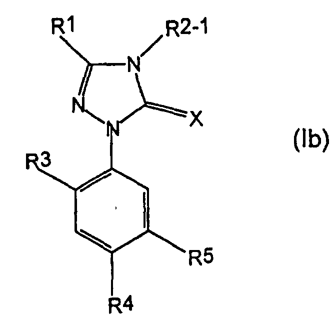 dans laquelle :
R¹, R³, R⁴, R⁵ et X ont les significations indiquées à la revendication 1,
R²⁻¹ représente le radical amino,
avec du nitrite de sodium en présence d'un acide et facultativement, en présence d'un agent de dilution, ou **en ce que** l'on : d) fait réagir une triazolinone substituée de formule (Ic) : 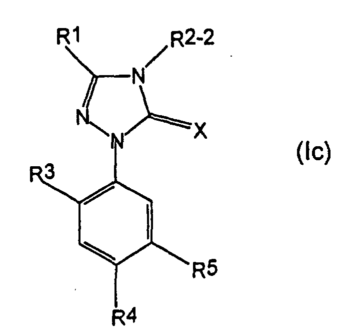 dans laquelle :
R¹, R³, R⁴, R⁵ et X ont les significations indiquées à la revendication 1,
R²⁻² représente l'atome d'hydrogène, avec un agent d'alcoylation de formule (V) :
R²⁻³ - E (V)
dans laquelle:
R²⁻³ représente un alcoyle, alcényle, alcynyle, halogénoalcoyle, halogénoalcényle, halogénoalcynyle, alcoxyalcoyle, ou un cycloalcoyle facultativement substitué, et
E représente un groupe partant capteur d'électrons,
facultativement en présence d'un agent de dilution et facultativement, en présence d'un auxiliaire de réaction.

4. Procédé de lutte contre des plantes non désirées, **caractérisé en ce que** l'on fait agir les triazolinones substituées suivant la revendication 1 ou 2, sur les plantes et/ou leur biotope.

5. Utilisation des triazolinones substituées suivant la revendication 1 ou 2, pour lutter contre des plantes non désirées.

6. Procédé de préparation d'agents herbicides et acaricides, **caractérisé en ce que** l'on mélange les triazolinones substituées suivant la revendication 1 ou 2, avec un agent de dilution et/ou , un agent tensioactif.

7. Procédé de lutte contre les arachnides,
**caractérisé en ce que** l'on fait agir les triazolinones substituées suivant la revendication 1 ou 2, sur les arachnides et/ou leur biotope.

8. Utilisation des triazolinones substituées suivant la revendication 1 ou 2, pour lutter contre des arachnides.

9. Triazolinones substituées suivant la revendication 1, **caractérisées en ce que** :
R¹, R², R³, R⁴ et X on les significations données à la revendication 1, et
R⁵ représente les atomes de fluor, de chlore, de brome ou d'iode.

10. Triazolinones substituées suivant la revendication 1, **caractérisées en ce que** :
R¹, R³, R⁴, R⁵ et X ont les significations données à la revendication 1, et
R² représente un radical amino.

11. Triazolinones substituées de formule (Ic) : 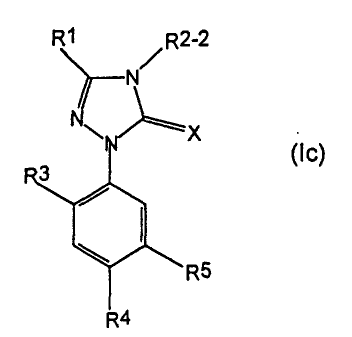 **caractérisées en ce que** :
R¹, R³, R⁴, R⁵ et X ont les significations données à la revendication 1, et
R²⁻² représente l'atome d'hydrogène.
